(19) Europäisches Patentamt
European Patent Office
Office européen des brevets

(11) **EP 1 604 634 A1**

(12) **DEMANDE DE BREVET EUROPEEN**

(43) Date de publication:
**14.12.2005 Bulletin 2005/50**

(51) Int Cl.[7]: **A61K 7/025**, A61K 7/032, A61K 7/043

(21) Numéro de dépôt: **05291036.1**

(22) Date de dépôt: **13.05.2005**

(84) Etats contractants désignés:
**AT BE BG CH CY CZ DE DK EE ES FI FR GB GR HU IE IS IT LI LT LU MC NL PL PT RO SE SI SK TR**
Etats d'extension désignés:
**AL BA HR LV MK YU**

(30) Priorité: **08.06.2004 FR 0406175**

(71) Demandeur: **L'OREAL**
**75008 Paris (FR)**

(72) Inventeurs:
- **Filippi, Vanina**
  **75015 Paris (FR)**
- **Lebre, Caroline**
  **94320 Thiais (FR)**

(74) Mandataire: **Chantraine, Sylvie Hélène**
**L'OREAL - D.I.P.I.**
**25-29, Quai Aulagnier**
**92600 Asnieres (FR)**

(54) **Composition cosmétique contenant un ester et un agent filmogène**

(57) La présente invention concerne une composition cosmétique de maquillage et/ou de soin de la peau, des lèvres et/ou des phanères comprenant dans un milieu physiologiquement acceptable au moins un ester de dimère diol et de dimère diacide d'acides gras insaturés et de dimère diol et au moins un agent filmogène.

EP 1 604 634 A1

## Description

La présente invention se rapporte à une composition cosmétique, notamment une composition cosmétique de maquillage ou de soin de la peau, y compris du cuir chevelu, aussi bien du visage que du corps humain, des lèvres ou des phanères des êtres humains, comme les cheveux, les cils, les sourcils ou les ongles, comprenant un milieu cosmétiquement acceptable.

La composition de l'invention peut en particulier constituer un produit de maquillage du corps, des lèvres ou des phanères d'êtres humains ayant en particulier des propriétés de soin et/ou de traitement non thérapeutique. Elle constitue notamment un rouge à lèvres ou un brillant à lèvres, un fard à joues ou à paupières, un produit pour tatouage, un mascara, un eye-liner, un vernis à ongles, un produit de bronzage artificiel de la peau, un produit de coloration ou de soin des cheveux.

Il existe de nombreuses compositions cosmétiques pour lesquelles les propriétés de brillance du film déposé, après application sur les matières kératiniques (peau, lèvres, phanères), sont souhaitables. On peut citer par exemple les rouges à lèvres, les vernis à ongles ou encore certains produits capillaires. Pour ce faire, le formulateur utilise généralement à titre d'agent actif principal en terme de brillance des lanolines associées à au moins une huile dite « brillante », comme a) des polymères huileux tels que les polybutènes qui ont une viscosité élevée, b) des esters d'acide ou d'alcool gras dont le nombre de carbone est élevé (typiquement supérieur à 16), ou encore c) certaines huiles végétales.

Toutefois, les compositions de l'art antérieur brillantes présentent le désavantage d'avoir une tenue insuffisante au cours du temps. Ces compositions, lorsqu'elles sont appliquées sur la peau ou les lèvres, peuvent être altérées lors du contact avec des liquides, notamment du sébum, de l'eau, des boissons ou des huiles comme les huiles alimentaires consommées par exemple lors d'un repas ou bien encore avec la salive.

On recherche donc des compositions cosmétiques de maquillage ou de soin formant un dépôt présentant une bonne tenue au contact des liquides mis en contact avec le maquillage, notamment au cours d'un repas.

L'invention a précisément pour objet de proposer une association particulière comprenant au moins un ester de dimère diol avec un diacide dicarboxylique spécifique permettant avantageusement de donner satisfaction en terme de brillance sans par ailleurs affecter la tenue de la composition cosmétique.

Les esters de dimères diols et d'acides mono ou dicarboxyliques ont été décrits d'une manière générale dans le document FR 2 795 309 comme étant utiles pour préparer des compositions cosmétiques dotées notamment de propriétés de stabilité améliorées. Plus récemment, les documents JP 2002-128623, 2002-128628 et 2002-128629 proposent des compositions cosmétiques, notamment de maquillage, incluant à titre d'agent actif pour la brillance des esters de diacides dilinoléiques avec des dimères diols dilinoléiques.

Plus précisément, l'invention repose sur l'observation par les inventeurs qu'une composition comprenant l'association d'au moins un ester de dimère diol et d'acide avec au moins un agent filmogène est brillante et de bonne tenue.

En conséquence, la présente invention concerne, selon un de ses aspects, une composition cosmétique comprenant dans un milieu physiologiquement acceptable au moins un ester de dimère diol et d'au moins un acide monocarboxylique en $C_4$ à $C_{34}$ ou di-carboxylique, et au moins un agent filmogène.

La présente invention concerne, selon un autre de ses aspects, un procédé de maquillage et/ou de soin de la peau, des lèvres et/ou des phanères comprenant l'application sur la peau, les lèvres et/ou les phanères d'au moins une composition selon l'invention.

Les esters de dimère diol et d'acide utilisables dans le cadre de la présente invention sont disponibles commercialement ou peuvent être préparés de manière conventionnelle. Ils sont notamment d'origine végétale et peuvent être obtenus par estérification d'un dimère diol avec un acide mono-carboxylique en $C_4$-$C_{34}$ comme par exemple un acide gras, ou avec un acide dicarboxylique tel qu'un dimère diacide.

Les esters de dimère diol et d'acide obtenus par estérification avec un acide mono-carboxylique peuvent avoir un poids moléculaire relativement élevé, allant d'environ 1000 à 1300 g/mol. On peut obtenir un dicarboxylate de dimère diol qui présente un poids moléculaire moyen en poids, déterminé par chromatographie de perméation de gel (GPC), allant de 2000 à 20 000 g/mol, de préférence entre 2000 et 4000 g/mol.

L'acide mono-carboxylique utilisable dans la présente invention comporte de 4 à 34 atomes de carbone, et notamment de 10 à 32 atomes de carbone.

A titre illustratif des exemples d'acide mono-carboxylique convenant à l'invention, on peut notamment citer :

- les acides linéaires saturés tels que l'acide butanoïque, l'acide pentanoïque, l'acide hexanoïque, l'acide heptanoïque, l'acide octanoïque, l'acide nonanoïque, l'acide décanoïque, l'acide undécanoïque, l'acide dodécanoïque, l'acide tridécanoïque, l'acide tétradécanoïque, l'acide heptadécanoïque, l'acide hexadécanoïque, l'acide pentadécanoïque, l'acide octadécanoïque, l'acide nonadécanoïque, l'acide eicosanoïque, l'acide docosanoïque, l'acide tétracosanoïque,
- les acides gras ramifiés tels que par exemple l'acide isobutanoïque, l'acide isopentanoïque, l'acide pivalique, l'acide

isohexanoïque, l'acide isoheptanoïque, l'acide isooctanoïque, l'acide diméthyloctanoïque, l'acide isononanoïque, l'acide isodécanoïque, l'acide isoundécanoïque, l'acide isododécanoïque, l'acide isotridécanoïque, l'acide isotétradécanoïque, l'acide isopentadécanoïque, l'acide isohexadécanoïque, l'acide isoheptadécanoïque, l'acide isooctadécanoïque, l'acide isononadécanoïque, l'acide isoeicosanoïque, l'acide 2-éthylhexanoïque, l'acide 2-butyloctanoïque, l'acide 2-hexyldécanoïque, l'acide 2-octyldodécanoïque, l'acide 2-décyltétradécanoïque, l'acide 2-dodécylhexadécanoïque, l'acide 2-tétradécyloctadécanoïque, l'acide 2-hexadécyloctadécanoïque, des acides gras à longue chaîne obtenus à partir de la lanoline,

- les acides gras linéaires insaturés en $C_8$ à $C_{34}$, tels que l'acide undécénoïque, l'acide lindérique, l'acide myristoléique, l'acide palmitoléique, l'acide oléique, l'acide linoléique, l'acide élaïdinique, l'acide gadolénoïque, l'acide eicosapentaénoïque, l'acide docosahexaénoïque, l'acide érucique, l'acide brassidique, l'acide arachidonique,
- des hydroxyacides tels que l'acide 2-hydroxybutanoïque, l'acide 2-hydropentanoïque, l'acide 2-hydroxyhexanoïque, l'acide 2-hydroxyheptanoïque, l'acide 2-hydroxyoctanoïque, l'acide 2-hydroxynonanoïque, l'acide 2-hydroxydécanoïque, l'acide 2-hydroxyundécanoïque, l'acide 2-hydroxydodécanoïque, l'acide 2-hydroxytridécanoïque, l'acide 2-hydroxytétradécanoïque, l'acide 2-hydroxyhexadécanoïque, l'acide 2-hydroxyheptadécanoïque, l'acide 2-hydroxyoctadécanoïque, l'acide 12-hydroxyoctadécanoïque, l'acide 2-hydroxynonadécanoïque, l'acide 2-hydroxyeicosanoïque, l'acide 2-hydroxydocosanoïque, l'acide 2-hydroxytétracosanoïque,
- des acides cycliques tels que l'acide cyclohexanoïque, la rosine hydrogénée, la rosine, l'acide abiétique, l'acide abiétique hydrogéné, l'acide benzoïque, l'acide p-oxybenzoïque, l'acide p-aminobenzoïque, l'acide cinnamique, l'acide p-méthoxycinnamique, l'acide salicylique, l'acide gallique, l'acide pyrrolidonecarboxylique, l'acide nicotinique, et
- des acides gras d'origine naturelle, tels que les acides gras d'huile d'orange, d'huile d'avocat, d'huile de macadamia, d'huile d'olive, d'huile de soja hydrogénée, d'huile de jojoba, d'huile de palme, d'huile de ricin, d'huile de germe de blé, d'huile de safran, d'huile de grains de coton, d'huile de vison et leurs mélanges.

Il s'agit plus particulièrement d'un acide gras, notamment tel que défini ci-dessus. Par acide gras, on entend un acide carboxylique obtenu par hydrolyse de graisses ou d'huiles végétales ou animales. L'acide gras peut être saturé ou insaturé.

L'ester obtenu peut être un diester, un monoester ou un de leurs mélanges. En l'occurrence, l'ester peut être un mélange de deux ou plusieurs types d'ester formés avec différents acides carboxyliques.

L'acide dicarboxylique utilisable selon l'invention doit contenir au moins deux groupes carboxyliques par molécule. Il peut notamment être représenté par la formule (I) suivante :

$$HOOC\text{-}(CH_2)_n\text{-}COOH \qquad (I)$$

dans laquelle n est un nombre entier de 1 à 16, de préférence de 3 à 16.

A titre illustratif et non limitatif des acides dicarboxyliques convenant à l'invention, on peut notamment citer l'acide malonique, l'acide succinique, l'acide glutarique, l'acide adipique, l'acide pimélique, l'acide subérique, l'acide azélaïque, l'acide sébacique, l'acide 1,9-nonaméthylène-dicarboxylique, l'acide 1,10-décaméthylènedicarboxylique, l'acide 1,11-ndécaméthylènedicarboxylique, l'acide 1,12-dodécaméthylène-dicarboxylique, l'acide 1,13-tridécaméthylènedicarboxylique, l'acide 1,14-tétradécaméthylènedicarboxylique, l'acide 1,15-pentadécaméthylènedicarboxylique, l'acide 1,16-hexadécaméthylènedicarboxylique et leurs mélanges.

L'acide dicarboxylique peut également être un dimère diacide. Un dimère diacide désigne un diacide obtenu par réaction de polymérisation, notamment de dimérisation, intermoléculaire d'au moins un acide mono-carboxylique insaturé.

Ils dérivent en particulier de la dimérisation d'un acide gras insaturé notamment en $C_8$ à $C_{34}$, notamment en $C_{12}$ à $C_{22}$, en particulier en $C_{16}$ à $C_{20}$, et plus particulièrement en $C_{18}$.

A titre représentatif de ces acides gras insaturés, on peut notamment citer comme précédemment l'acide undécénoïque, l'acide lindérique, l'acide myristoléique, l'acide palmitoléique, l'acide oléique, l'acide linoléique, l'acide élaïdinique, l'acide gadolénoïque, l'acide eicosapentaénoïque, l'acide docosahexaénoïque, l'acide érucique, l'acide brassidique, l'acide arachidonique et leurs mélanges.

Selon une variante particulière, il s'agit plus particulièrement du dimère diacide dont dérive également le dimère diol à estérifier.

Plus particulièrement, il s'agit du dimère diacide obtenu par dimérisation de l'acide linoléique, éventuellement suivie d'une hydrogénation des liaisons carbone carbone. Le dimère diacide peut être sous forme saturée, c'est-à-dire ne comporter aucune double liaison carbone carbone. Selon un autre mode de réalisation, les éventuelles double liaison carbone carbone du dimère diacide sont toutes ou en partie hydrogénées, après réaction d'estérification du

dimère diacide avec le dimère diol.

Selon un mode de réalisation, le dimère diacide est un produit commercialisé constitué d'un acide dicarboxylique ayant environ 36 atomes de carbone. Ce produit contient également un acide trimérique et un acide monomère, dans des proportions qui dépendent du degré de pureté du produit. Classiquement, on trouve dans le commerce des produits dont la teneur en dimère diacide est supérieure à 70 % et d'autres dont la teneur en dimère diacide a été ajustée à 90 % ou plus.

On trouve également dans le commerce des dimères diacides et notamment des diacides dilinoléiques dont la stabilité vis-à-vis de l'oxydation a été améliorée par hydrogénation des doubles liaisons restant après la réaction de dimérisation.

Dans la présente invention, on peut utiliser tout dimère diacide disponible actuellement dans le commerce.

Dans une réaction d'estérification avec un acide dicarboxylique et en particulier un dimère diacide, le degré moyen d'estérification et le poids moléculaire moyen de l'ester obtenu peuvent être ajustés en faisant varier le rapport du dimère diol à l'acide dicarboxylique et en particulier au dimère diacide.

Le rapport, exprimé comme la proportion molaire d'un acide dicarboxylique basée sur le poids moléculaire moyen calculé à partir de son indice d'acide pour 1 mole de dimère diol basée sur le poids moléculaire moyen calculé à partir de son indice d'hydroxyle, est compris généralement entre 0,2 et 1,2 mole, en particulier entre 0,4 et 1,0, par exemple égal à 0,5 ou 0,7.

Au sens de la présente invention, on entend plus particulièrement désigner sous le terme « dimère diol » des diols saturés produits par hydrogénation des dimères diacides correspondants, un dimère diacide étant tel que défini ci-dessus.

En ce qui concerne le dimère diol fabriqué industriellement, il contient également généralement d'autres composants, par exemple, un trimère triol, un monoalcool et des composés de type éther, selon le degré de purification de l'acide dimérique et/ou de l'ester d'alcool inférieur de celui-ci, utilisé comme matière première. Généralement, les produits dont la teneur en dimère diol est supérieure à 70 % peuvent être utilisés dans la présente invention. Toutefois, il est préférable d'utiliser un dimère diol de grande pureté, tel qu'un composé dont la teneur en dimère diol est supérieure à 90 %.

Ainsi, un dimère diol peut être produit par hydrogénation catalytique d'un dimère diacide, lui-même obtenu par dimérisation d'au moins un acide gras insaturé notamment en $C_8$ à $C_{34}$, tels que ceux cités précédemment, notamment en $C_{12}$ à $C_{22}$, en particulier en $C_{16}$ à $C_{20}$, et plus particulièrement en $C_{18}$ à l'image par exemple de l'acide oléique et de l'acide linoléique.

Selon un m ode de réalisation particulier, le dimère diol dérive de l'hydrogénation des fonctions acides du diacide dilinoléique.

Plus particulièrement, il s'agit du dimère diol obtenu par dimérisation de l'acide linoléique, suivie d'une hydrogénation des fonctions acides. Le dimère diol peut être sous forme saturée, c'est-à-dire ne comporter aucune double liaison carbone carbone. Selon un autre mode de réalisation, les éventuelles double liaison carbone carbone du dimère diol sont toutes ou en partie hydrogénées, après réaction d'estérification du dimère diacide avec le dimère diol.

Selon une variante de l'invention, l'ester de dimère diol est un ester de dimère diol et de dimère diacide et notamment est un composé de formule générale (II)

$$HO\text{-}R^1\text{-}(\text{-}OCO\text{-}R^2\text{-}COO\text{-}R^1\text{-})_h\text{-}OH \qquad (II)$$

dans laquelle :

- $R^1$ représente un reste de dimère diol obtenu par hydrogénation du diacide dilinoléique
- $R^2$ représente un reste de diacide dilinoléique hydrogéné, et
- h représente un entier variant de 1 à 9.

A titre illustratif des esters convenant à l'invention, on peut notamment citer les esters de diacides dilinoléiques et de dimères diols dilinoléiques commercialisés par la société NIPPON FINE CHEMICAL sous la dénomination commerciale LUSPLAN DD-DA5® et DD-DA7®.

La quantité en ester selon l'invention est généralement ajustée de manière à contrôler la brillance moyenne de la composition à la valeur souhaitée. En l'occurrence, l'ester peut être présent à raison de 1 à 99 % en particulier, notamment de 2 à 60 % en poids, en particulier de 5 à 40 % et plus particulièrement de 10 à 35 % en poids par rapport au poids total de la composition.

Agent filmogène

Par polymère "filmogène", on entend un polymère apte à former à lui seul ou en présence d'un agent auxiliaire de filmification, un film continu et adhérent sur un support, notamment sur les matières kératiniques, et de préférence un film cohésif et mieux encore, un film dont la cohésion et les propriétés mécaniques sont telles que ledit film peut être isolé dudit support.
Parmi les autres polymères filmogènes utilisables dans la composition de la présente invention, on peut citer les polymères synthétiques, de type radicalaire ou de type polycondensat, les polymères d'origine naturelle et leurs mélanges. Comme polymère filmogène, on peut citer en particulier les polymères acryliques, les polyuréthanes, les polyesters, les polyamides, les polyurées, les polymères cellulosiques comme la nitrocellulose.
Dans un mode de réalisation, le polymère organique filmogène est au moins un polymère choisi parmi le groupe comprenant :

- les polymères filmogènes solubles dans la phase grasse liquide, en particulier les polymères liposolubles, lorsque la phase grasse liquide comprend au moins une huile,
- les polymères filmogènes dispersibles dans la phase grasse liquide, distincts du polymère éthylénique greffé, en particulier les polymères sous la forme de dispersions non aqueuses de particules de polymères, de préférence des dispersions dans les huiles siliconées ou hydrocarbonées; dans un mode de réalisation, les dispersions non aqueuses de polymère comprennent des particules de polymères stabilisées sur leur surface mentionnées précédemment,
- les dispersions aqueuses de particules de polymères filmogènes, souvent appelées « latex » ; dans ce cas, la composition doit comprendre, outre la phase grasse liquide, une phase aqueuse,
- les polymères filmogènes hydrosolubles ; dans ce cas, la composition doit comprendre, outre la phase grasse liquide, une phase aqueuse.

La composition selon l'invention peut comprendre, à titre d'agent filmogène, une dispersion de particules d'un polymère éthylénique greffé dans une phase grasse liquide.
Par polymère "éthylénique", on entend un polymère obtenu par polymérisation de monomères comprenant une insaturation éthylénique.
La dispersion de polymère éthylénique greffé est notamment exempte de polymère stabilisant distinct dudit polymère greffé, tels que ceux décrits dans EP749747 et décrits plus loin, et les particules de polymère éthylénique greffé ne sont donc pas stabilisées en surface par de tels polymères stabilisants additionnels. Le polymère greffé est donc dispersé dans la phase grasse liquide en l'absence de stabilisant additionnel en surface des particules.
Par polymère "greffé", on entend un polymère ayant un squelette comprenant au moins une chaîne latérale pendante ou située en bout de chaîne, et de préférence pendante.
Avantageusement, le polymère éthylénique greffé comprend un squelette éthylénique insoluble dans ladite phase grasse liquide, et des chaînes latérales liées de manière covalente audit squelette et solubles dans la phase grasse liquide.
Le polymère éthylénique greffé est notamment un polymère non réticulé. En particulier, le polymère est obtenu par polymérisation de monomères comprenant un seul groupement polymérisable.
Selon un mode de réalisation de l'invention, le polymère éthylénique greffé est un polymère acrylique greffé.
Le polymère éthylénique greffé est notamment susceptible d'être obtenu par polymérisation radicalaire dans un milieu organique de polymérisation :

- d'au moins un monomère éthylénique, en particulier d'au moins un monomère acrylique et éventuellement d'au moins un monomère additionnel vinylique non acrylique, pour former ledit squelette insoluble ; et
- d'au moins un macromonomère comportant un groupe terminal polymérisable pour former les chaînes latérales, ledit macromonomère ayant une masse moléculaire moyenne en poids supérieure ou égale à 200 et la teneur en macromonomère polymérisé représentant de 0,05 à 20 % en poids du polymère.

La phase grasse liquide peut contenir le milieu organique de polymérisation du polymère éthylénique greffé.
Le milieu organique liquide de dispersion, correspondant au milieu dans lequel est fourni le polymère greffé, peut être identique au milieu de polymérisation.
Toutefois, le milieu de polymérisation peut être substitué en tout ou partie par un autre milieu organique liquide. Cet autre milieu organique liquide peut être ajouté, après polymérisation, au milieu de polymérisation. Ce dernier est ensuite évaporé en tout ou partie.

La phase grasse liquide peut contenir des composés liquides organiques autres que ceux présents dans le milieu de dispersion. Ces autres composés sont choisis de manière à ce que le polymère greffé reste à l'état de dispersion

dans la phase grasse liquide.

Le milieu liquide organique de dispersion est présent dans la phase grasse liquide de la composition selon l'invention du fait de l'introduction dans la composition de la dispersion de polymère greffé obtenue.

La phase grasse liquide comprend, de préférence majoritairement un ou plusieurs composés organiques liquides (ou huiles) tels que définis ci-après.

En particulier, la phase grasse liquide est une phase organique liquide non aqueuse et non miscible à l'eau à la température ambiante (25 °C).

On entend par "composé organique liquide" un composé non aqueux qui est à l'état liquide à la température ambiante (25 °C) et qui s'écoule donc de son propre poids.

On entend par "composé siliconé" un composé contenant au moins un atome de silicium.

La composition selon l'invention contient avantageusement u ne huile volatile telle que décrite ci-après.

Par huile volatile, on entend une huile susceptible de s'évaporer de la peau, des lèvres ou des fibres kératiniques en moins d'une heure, ayant notamment une pression de vapeur, à température ambiante et pression atmosphérique allant de $10^{-3}$ à 300 mm de Hg (0,13 Pa à 40 000 Pa).

L'huile volatile peut être siliconée ou non siliconée. Elle peut être notamment choisie parmi l'octaméthylcyclotétrasiloxane, le décaméthylcyclopentasiloxane, le dodécaméthylcyclohexasiloxane, l'heptaméthyl-hexyltrisiloxane, l'heptaméthyloctyltrisiloxane, l'octaméthyltrisiloxane, le décaméthyltétrasiloxane, l'isododécane, l'isodécane, l'isohexadécane, et leurs mélanges.

L'huile volatile est avantageusement présente en une teneur allant de 1 % à 70 % en poids, par rapport au poids total de la composition, de préférence allant de 5 % à 50 % en poids, et préférentiellement allant de 10 % à 35 % en poids.

La phase grasse liquide peut contenir une huile non volatile telle que décrite ci-après. L'huile non volatile est avantageusement présente en une teneur allant de 1 % à 80 % en poids, par rapport au poids total de la composition, de préférence allant de 5 % à 60 % en poids, et préférentiellement allant de 10 % à 50 % en poids.

Parmi les composés organiques liquides ou huiles pouvant être présents dans le milieu organique liquide de dispersion, on peut citer :

- les composés organiques liquides, notamment non siliconés ou siliconés, ayant un paramètre de solubilité global selon l'espace de solubilité de Hansen inférieur ou égal à 18 $(MPa)^{1/2}$, de préférence inférieur ou égal à 17 $(MPa)^{1/2}$,
- les monoalcools ayant un paramètre de solubilité global selon l'espace de solubilité de Hansen inférieur ou égal à 20 $(MPa)^{1/2}$; et
- leurs mélanges.

Le paramètre de solubilité global δ selon l'espace de solubilité de Hansen est défini dans l'article « Solubility parameter values » de Eric A.Grulke de l'ouvrage « Polymer Handbook », 3éme édition, Chapitre VII, p.519-559 par la relation :

$$\delta = (\delta_D^{\ 2} + \delta_P^{\ 2} + \delta_H^{\ 2})^{1/2}$$

dans laquelle

- $d_D$ caractérise les forces de dispersion de LONDON issues de la formation de dipôles induits lors des chocs moléculaires,
- $d_P$ caractérise les forces d'intéractions de DEBYE entre dipôles permanents, et
- $d_H$ caractérise les forces d'intéractions spécifiques (type liaisons hydrogène, acide/base, donneur/accepteur, etc.).

La définition des solvants dans l'espace de solubilité selon Hansen est décrite dans l'article de C.M.Hansen « The three dimensional solubility parameters » J.Paint Technol. 39, 105 (1967).

Parmi les composés liquides organiques, notamment non siliconés ou siliconés, ayant un paramètre de solubilité global selon l'espace de solubilité de Hansen inférieur ou égal à 18 $(Mpa)^{1/2}$, on peut citer des corps gras liquides, notamment des huiles, qui peuvent être choisis parmi les huiles naturelles ou synthétiques, carbonées, hydrocarbonées, fluorées, siliconées, éventuellement ramifiées, seules ou en mélange.

Parmi ces huiles, on peut citer les huiles végétales formées par des esters d'acides gras et de polyols, en particulier les triglycérides, telles que l'huile de tournesol, de sésame ou de colza, ou les esters dérivés d'acides ou d'alcools à longue chaîne (c'est-à-dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates et les benzoates, notamment l'adipate de diisopropyle.

On peut également citer les alcanes linéaires, ramifiés et/ou cycliques éventuellement volatils et notamment des huiles de paraffine, de vaseline, ou le polyisobutylène hydrogéné, l'isododécane, ou encore les 'ISOPARS', les isoparaffines volatiles. On peut citer également les esters, les éthers, les cétones.

On peut encore citer les huiles siliconées telles que les polydiméthylsiloxanes et les polyméthylphénylsiloxanes, éventuellement substitués par des groupements aliphatiques et/ou aromatiques, éventuellement fluorés, ou par des groupements fonctionnels tels que des groupements hydroxyles, thiols et/ou amines, et les huiles siliconées volatiles, notamment cycliques.

En particulier, on peut citer les huiles de silicone, éventuellement ramifiées, volatiles et/ou non volatiles.

Par huile volatile, on entend une huile susceptible de s'évaporer de la peau ou des lèvres en moins d'une heure, ayant notamment une pression de vapeur, à température ambiante et pression atmosphérique allant de $10^{-3}$ à 300 mm de Hg (0,13 Pa à 40 000 Pa).

Comme huile siliconée volatile utilisable dans l'invention, on peut citer les silicones linéaires ou cycliques ayant de 2 à 7 atomes de silicium, ces silicones comportant éventuellement des groupes alkyle ou alkoxy ayant de 1 à 10 atomes de carbone. En particulier, on peut citer l'octaméthylcyclotétrasiloxane, le décaméthylcyclopentasiloxane, le dodécaméthylcyclohexasiloxane, l'heptaméthylhexyltrisiloxane, l'heptaméthyloctyltrisiloxane, l'octaméthyltrisiloxane, le décaméthyltétrasiloxane et leurs mélanges.

Comme huile siliconée non volatile, on peut citer les polydialkylsiloxanes non volatils, tels que les polydiméthylsiloxanes (PDMS) non volatils; les polydiméthylsiloxanes comportant des groupements alkyle, alcoxy ou phényle, pendant ou en bout de chaîne siliconée, groupements ayant de 2 à 24 atomes de carbone; les silicones phénylées comme les phényl triméthicones, les phényl diméthicones, les phényl triméthylsiloxy diphénylsiloxanes, les diphényl diméthicones, les diphényl méthyldiphényl trisiloxanes, les polyméthylphénylsiloxanes; les polysiloxanes modifiés par des acides gras (notamment en $C_8$-$C_{20}$), des alcools gras (notamment en $C_8$-$C_{20}$) ou des polyoxyalkylènes (notamment polyoxyéthyléne et/ou polyoxypropylène); les polysiloxanes aminées ; les polysiloxanes à groupement hydroxyles; les polysiloxanes fluorés comportant un groupement fluoré pendant ou en bout de chaîne siliconée ayant de 1 à 12 atomes de carbone dont tout ou partie des hydrogène sont substitués par des atomes de fluor ; et leurs mélanges.

On peut citer, en particulier, comme composés organiques liquides non siliconés ayant un paramètre de solubilité global selon l'espace de solubilité de Hansen inférieur ou égal à 18 $(Mpa)^{1/2}$:

- les esters linéaires, ramifiés ou cycliques, ayant au moins 6 atomes de carbone, notamment de 6 à 30 atomes de carbone;
- les éthers ayant au moins 6 atomes de carbone, notamment de 6 à 30 atomes de carbone ; et
- les cétones ayant au moins 6 atomes de carbone, notamment de 6 à 30 atomes de carbone.

Par monoalcools liquides ayant un paramètre de solubilité global selon l'espace de solubilité de Hansen inférieur ou égal à 20 $(MPa)^{1/2}$, on entend les monoalcools liquides gras aliphatiques ayant de 6 à 30 atomes de carbone, la chaîne hydrocarbonée ne comportant pas de groupement de substitution. Comme monoalcools selon l'invention, on peut citer l'alcool oléique, le décanol, l'octyldodécanol et l'alcool linoléique.

Selon un premier mode de réalisation de l'invention, la phase grasse liquide peut être une phase grasse liquide non siliconée.

On entend par "phase grasse liquide non siliconée" une phase grasse comprenant un ou plusieurs composés organiques liquides ou huiles non siliconé(e)s, tels que ceux cités précédemment, lesdits composés non siliconés étant présents majoritairement dans la phase grasse liquide, c'est-à-dire à au moins 50 % en poids, notamment de 50 à 100 % en poids, de préférence de 60 % à 100 % en poids (par exemple de 60 à 99 % en poids),
ou encore de 65 % à 100 % en poids (par exemple de 65 à 95 % en poids), par rapport au poids total de la phase grasse liquide.

Les composés organiques liquides non siliconés peuvent être notamment choisis parmi :

- les composés organiques liquides non siliconés ayant un paramètre de solubilité global selon l'espace de solubilité de Hansen inférieur ou égal à 18 $(MPa)^{1/2}$,
- les monoalcools ayant un paramètre de solubilité global selon l'espace de solubilité de Hansen inférieur ou égal à 20 $(MPa)^{1/2}$; et
- leurs mélanges.

Ladite phase grasse liquide non siliconée peut donc éventuellement comprendre des composés organiques liquide ou huiles siliconé(e)s, tels que ceux cités précédemment, qui peuvent être présents en une quantité inférieure à 50 % en poids, notamment allant de 0,1 à 40 % en poids, voire allant de 1 à 35 % en poids, ou encore allant de 5 à 30 % en poids, par rapport au poids total de la phase grasse liquide.

Selon un mode particulier de réalisation de l'invention, la phase grasse liquide non siliconée ne contient pas de

composés organiques liquides ou huiles siliconé(e)s.

Lorsque la phase grasse liquide est une phase grasse liquide non siliconée, les macromonères présents dans le polymère greffé sont avantageusement des macromonomères carbonés tels que décrits ci-après.

En particulier, lorsque la phase grasse liquide est une phase grasse liquide non siliconée, le polymère greffé présent dans la composition est avantageusement un polymère greffé non siliconé.

Par polymère greffé non siliconé, on entend un polymère greffé contenant majoritairement un macromonomère carboné et contenant éventuellement au plus 7 % en poids, de préférence au plus 5 % en poids, voire est exempt, de macromonomère siliconé.

Selon un deuxième mode de réalisation de l'invention, la phase grasse liquide peut être une phase grasse liquide siliconée.

On entend par "phase grasse liquide siliconée" une phase grasse comprenant un ou plusieurs composés organiques liquides siliconés ou huiles siliconées tels que ceux décrits précédemment, lesdits composés siliconés étant présents majoritairement dans la phase grasse liquide, c'est-à-dire à au moins 50 % en poids, notamment de 50 à 100 % en poids, de préférence de 60 % à 100 % en poids (par exemple de 60 à 99 % en poids),
ou encore de 65 % à 100 % en poids (par exemple de 65 à 95 % en poids), par rapport au poids total de la phase grasse liquide.

Les composés organiques liquides siliconés peuvent être notamment choisis parmi :

- les composés organiques liquides, notamment non siliconés ou siliconés, ayant un paramètre de solubilité global selon l'espace de solubilité de Hansen inférieur ou égal à 18 $(MPa)^{1/2}$.

Ladite phase grasse liquide siliconée peut donc éventuellement comprendre des composés organiques liquides ou huiles non siliconé(e)s, tels que décrits précédemment, qui peuvent être présents en une quantité inférieure à 50 % en poids, notamment allant de 0,1 à 40 % en poids, voire allant de 1 à 35 % en poids, ou encore allant de 5 à 30 % en poids, par rapport au poids total da la phase grasse liquide.

Selon un mode particulier de réalisation de l'invention, la phase grasse liquide siliconée ne contient pas de composés organiques liquides non siliconés.

Lorsque la phase grasse liquide est une phase grasse liquide siliconée, les macromonères présents dans le polymère greffé sont avantageusement des macromonomère siliconés tels que décrits ci-après.

En particulier, lorsque la phase grasse liquide est une phase grasse liquide siliconée, le polymère greffé présent dans la composition est avantageusement un polymère greffé siliconé.

Par polymère greffé siliconé, on entend un polymère greffé contenant majoritairement un macromonomère siliconé et contenant éventuellement au plus 7 % en poids, de préférence au plus 5 % en poids, voire est exempt, de macromonomère carboné.

Le choix des monomères constituant le squelette du polymère, des macromonomères, le poids moléculaire du polymère, la proportion des monomères et des macromonomères peut être fait en fonction du milieu organique liquide de dispersion de manière à obtenir avantageusement une dispersion de particules de polymères greffés en particulier une dispersion stable, ce choix pouvant être effectué par l'homme du métier.

Par "dispersion stable", on entend une dispersion qui n'est pas susceptible de former de dépôt solide ou de déphasage liquide/solide notamment après une centrifugation, par exemple, à 4000 tours/minute pendant 15 minutes.

Le polymère éthylénique greffé formant les particules en dispersion comprend donc un squelette insoluble dans ledit milieu de dispersion et une partie soluble dans ledit milieu de dispersion.

Le polymère éthylénique greffé peut être un polymère statistique.

Selon l'invention, on entend par "polymère éthylénique greffé" un polymère susceptible d'être obtenu par polymérisation radicalaire :

- d'un ou plusieurs monomère(s) éthylénique(s),
- avec un ou plusieurs macromonomère(s), dans un milieu organique de polymérisation.

Selon l'invention, on entend par "polymère acrylique greffé " un polymère susceptible d'être obtenu par polymérisation radicalaire :

- d'un ou plusieurs monomère(s) acrylique(s), et éventuellement d'un ou plusieurs monomère(s) additionnel(s) vinylique(s) non acrylique(s),
- avec un ou plusieurs macromonomère(s), dans un milieu organique de polymérisation.

Avantageusement, les monomères acryliques représentent de 50 à 100 % en poids, de préférence de 55 à 100 % en poids (notamment de 55 à 95 % en poids), préférentiellement de 60 à 100 % en poids (notamment de 60 à

90 % en poids) du mélange monomères acryliques + monomères vinyliques non acryliques éventuels.

De préférence, les monomères acryliques sont choisis parmi les monomères dont l'homopolymère est insoluble dans le milieu de dispersion considéré, c'est-à-dire que l'homopolymère est sous forme solide (ou non dissous) à une concentration supérieure ou égale à 5% en poids à température ambiante (20°C) dans ledit milieu de dispersion.

Selon l'invention, on entend par "macromonomère ayant un groupe terminal polymérisable" tout polymère comportant sur une seule de ses extrémités un groupe terminal polymérisable apte à réagir lors de la réaction de polymérisation avec les monomères acryliques et éventuellement les monomères vinyliques non acryliques additionnels constituant le squelette. Le macromonomère permet de former les chaînes latérales du polymère acrylique greffé. Le groupe polymérisable du macromonomère peut être avantageusement un groupe à insaturation éthylénique susceptible de se polymériser par voie radicalaire avec les monomères constituant le squelette.

Par "macromonomère carboné" on entend un macromonomère non siliconé, et notamment un macromonomère oligomère obtenu par polymérisation de monomère(s) non siliconé(s) à insaturation éthylénique, et principalement par polymérisation de monomères acryliques et/ou vinyliques non acryliques.

Par "macromonomère siliconé" on entend un macromonomère organopolysiloxane, et en particulier un macromonomère polydiméthylsiloxane.

De préférence, le macromonomère est choisi parmi les macromonomères dont l'homopolymère est soluble dans le milieu de dispersion considéré, c'est-à-dire complètement dissous à une concentration supérieure ou égale à 5 % en poids et à température ambiante dans ledit milieu de dispersion.

Ainsi, le polymère acrylique greffé comprend un squelette (ou chaîne principale) constitué par un enchaînement de motifs acryliques résultant de la polymérisation notamment d'un ou plusieurs monomères acryliques et des chaînes latérales (ou greffons) issus de la réaction des macromonomères, lesdites chaînes latérales étant liées de manière covalente à ladite chaîne principale.

Le squelette (ou chaîne principale) est insoluble dans le milieu de dispersionconsidéré alors que les chaînes latérales (ou greffons) sont solubles dans ledit milieu de dispersion.

Par "monomère acryliques", on entend dans la présente demande des monomères choisis parmi l'acide (méth)acrylique, les esters de l'acide (méth)acrylique (appelés également les (méth)acrylates), les amides de l'acide (méthacrylique) (appelés également les (méth)acrylamides).

Comme monomère acrylique susceptible d'être employé pour former le squelette insoluble du polymère, on peut citer, seul ou en mélange, les monomères suivants, ainsi que leurs sels :

- (i) les (méth)acrylates de formule :

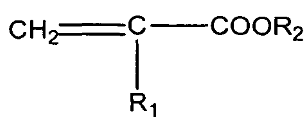

dans laquelle :

- $R_1$ désigne un atome d'hydrogène ou un groupe méthyle ;
- $R_2$ représente un groupe choisi parmi :
  - un groupe alkyle linéaire ou ramifié, comprenant de 1 à 6 atomes de carbone, ledit groupe pouvant comporter dans sa chaîne un ou plusieurs hétéroatomes choisis parmi O, Net S; et/ou pouvant comporter un ou plusieurs substituants choisis parmi -OH, les a tomes d'halogène (F, Cl, Br, I) et - NR'R" avec R ' et R" identiques ou différents choisis parmi les alkyles linéaires ou ramifiés en $C_1$-$C_4$; et/ou pouvant être substitué par au moins un groupe polyoxyalkylène, en particulier avec alkylène en $C_2$-$C_4$, notamment polyoxyéthylène et/ou polyoxypropylène, ledit groupe polyoxyalkylène étant constitué par la répétition de 5 à 30 motifs oxyalkylène;
  - un groupe alkyle cyclique comprenant de 3 à 6 atomes de carbone, ledit groupe pouvant comporter dans sa chaîne un ou plusieurs hétéroatomes choisis parmi O, N et S, et/ou pouvant comporter un ou plusieurs substituants choisis parmi OH et les atomes d'halogène (F, Cl, Br, I);

A titre d'exemples de $R_2$, on peut citer le groupe méthyle, éthyle, propyle, butyle, isobutyle, méthoxyéthyle, éthoxyéthyle, méthoxy-polyoxyéthylène 350 OE , trifluoroéthyle, 2-hydroxyéthyle, 2-hydroxypropyle, diméthylaminoéthyle, diéthylaminoéthyle, diméthylaminopropyle.

- (ii) les (méth)acrylamides de formule :

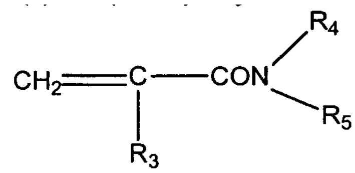

dans laquelle :

- $R_3$ désigne un atome d'hydrogène ou un groupe méthyle ;
- $R_4$ et $R_5$, identiques ou différents, représentent un atome d'hydrogène ou un groupe alkyle, linéaire ou ramifié, comportant de 1 à 6 atomes de carbone, pouvant comporter un ou plusieurs substituants choisis parmi -OH, les atomes d'halogène (F, Cl, Br, I) et -NR'R'' avec R' et R'' identiques ou différents choisis parmi les alkyles linéaires ou ramifiés en $C_1$-$C_4$;ou
- $R_4$ représente un atome d'hydrogène et $R_5$ représente un groupe 1,1-diméthyl-3-oxobutyle.
  A titre d'exemples de groupes alkyles pouvant constituer $R_4$ et $R_5$, on peut citer n-butyle, t-butyle, n-propyle, diméthylaminoéthyle, diéthylaminoéthyle, diméthylaminopropyle.

- (iii) les monomères (méth)acryliques comprenant au moins une fonction acide carboxylique, phosphorique ou sulfonique, tels que l'acide acrylique, l'acide méthacrylique, l'acide acrylamidopropanesulfonique.

Parmi ces monomères acryliques, on peut tout particulièrement citer les (méth)acrylates de méthyle, d'éthyle, de propyle, de butyle, d'isobutyle; les (méth)acrylates de méthoxyéthyle ou d'éthoxyéthyle; le méthacrylate de trifluoroéthyle; le méthacrylate de diméthylaminoéthyle, le méthacrylate de diéthylaminoéthyle, le méthacrylate de 2-hydroxypropyle, le méthacrylate de 2-hydroxyéthyle, l'acrylate de 2-hydroxypropyle, l'acrylate de 2-hydroxyéthyle ; le diméthylaminopropylméthacrylamide; et leurs sels ; et leurs mélanges.

De préférence, les monomères acryliques sont choisis parmi l'acrylate de méthyle, l'acrylate de méthoxyéthyle, le méthacrylate de méthyle, le méthacrylate de 2-hydroxyéthyle, l'acide acrylique, le méthacrylate de diméthylaminoéthyle, et leurs mélanges.

Parmi les monomères additionnels vinyliques non acryliques, on peut citer :

- les esters vinylique de formule : $R_6$-COO-CH=$CH_2$

dans laquelle $R_6$ représente un groupe alkyle linéaire ou ramifié, comprenant de 1 à 6 atomes, ou un groupe alkyle cyclique comportant de 3 à 6 atomes de carbone et/ou un groupe aromatique, par exemple de type benzénique, anthracénique, et naphtalénique ;

- les monomères vinyliques non acryliques comprenant au moins une fonction acide carboxylique, phosphorique ou sulfonique, tels que l'acide crotonique, l'anhydride maléique, l'acide itaconique, l'acide fumarique, l'acide maléique, l'acide styrènesulfonique, l'acide vinylbenzoïque, l'acide vinylphosphorique, et leurs sels ;
- les monomères vinyliques non acryliques comprenant au moins une fonction amine tertiaire, tels que la 2-vinylpyridine, la 4-vinylpyridine ;
- et leurs mélanges.

Avantageusement, les monomères acryliques présents dans le polymère greffés comprennent au moins l'acide (méth)acrylique et au moins un monomère choisi parmi les (méth)acrylates et les (méth)acrylamides décrits précédemment aux points (i) et (ii). De préférence, les monomères acryliques comprennent au moins l'acide (méth)acrylique et au moins un monomère choisi parmi les (méth)acrylates d'alkyle en $C_1$-$C_3$. L'acide (méth)acrylique peut être présent en une teneur d'au moins 5 % en poids, par rapport au poids total du polymère, notamment allant de 5 % à 80 % en poids, de préférence d'au moins 10 % en poids, notamment allant de 10 % en poids à 70 % en poids, préférentiellement d'au moins 15 % en poids, notamment allant de 15 % à 60 % en poids.

Parmi les sels, on peut citer ceux obtenus par neutralisation des groupements acides à l'aide de base inorganiques telles que l'hydroxyde de sodium, l'hydroxyde de potassium, l'hydroxyde d'ammonium ou de bases organiques de type alkanols amines comme la monoéthanolamine, la diéthanolamine, la triéthanolamine, la 2-méthyl-2-amino-1-propanol.

On peut également citer les sels formés par neutralisation des motifs amine tertiaire, par exemple à l'aide d'acide minéral ou organique. Parmi les acides minéraux, on peut citer l'acide sulfurique ou l'acide chlorhydrique, l'acide bromhydrique, iodhydrique, l'acide phosphorique, l'acide borique. Parmi les acides organiques, on peut citer les acides

comportant un ou plusieurs groupes carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyles. On peut notamment citer l'acide acétique o u l'acide p ropionique, l'acide téréphtalique, ainsi que l'acide citrique et l'acide tartrique.

Selon un mode de réalisation de l'invention, le polymère éthylénique greffé ne contient pas de monomères vinyliques non acryliques additionnels tels que décrits précédemment. Dans ce mode de réalisation, le squelette insoluble du polymère éthylénique greffé est formé uniquement de monomères acryliques tels que décrits précédemment.

Il est entendu que ces monomères acryliques non polymérisés peuvent être solubles dans le milieu de dispersion considéré, mais le polymère formé avec ces monomères est insoluble dans le milieu de dispersion.

Selon un mode particulier de réalisation de l'invention, le polymère éthylénique greffé est susceptible d'être obtenu par polymérisation radicalaire dans un milieu organique de polymérisation :

- d'un monomère acrylique principal choisi parmi les (méth)acrylates d'alkyle en $C_1$-$C_3$, seul ou en mélange, et éventuellement d'un ou plusieurs monomères acryliques additionnels choisis parmi l'acide (meth)acrylique, l'acide méthacrylique et les (méth)acrylates d'alkyle de formule (I) définie ci-après, et leurs sels, pour former ledit squelette insoluble ; et
- et d'au moins un macromonomère siliconé comportant un groupe terminal polymérisable, tel que défini précédemment.

Comme monomère acrylique principal, on peut utiliser l'acrylate de méthyle, le méthacrylate de méthyle, l'acrylate d'éthyle, le méthacrylate d'éthyle, l'acrylate de n-propyle, le méthacrylate de n-propyle, l'acrylate d'iso-propyle et le méthacrylate d'isopropyle, et leurs mélanges.

On préfère tout particulièrement l'acrylate de méthyle, le méthacrylate de méthyle, le méthacrylate d'éthyle.

Les monomères acryliques additionnels peuvent être choisis parmi :

- l'acide (méth)acrylique et ses sels,
- les (méth)acrylates de formule (I) et leurs sels :

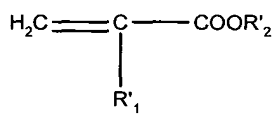

(I)

dans laquelle :

- R'$_1$ désigne un atome d'hydrogène ou un groupe méthyle ;
- R'$_2$ représente
  - un groupe alkyle linéaire ou ramifié comprenant de 1 à 6 atomes de carbone, ledit groupe comportant dans sa chaîne un ou plusieurs atomes d'oxygène et/ou comportant un ou plusieurs substituants choisis parmi
  - OH, les atomes d'halogène (F, Cl, Br, I) et -NR'R" avec R' et R" identiques ou différents choisis parmi les alkyles linéaires ou ramifiés en $C_1$-$C_3$ ;
  - un groupe alkyle cyclique comprenant de 3 à 6 atomes de carbone, ledit groupe pouvant comporter dans sa chaîne un ou plusieurs atomes d'oxygène et/ou pouvant comporter un ou plusieurs substituants choisis parmi OH et les atomes d'halogène (F, Cl, Br, I) ;
- et leurs mélanges.

A titre d'exemples de R'$_2$, on peut citer le groupe méthoxyéthyle, éthoxyéthyle, trifluoroéthyle; 2-hydroxyéthyle, 2-hydroxypropyle, diméthylaminoéthyle, diéthylaminoéthyle, diméthylaminopropyle.

Parmi ces monomères acryliques additionnels, on peut tout particulièrement citer l'acide (méth)acrylique, les (méth)acrylates de méthoxyéthyle ou d'éthoxyéthyle; le méthacrylate de trifluoroéthyle; le méthacrylate de diméthylaminoéthyle, le méthacrylate de diéthylaminoéthyle, le méthacrylate de 2-hydroxypropyle, le méthacrylate de 2-hydroxyéthyle, l'acrylate de 2-hydroxypropyle, l'acrylate de 2-hydroxyéthyle, leurs sels, et leurs mélanges.

On préfère tout particulièrement l'acide acrylique, l'acide méthylacrylique.

Les macromonomères comportent à une des extrémités de la chaîne un groupe terminal polymérisable apte à réagir au cours de la polymérisation avec les monomères acryliques et éventuellement les monomères vinyliques additionnels, pour former les chaînes latérales du polymère éthylénique greffé. Ledit groupe terminal polymérisable peut être en particulier un groupe vinyle ou (méth)acrylate (ou (méth)acryloxy), et de préférence un groupe (méth) acrylate.

Les macromonomères sont choisis préférentiellement parmi les macromonomères dont l'homopolymère a une température de transition vitreuse (Tg) inférieure ou égale à 25°C, notamment allant de - 100°C à 25°C, de préférence allant de - 80°C à 0°C.

Les macromonomères ont une masse moléculaire moyenne en poids supérieure ou égale à 200, de préférence supérieure ou égale à 300, préférentiellement supérieure ou égale à 500, et plus préférentiellement supérieure à 600.

De préférence, les macromonomères ont une masse moléculaire moyenne en poids (Mw) allant de 200 à 100 000, de préférence allant de 500 à 50 000, préférentiellement allant de 800 à 20 000, plus préférentiellement allant de 800 à 10000, et encore plus préférentiellement allant de 800 à 6000.

Dans la présente demande, les masses molaires moyennes en poids (Mw) et en nombre (Mn) sont déterminées par chromatographie liquide par perméation de gel (solvant THF, courbe d'étalonnage établie avec des étalons de polystyrène linéaire, détecteur réfractométrique).

Comme macromonomères carbonés, on peut en particulier citer :

- (i) les homopolymères et les copolymères (méth)acrylate d'alkyle linéaire ou ramifié en C8-C22, présentant un groupe terminal polymérisable choisi parmi les groupes vinyle

  ou (méth)acrylate parmi lesquels on peut citer en particulier : les macromonomères de poly(acrylate d'éthyl-2 hexyle) à extrémité mono(méth)acrylate ; les macromonomères de poly(acrylate de dodécyle) ou de poly(méthacrylate de dodécyle) à extrémité mono(méth)acrylate ; les macromonomères de poly(acrylate de stéaryle) ou de poly (méthacrylate de stéaryle) à extrémité mono(méth)acrylate.

  De tels macromonomères sont notamment décrits dans les brevets EP895467 et EP96459 et dans l'article Gillman K.F., Polymer Letters, Vol 5, page 477-481 (1967).

  On peut en particulier citer les macromonomères à base de poly(acrylate d'éthyl-2-hexyle) ou de poly(acrylate de dodécyle) à extrémité mono(méth)acrylate.
- (ii) les polyoléfines ayant un groupe terminal à insaturation éthylénique , en particulier ayant un groupement terminal (méth)acrylate. Comme exemple de telles polyoléfines, on peut citer en particulier les macromonomères suivants, étant entendu qu'ils ont un groupe terminal (méth)acrylate : les macromonomères de polyéthylène, les macromonomères de polypropylène, les macromonomères de copolymère polyéthylène/polypropylène, les macromonomères de copolymère polyéthylène/polybutylène, les macromonomères de polyisobutylène ; les macromonomères de polybutadiène; les macromonomères de polyisoprène ; les macromonomères de polybutadiène; les macromonomères de poly(éthylène/butylène)-polyisoprène ;

  De tels macromonomères sont en particulier décrits dans US5625005 qui mentionne des macromonomères éthylène/butylène et éthylène/propylène à groupement terminal réactif (méth)acrylate.

On peut en particulier citer le méthacrylate de poly(éthylène/butylène), tel que celui commercialisé sous la dénomination Kraton Liquid L-1253 par Kraton Polymers.

Comme macromonomères siliconés, on peut en particulier citer les polydiméthylsiloxanes à groupement terminal mono (méth)acrylate, et notamment ceux de formule (II) suivante :

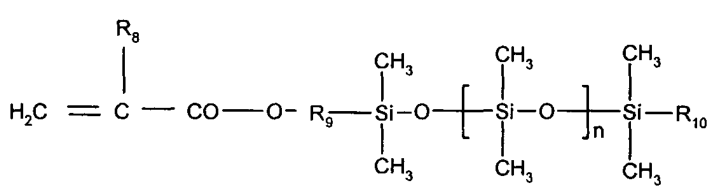

(II)

dans laquelle $R_8$ désigne un atome d'hydrogène ou un groupement méthyle ; $R_9$ désigne un groupe hydrocarboné divalent ayant de 1 à 10 atomes de carbone et contient éventuellement une ou deux liaisons éther -O- ; R10 désigne un groupe alkyl ayant de 1 à 10 atomes de carbone, notamment de 2 à 8 atomes de carbone ; n désigne un nombre entier allant de 1 à 300, de préférence allant de 3 à 200, et préférentiellement allant de 5 à 100.

Comme macromonomères siliconés, on peut utiliser les monométhacryloxypropyl polydiméthylsiloxanes tels que ceux commercialisés sous la dénomination PS560-K6 par la société United Chemical Technologies Inc. (UCT) ou

sous la dénomination MCR-M17 par la société Gelest Inc.

De préférence, le macromonomère polymérisé (constituant les chaînes latérales du polymère greffé) représente de 0,1 à 15 % en poids du poids total du polymère, préférentiellement de 0,2 à 10 % en poids, et plus préférentiellement de 0,3 à 8 % en poids.

Comme polymère éthylénique greffé particulièrement préféré dispersé dans une phase grasse liquide non siliconée, on peut utiliser ceux obtenus par polymérisation :

- de l'acrylate de méthyle et du macromonomère polyéthylène/polybutylène à groupement terminal méthacrylate (notamment Kraton L-1253), en particulier dans un solvant choisi parmi l'isododécane, l'isononanoate d'isononyle, l'octyldodécanol , le malate de diisostéaryle, un benzoate d'alkyl $C_{12}$-$C_{15}$ (tel que Finsolv TN) ;
- de l'acrylate de méthoxyéthyle et du macromonomère polyéthylène/polybutylène à groupement terminal méthacrylate (notamment Kraton L-1253), en particulier dans l'isododécane ;
- des monomères acrylate de méthyle / méthacrylate de méthyle et du macromonomère polyéthylène/polybutylène à groupement terminal méthacrylate (notamment Kraton L-1253), en particulier dans l'isododécane ;
- des monomères acrylate de méthyle / acide acrylique et du macromonomère polyéthylène/polybutylène à groupement terminal méthacrylate (notamment Kraton L-1253), en particulier dans l'isododécane ;
- des monomères acrylate de méthyle / méthacrylate de diméthylaminoéthyle et du macromonomère polyéthylène/polybutylène à groupement terminal méthacrylate (notamment Kraton L-1253), en particulier dans l'isododécane ;
- des monomères acrylate de méthyle / méthacrylate de 2-hydroxyéthyle et du macromonomère polyéthylène/polybutylène à groupement terminal méthacrylate (notamment Kraton L-1253), en particulier dans l'isododécane.

Comme polymère acrylique greffé particulièrement préféré dispersé dans une phase grasse liquide siliconée, on peut utiliser ceux obtenus par polymérisation :

- de l'acrylate de méthyle et du macromonomère monométhacryloylxypropylpolydiméthylsiloxane ayant un poids moléculaire moyen en poids allant de 800 à 6000, en particulier dans le décaméthylcyclopentasiloxane ou le phényltriméthicone ;
- de l'acrylate de méthyle, d'acide acrylique et du macromonomère monométhacryloxypropylpolydiméthylsiloxane ayant un poids moléculaire moyen en poids allant de 800 à 6000, en particulier dans le décaméthylcyclopentasiloxane ou le phényltriméthicone.

De préférence, le polymère greffé a une masse moléculaire moyenne en poids (Mw) comprise entre 10 000 et 300 000, notamment entre 20 000 et 200 000, mieux encore entre 25 000 et 150 000.

Grâce aux caractéristiques susmentionnées, dans un milieu organique de dispersion donné, les polymères ont la capacité de se replier sur eux-mêmes, formant ainsi des particules de forme sensiblement sphérique, avec sur le pourtour de ces particules les chaînes latérales déployées, qui assurent la stabilité de ces particules. De telles particules résultant des caractéristiques du polymère greffé ont la particularité de ne pas s'agglomérer dans ledit milieu et donc de s'autostabiliser et de former une dispersion de particules de polymère particulièrement stable.

En particulier, les polymères éthyléniques greffés de la dispersion peuvent former des particules nanométriques, de taille moyenne allant de 10 à 400 nm, de préférence de 20 à 200 nm.

Du fait de cette taille très faible, les particules de polymère greffé en dispersion sont particulièrement stables et donc peu susceptibles de former des agglomérats.

La dispersion de polymère greffé peut donc être une dispersion stable et ne forme pas de sédiments, lorsqu'elle est placée pendant une durée prolongée (par exemple 24 heures) à température ambiante (25 °C).

De préférence, la dispersion de particules de polymère greffé présente un taux de matière sèche (ou extrait sec) en polymère pouvant aller de 40 % à 70 % en poids de matière sèche, notamment allant de 45 % à 65 % en poids.

On peut préparer la dispersion de particules de polymère greffé par un procédé comprenant une étape de copolymérisation radicalaire, dans un milieu organique de polymérisation, d'un ou plusieurs monomères acryliques tels que définis précédemment avec un ou plusieurs macromonomères tels que définis précédemment.

Comme indiqué précédemment, le milieu organique liquide de dispersion peut être identique ou different du milieu de polymérisation.

D'une manière classique, la copolymérisation peut être effectuée en présence d'un initiateur de polymérisation. Les initiateurs de polymérisation peuvent être des amorceurs radicalaires. De manière générale, un tel initiateur de polymérisation peut être choisi parmi les composés organiques peroxydés tels que le dilauroyl peroxyde, le dibenzoyl peroxyde, le tert-butyl peroxy-2-éthylhexanoate ; les composés diazotés tels que l'azobisisobutyronitrile, l'azobisdiméthylvalero-nitrile.

La réaction peut être également initiée à l'aide de photoinitiateurs ou par une radiation telle que des UV, des neutrons ou par plasma.

D'une manière générale, pour mettre en oeuvre ce procédé, on introduit, dans un réacteur de taille appropriée à la quantité de polymère que l'on va réaliser, au moins une partie du milieu organique de polymérisation, une partie des monomères acryliques et/ou vinyliques additionnels, qui constituera, après polymérisation, le squelette insoluble, la totalité du macromonomère (qui constituera les chaînes latérales du polymère) et une partie de l'initiateur de polymérisation. A ce stade d'introduction, le milieu réactionnel forme un milieu relativement homogène.

Le milieu réactionnel est ensuite agité et chauffé jusqu'à une température pour obtenir une polymérisation des monomères et macromonomères. Après un certain temps, le milieu initialement homogène et limpide conduit à une dispersion d'aspect laiteux. On ajoute ensuite un mélange constitué de la partie restante de monomères et de l'initiateur de polymérisation. Après un temps adéquat pendant lequel le mélange est chauffé sous agitation, le milieu se stabilise sous forme d'une dispersion laiteuse, la dispersion comprenant des particules de polymères stabilisés dans le milieu dans lequel elles ont été créées, ladite stabilisation étant due à la présence, dans le polymère, de chaînes latérales solubles dans ledit milieu de dispersion.

Le polymère greffé peut être présent dans la composition selon l'invention en une teneur en matière sèche (ou matière active) allant de 1 à 70% en poids par rapport au poids total de la composition, mieux de 5 à 60% en poids, de préférence allant de 6 à 45% et mieux allant de 8 à 40% en poids.

La composition selon l'invention peut contenir, à titre d'agent filmogène un polymère éthylénique séquencé linéaire, appelé par la suite "polymère séquencé", de structure particulière telle que décrite ci-après.

Par polymère "séquencé", on entend un polymère comprenant au moins 2 séquences distinctes, de préférence au moins 3 séquences distinctes.

Le polymère est un polymère à structure linéaire. Par opposition, un polymère de structure non linéaire est, par exemple, un polymère à structure ramifiée, en étoile, greffée, ou autre.

Avantageusement, le polymère séquencé est exempt de styrène. Par "polymère exempt de styrène", on entend un polymère contenant moins de 10 % en poids, par rapport au poids total du polymère, de préférence moins de 5 % en poids, mieux moins de 2 % en poids, mieux moins de 1 % en poids, voire ne contenant pas, de monomère styrénique comme le styrène, les dérivés de styrène tels que le méthylstyrène, le chlorostyrène ou le chlorométhylstyrène.

De préférence, le polymère séquencé comprend au moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.

Par "au moins" une séquence, on entend une ou plusieurs séquences.

La séquence intermédiaire est une séquence comprenant au moins un monomère constitutif de la première séquence et au moins un monomère constitutif de la deuxième séquence du polymère permet de "compatibiliser" ces séquences.

On précise que dans ce qui précède et ce qui suit les termes "première" et "deuxième" séquences ne conditionnent nullement l'ordre desdites séquences (ou blocs) dans la structure du polymère séquencé.

Avantageusement, les première et deuxième séquences et du polymère séquencé sont incompatibles l'une avec l'autre.

Par "séquences incompatibles l'une avec l'autre", on entend que le mélange formé du polymère correspondant à la première séquence et du polymère correspondant à la deuxième séquence, n'est pas miscible dans le liquide organique majoritaire en poids du la phase grasse liquide, à température ambiante (25°C) et pression atmosphérique ($10^5$ Pa), pour une teneur du mélange de polymères supérieure ou égale à 5 % en poids, par rapport au poids total du mélange (polymères et solvant), étant entendu que :

i) lesdits polymères sont présents dans le mélange en une teneur telle que le rapport pondéral respectif va de 10/90 à 90/10, et que

ii) chacun des polymères correspondant au première et seconde séquences a une masse moléculaire moyenne (en poids ou en nombre) égale à celle du polymère séquencé +/- 15%.

Dans le cas où la phase grasse liquide comprend un mélange de liquide organiques, et dans l'hypothèse de deux ou plusieurs liquides organiques présents en proportions massiques identiques, ledit mélange de polymères est non miscible dans au moins l'un d'entre eux.

Bien entendu, dans le cas où la phase grasse liquide comprend un unique liquide organique, ce dernier est le liquide organique majoritaire.

De façon préférentielle, le polymère séquencé ne comprend pas d'atomes de silicium dans son squelette. Par "squelette", on entend la chaîne principale du polymère, par opposition aux chaînes latérales pendantes.

De préférence, le polymère séquencé n'est pas soluble dans l'eau ou dans un mélange d'eau et de monoalcools inférieurs linéaires ou ramifiés ayant de 2 à 5 atomes de carbone comme l'éthanol, l'isopropanol ou le n-propanol,

sans modification de pH, à une teneur en matière active d'au moins 1 % en poids, à température ambiante (25°C).

De préférence, le polymère séquencé n'est pas un élastomère.

Par "polymère non élastomère", on entend un polymère qui, lorsqu'il est soumis à une contrainte visant à l'étirer (par exemple de 30% relativement à sa longueur initiale), ne revient pas à une longueur sensiblement identique à sa longueur initiale lorsque cesse la contrainte.

De manière plus spécifique, par "polymère non élastomére" on désigne un polymère ayant une recouvrance instantanée $R_i$ < à 50% et une recouvrance retardée $R_{2h}$ < 70% après avoir subi un allongement de 30%. De préférence, $R_i$ est < à 30 %, et $R_{2h}$ < 50.

Plus précisément, le caractère non élastomérique du polymère est déterminé selon le protocole suivant :

On prépare un film de polymère par coulage d'une solution du polymère dans une matrice téflonnée puis séchage pendant 7 jours dans une ambiance contrôlée à 23±5°C et 50±10 % d'humidité relative.

On obtient alors un film d'environ 100 µm d'épaisseur dans lequel sont découpées des éprouvettes rectangulaires (par exemple à l'emporte-pièce) d'une largeur de 15 mm et d'une longueur de 80 mm.

On impose à cet échantillon une sollicitation de traction à l'aide d'un appareil commercialisé sous la référence Zwick, dans les mêmes conditions de température et d'humidité que pour le séchage.

Les éprouvettes sont étirées à une vitesse de 50 mm/min et la distance entre les mors est de 50 mm, ce qui correspond à la longueur initiale ($l_0$) de l'éprouvette.

On détermine la recouvrance instantanée Ri de la manière suivante :

- on étire l'éprouvette de 30 % ($\varepsilon_{max}$) c'est-à-dire environ 0,3 fois sa longueur initiale ($l_0$)
- on relâche la contrainte en imposant une vitesse de retour égale à la vitesse de traction, soit 50 mm/min et on mesure l'allongement résiduel de l'éprouvette en pourcentage, après retour à contrainte nulle ($\varepsilon_i$).

La recouvrance instantanée en % ($R_i$) est donnée par la formule ci-après:

$$R_i = (\varepsilon_{max} - \varepsilon_i)/ \varepsilon_{max}) \times 100$$

Pour déterminer la recouvrance retardée, on mesure l'allongement résiduel de l'éprouvette en pourcentage ($\varepsilon_{2h}$), 2 heures après retour à la contrainte nulle.

La recouvrance retardée en % ($R_{2h}$) est donnée par la formule ci-après:

$$R_{2h} = (\varepsilon_{max} - \varepsilon_{2h})/\varepsilon_{max}) \times 100$$

A titre purement indicatif, un polymère selon un mode de réalisation de l'invention possède une recouvrance instantanée $R_i$ de 10% et une recouvrance retardée $R_{2h}$ de 30%.

Avantageusement, le polymère séquencé a un indice de polydispersité I supérieur à 2, par exemple allant de 2 à 9, de préférence supérieur ou égal à 2,5, par exemple allant de 2,5 à 8, et mieux supérieur ou égal à 2,8 et notamment, allant de 2,8 à 6.

L'indice de polydispersité I du polymère séquencé est égal au rapport de la masse moyenne en poids Mw sur la masse moyenne en nombre Mn.

On détermine les masses molaires moyennes en poids (Mw) et en nombre (Mn) par chromatographie liquide par perméation de gel (solvant THF, courbe d'étalonnage établie avec des étalons de polystyrène linéaire, détecteur réfractométrique).

La masse moyenne en poids (Mw) du polymère séquencé est de préférence inférieure ou égale à 300 000, elle va par exemple de 35 000 à 200 000, et mieux de 45 000 à 150 000.

La masse moyenne en nombre (Mn) du polymère séquencé est de préférence inférieure ou égale à 70 000, elle va par exemple de 10 000 à 60 000, et mieux de 12 000 à 50 000.

Chaque séquence ou bloc du polymère séquencé est issue d'un type de monomère ou de plusieurs types de monomères différents.

Cela signifie que chaque séquence peut être constituée d'un homopolymère ou d'un copolymère ; ce copolymère constituant la séquence pouvant être à son tour statistique ou alterné.

Avantageusement, la séquence intermédiaire comprenant au moins un monomère constitutif de la première séquence et au moins un monomère constitutif de la deuxième séquence du polymère séuqnecé est un polymère statistique.

De préférence, la séquence intermédiaire est issue essentiellement de monomères constitutifs de la première séquence et de la deuxième séquence.

Par "essentiellement", on entend au moins à 85%, de préférence au moins à 90%, mieux à 95% et encore mieux à 100%.

Avantageusement, la séquence intermédiaire a une température de transition vitreuse Tg comprise entre les températures de transition vitreuse des première et deuxième séquences.

Les températures de transition vitreuse indiquées des première et deuxième séquences peuvent être des Tg théoriques déterminées à partir des Tg théoriques des monomères constitutifs de chacune des séquences, que l'on peut trouver dans un manuel de référence tel que le Polymer H andbook, 3rd ed, 1 989, John Wiley, selon la relation suivante, dite Loi de Fox :

$$1/Tg = \sum_i (\varpi_i / Tg_i) ,$$

$\omega_i$ étant la fraction massique du monomère i dans la séquence considérée et $Tg_i$ étant la température de transition vitreuse de l'homopolymère du monomère i.

Sauf indication contraire, les Tg indiquées pour les première et deuxième séquences dans la présente demande sont des Tg théoriques.

L'écart entre les températures de transition vitreuse des première et deuxième séquences est généralement supérieur à 10°C, de préférence supérieur à 20°C, et mieux supérieur à 30°C.

En particulier, la première séquence du polymère séquencé peut être choisie parmi :

a) une séquence ayant une Tg supérieure ou égale à 40°C,
b) une séquence ayant une Tg inférieure ou égale à 20°C,
c) une séquence ayant une Tg comprise entre 20 et 40°C,

et la deuxième séquence choisie dans une catégorie a), b) ou c) différente de la première séquence.

On entend désigner dans la présente invention, par l'expression :

« compris entre ... et ... », un intervalle de valeurs dont les bornes mentionnées sont exclues, et
« de ... à ... » et « allant de ... à ... », un intervalle de valeurs dont les bornes sont inclues.

<u>a) Séquence ayant une Tg supérieure ou égale à 40°C</u>

La séquence ayant une Tg supérieure ou égale à 40°C a par exemple une Tg allant de 40 à 150°C, de préférence supérieure ou égale à 50°C, allant par exemple de 50°C à 120 °C, et mieux supérieure ou égale à 60°C, allant par exemple de 60°C à 120°C.

La séquence ayant une Tg supérieure ou égale à 40°C peut être un homopolymère ou un copolymère.

Dans le cas où cette séquence est un homopolymère, elle est issue de monomères, qui sont tel(s) que les homopolymères préparés à partir de ces monomères ont des températures de transition vitreuse supérieures ou égales à 40°C. Cette première séquence peut être un homopolymère, constitué par un seul type de monomère (dont la Tg de l'homopolymère correspondant est supérieure ou égale à 40°C).

Dans le cas où la première séquence est un copolymère, elle peut être issue en totalité ou en partie de un ou de plusieurs monomères, dont la nature et la concentration sont choisies de façon que la Tg du copolymère résultant soit supérieure ou égale à 40°C. Le copolymère peut par exemple comprendre :

- des monomères qui sont tel(s) que les homopolymères préparés à partir de ces monomères ont des Tg supérieures ou égales à 40°C, par exemple une Tg allant de 40 à 150 °C, de préférence supérieure ou égale à 50°C, allant par exemple de 50°C à 120°C, et mieux supérieure ou égale à 60°C, allant par exemple de 60°C à 120°C, et
- des monomères qui sont tel(s) que les homopolymères préparés à partir de ces monomères ont des Tg inférieures à 40°C, choisis parmi les monomères ayant une Tg comprise entre 20 à 40°C et/ou les monomères ayant une Tg inférieure ou égale à 20°C, par exemple une Tg allant de -100 à 20°C, de préférence inférieure à 15°C, notamment allant de - 80°C à 15°C et mieux inférieur à 10°C, par exemple allant de -50°C à 0°C à, tels que décrits plus loin, .

Les monomères dont les homopolymères ont une température de transition vitreuse supérieure ou égale à 40°C sont, de préférence, choisis parmi les monomères suivants, appelés aussi monomères principaux :

- les méthacrylates de formule $CH_2 = C(CH_3)\text{-}COOR_1$

dans laquelle $R_1$ représente un groupe alkyle non substitué, linéaire ou ramifié, contenant de 1 à 4 atomes de carbone, tel qu'un groupe méthyle, éthyle, propyle ou isobutyle ou $R_1$ représente un groupe cycloalkyle $C_4$ à $C_{12}$,

- les acrylates de formule $CH_2 = CH\text{-}COOR_2$

dans laquelle $R_2$ représente un groupe cycloalkyle en $C_4$ à $C_{12}$ tel que l'acrylate d'isobornyle ou un groupe tertio butyle,

- les (méth)acrylamides de formule :

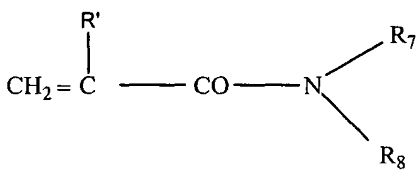

où $R_7$ et $R_8$ identiques ou différents représentent chacun un atome d'hydrogène ou un groupe alkyle en $C_1$ à $C_{12}$ linéaire ou ramifié, tel qu'un groupe n-butyle, t-butyle, isopropyle, isohexyle, isooctyle, ou isononyle ; ou $R_7$ représente H et $R_8$ représente un groupement 1,1-diméthyl-3-oxobutyl,

et R' désigne H ou méthyle. Comme exemple de monomères, on peut citer le N-butylacrylamide, le N-t-butylacrylamide, le N-isopropylacrylamide, le N,N-diméthylacrylamide et le N,N-dibutylacrylamide ,

- et leurs mélanges.

Des monomères principaux particulièrement préférés sont le méthacrylate de méthyle, le (méth)acrylate d'isobutyle, le (méth)acrylate d'isobornyle et leurs mélanges.

b) Séquence ayant une Tg inférieure ou égalé à 20°C

La séquence ayant une Tg inférieure ou égale à 20°C a par exemple une Tg allant de -100 à 20°C, de préférence inférieure ou égale à 15°C, notamment allant de -80°C à 15°C et mieux inférieure ou égale à 10°C, par exemple allant de -50°C à 0°C.

La séquence ayant une Tg inférieure ou égale à 20°C peut être un homopolymère ou un copolymère.

Dans le cas où cette séquence est un homopolymère, elle est issue de monomères, qui sont tel(s) que les homopolymères préparés à partir de ces monomères ont des températures de transition vitreuse inférieures ou égales à 20°C. Cette deuxième séquence peut être un homopolymère, constitué par un seul type de monomère (dont la Tg de l'homopolymère correspondant est inférieure ou égale à 20°C).

Dans le cas où la séquence ayant une Tg inférieure ou égale à 20°C est un copolymère, elle peut être issue en totalité ou en partie de un ou de plusieurs monomères, dont la nature et la concentration sont choisis de façon que la Tg du copolymère résultant soit inférieure ou égale à 20°C.

Elle peut par exemple comprendre

- un ou plusieurs monomères dont l'homopolymère correspondant a une Tg inférieure ou égale à 20°C, par exemple une Tg allant de -100°C à 20 °C, de préférence inférieure à 15°C, notamment allant de - 80°C à 15°C et mieux inférieur à 10°C, par exemple allant de -50°C à 0°C et
- un ou plusieurs monomères dont l'homopolymère correspondant a une Tg supérieure à 20°C, tels que les monomères ayant une Tg supérieure ou égale à 40°C, par exemple une Tg allant de 40 à 150 °C, de préférence supérieure ou égale à 50°C, allant par exemple de 50°C à 120°C, et mieux supérieure ou égale à 60°C, allant par exemple de 60°C à 120°C et /ou les monomère ayant une Tg comprise entre 20 et 40°C, tels que décrits plus haut.

De préférence, la séquence ayant une Tg inférieure ou égale à 20°C est un homopolymère.

Les monomères dont l'homopolymère a une Tg inférieure ou égale à 20°C sont, de préférence, choisis parmi les monomères suivants, ou monomère principaux :

- les acrylates de formule $CH_2 = CHCOOR_3$,
  $R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S,

- les méthacrylates de formule $CH_2 = C(CH_3)\text{-}COOR_4$,
  $R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi 0, N et S;

- les esters de vinyle de formule $R_5\text{-CO-O-CH} = CH_2$

  où $R_5$ représente un groupe alkyle en $C_4$ à $C_{12}$ linéaire ou ramifié ;

- les éthers d'alcool vinylique et d'alcool en $C_4$ à $C_{12}$,

- les N-alkyl en $C_4$ à $C_{12}$ acrylamides, tels que le N-octylacrylamide,

- et leurs mélanges.

Les monomères principaux particulièrement préférés pour la séquence ayant une Tg inférieure ou égale à 20°C sont les acrylates d'alkyles dont la chaîne alkyle comprend de 1 à 10 atomes de carbone, à l'exception du groupe tertiobutyle, tels que l'acrylate de méthyle, l'acrylate d'isobutyle, l'acrylate d'éthyl-2 hexyle et leurs mélanges.

c) Séquence ayant une Tg comprise entre 20 et 40°C

La séquence qui a une Tg comprise entre 20 et 40°C peut être un homopolymère ou un copolymère.

Dans le cas où cette séquence est un homopolymère, elle est issue de monomères (ou monomère principaux), qui sont tel(s) que les homopolymères préparés à partir de ces monomères ont des températures de transition vitreuse comprises entre 20 et 40°C. Cette première séquence peut être un homopolymère, constitué par un seul type de monomère (dont la Tg de l'homopolymère correspondant va de 20°C à 40°C).

Les monomères dont l'homopolymère a une température de transition vitreuse comprise entre 20 et 40°C sont, de préférence, choisis parmi le méthacrylate de n-butyle, l'acrylate de cyclodécyle, l'acrylate de néopentyle, l'isodécylacrylamide et leurs mélanges.

Dans le cas où la séquence ayant une Tg comprise entre 20 et 40°C est un copolymère, elle est issue en totalité ou en partie de un ou de plusieurs monomères (ou monomère principaux), dont la nature et la concentration sont choisis de telle sorte que la Tg du copolymère résultant soit comprise entre 20 et 40°C.

Avantageusement, la séquence ayant une Tg comprise entre 20 et 40°C est un copolymère issue en totalité ou en partie :

- de monomères principaux dont l'homopolymère correspondant a une Tg supérieure ou égale à 40°C, par exemple une Tg allant de 40°C à 150°C, de préférence supérieure ou égale à 50°C, allant par exemple de 50 à 120°C, et mieux supérieure ou égale à 60°C, allant par exemple de 60°C à 120°C, tels que décrits plus haut, et/ou
- de monomères principaux dont l'homopolymère correspondant a une Tg inférieure ou égale à 20°C, par exemple une Tg allant de -100 à 20°C, de préférence inférieure ou égale à 15°C, notamment allant de -80°C à 15°C et mieux inférieure ou égale à 10°C, par exemple allant de -50°C à 0°C, tels que décrits plus haut,

lesdits monomères étant choisis de telle sorte que la Tg du copolymère formant la première séquence est comprise entre 20 et 40°C.

De tels monomères principaux sont par exemple choisis parmi le méthacrylate de méthyle, l'acrylate et le méthacrylate d'isobornyle, l'acrylate de butyle, l'acrylate d'éthyl-2 hexyle et leurs mélanges.

De préférence, la proportion de la deuxième séquence ayant une Tg inférieure ou égale à 20°C va de 10 à 85% en poids du polymère, mieux de 20 à 70% et encore mieux de 20 à 50%.

Chacune des séquences peut néanmoins contenir en proportion minoritaire au moins un monomère constitutif de l'autre séquence.

Ainsi la première séquence peut contenir au moins un monomère constitutif de la deuxième séquence et inversement.

Chacune des première et/ou deuxième séquence du polymère séquencé peu(ven)t comprendre, outre les monomères indiqués ci-dessus, un ou plusieurs autres monomères appelés monomères additionnels, différents des monomères principaux cités précédemment.

La nature et la quantité de ce ou ces monomères additionnels sont choisies de manière à ce que la séquence dans laquelle ils se trouvent ait la température de transition vitreuse désirée.

Ce monomère additionnel est par exemple choisi parmi :

les monomères hydrophiles tels que :

- les monomères à insaturation(s) éthylénique(s) comprenant au moins une fonction acide carboxylique ou sulfonique comme par exemple :

  l'acide acrylique, l'acide méthacrylique, l'acide crotonique, l'anhydride maléique, l'acide itaconique, l'acide fumarique, l'acide maléique, l'acide acrylamidopropanesulfonique, l'acide vinylbenzoïque, l'acide vinylphosphorique et les sels de ceux-ci,

- les monomères à insaturation(s) éthylénique(s) comprenant au moins une fonction amine tertiaire comme la 2-vinylpyridine, la 4-vinylpyridine, le méthacrylate de diméthylaminoéthyle, le méthacrylate de diéthylaminoéthyle, le diméthylaminopropyl méthacrylamide et les sels de ceux-ci,
- les méthacrylates de formule $CH_2 = C(CH_3)\text{-}COOR_6$

  dans laquelle $R_6$ représente un groupe alkyle linéaire ou ramifié, contenant de 1 à 4 atomes de carbone, tel qu'un groupe méthyle, éthyle, propyle ou isobutyle, ledit groupe alkyle étant substitué par un ou plusieurs substituants choisis parmi les groupes hydroxyle (comme le méthacrylate de 2-hydroxypropyle, le méthacrylate de 2-hydroxyéthyle) et les atomes d'halogènes (Cl, Br, I, F), tel que le méthacrylate de trifluoroéthyle,
- les méthacrylates de formule $CH_2 = C(CH_3)\text{-}COOR_9$,

  $R_9$ représentant un groupe a lkyle en $C_6$ à $C_{12}$ linéaire ou ramifié, dans lequel se trouve(nt) éventuellement intercalé(s) un ou plusieurs hétéroatomes choisis parmi 0, N et S, ledit groupe alkyle étant substitué par un ou plusieurs substituants choisis parmi les groupes hydroxyle et les atomes d'halogènes (Cl, Br, I, F) ;
- les acrylates de formule $CH_2 = CHCOOR_{10}$,

  $R_{10}$ représentant un groupe alkyle en $C_1$ à $C_{12}$ linéaire ou ramifié substitué par un ou plusieurs substituants choisis parmi les groupes hydroxyle et les atomes d'halogène (Cl, Br, I et F), tel que l'acrylate de 2-hydroxypropyle et l'acrylate de 2-hydroxyéthyle, ou $R_{10}$ représente un alkyle en $C_1$ à $C_{12}$-O-POE (polyoxyéthylène) avec répétition du motif oxyéthylène de 5 à 30 fois, par exemple méthoxy-POE, ou $R_8$ représente un groupement polyoxyéthylèné comprenant de 5 à 30 motifs d'oxyde d'éthylène

  b) les monomères à insaturation éthylénique comprenant un ou plusieurs atomes de silicium tels que le méthacryloxypropyl triméthoxy silane, le méthacryloxypropyl tris (triméthylsiloxy) silane,
- et leurs mélanges.

Des monomères additionnels particulièrement préférés sont l'acide acrylique, l'acide méthacrylique, le méthacrylate de trifluoroéthyle et leurs mélanges.

Selon un mode préféré de réalisation, le polymère séquencé est un polymère non siliconé, c'est à dire un polymère exempt d'atome de silicium.

Ce ou ces monomères additionnels représente(nt) généralement une quantité inférieure ou égale à 30% en poids, par exemple de 1 à 30% en poids, de préférence de 5 à 20% en poids et, de préférence encore, de 7 à 15% en poids du poids total des première et/ou deuxième séquences.

De préférence, chacune des première et deuxième séquences comprend au moins un monomère choisi parmi les esters d'acide (méth)acrylique, et éventuellement au moins un monomère choisi parmi l'acide (méth)acrylique, et leurs mélanges.

Avantageusement, chacune des première et deuxième séquences du polymère séquencé est issue en totalité d'au moins un monomère choisi parmi l'acide acrylique, les esters d'acide (méth)acrylique, et éventuellement d'au moins un monomère choisi parmi l'acide (méth)acrylique, et leurs mélanges

Le polymère séquencé peut être obtenu par polymérisation radicalaire en solution selon le procédé de préparation suivant :

- une partie du solvant de polymérisation est introduite dans un réacteur adapté et chauffée jusqu'à atteindre la température adéquate pour la polymérisation (typiquement entre 60 et 120°C),

- une fois cette température atteinte, les monomères constitutifs de la première séquence sont introduits en présence d'une partie de l'initiateur de polymérisation,
- au bout d'un temps T correspondant à un taux de conversion maximum de 90%, les monomères constitutifs de la deuxième séquence et l'autre partie de l'initiateur sont introduits,
- on laisse réagir le mélange pendant un temps T' (allant de 3 à 6 h) au bout duquel le mélange est ramené à température ambiante,
- on obtient le polymère en solution dans le solvant de polymérisation.

Par solvant de polymérisation, on entend un solvant ou un mélange de solvants. Le solvant de polymérisation peut être choisis notamment parmi l'acétate d'éthyle, l'acétate de butyle, les alcools tels que l'isopropanol, l'éthanol, les alcanes aliphatiques tels que l'isododécane et leurs mélanges. De préférence, le solvant de polymérisation est un mélange acétate de butyle et isopropanol ou l'isododécane.

Selon un premier mode de réalisation, le polymère séquencé comprend une première séquence ayant une Tg supérieure ou égale à 40°C, telle que décrite plus haut au a) et une deuxième séquence ayant une Tg inférieure ou égale à 20°C, telle que décrite plus haut au b).

De préférence, la première séquence ayant une Tg supérieure ou égale à 40°C est un copolymère issu de monomères qui sont tels que l'homopolymère préparé à partir de ces monomères a une température de transition vitreuse supérieure ou égale à 40°C, tels que les monomère décrits plus haut.

Avantageusement, la deuxième séquence ayant une Tg inférieure ou égale à 20°C est un homopolymère issu de monomères qui sont tel(s) que l'homopolymère préparé à partir de ces monomères a une température de transition vitreuse inférieure ou égale à 20°C, tels que les monomères décrits plus haut.

De préférence, la proportion de la séquence ayant une Tg supérieure ou égale à 40°C va de 20 à 90% en poids du polymère, mieux de 30 à 80% et encore mieux de 50 à 70%.

De préférence, la proportion de la séquence ayant une Tg inférieure ou égale à 20°C va de 5 à 75% en poids du polymère, de préférence de 15 à 50% et mieux de 25 à 45%.

Avantageusement, le polymère séquencé peut comprendre :

- une première séquence de Tg supérieure ou égale à 40°C, par exemple allant de 85 à 115°C, qui est un copolymère acrylate d'isobornyle/méthacrylate d'isobutyle,
- une deuxième séquence de Tg inférieure ou égale à 20°C, par exemple allant de -85 à -55°C, qui est un homopolymère d'acrylate d'éthyl-2 hexyle et
- une séquence intermédiaire qui est un copolymère statistique acrylate d'isobornyle/méthacrylate d'isobutyle/acrylate d'éthyl-2 hexyle.

Selon un second mode de réalisation, le polymère séquencé comprend une première séquence ayant une température de transition vitreuse (Tg) comprise entre 20 et 40°C, conforme aux séquences décrites au c) et une deuxième séquence ayant une température de transition vitreuse inférieure ou égale à 20°C, telle que décrite plus haut au b) ou une température de transition vitreuse supérieure ou égale à 40°C, t elle que décrite au a) ci-dessus.

De préférence, la proportion de la première séquence ayant une Tg comprise entre 20 et 40°C va de 10 à 85% en poids du polymère, mieux de 30 à 80% et encore mieux de 50 à 70%.

Lorsque la deuxième séquence est une séquence ayant une Tg supérieure ou égale à 40°C, elle est de préférence présente en une proportion allant de 10 à 85% en poids du polymère, mieux de 20 à 70% et encore mieux de 30 à 70%.

Lorsque la deuxième séquence est une séquence ayant une Tg inférieure ou égale à 20°C, elle est de préférence présente en une proportion allant de 10 à 85% en poids du polymère, mieux de 20 à 70% et encore mieux de 20 à 50%.

De préférence, la première séquence ayant une Tg comprise entre 20 et 40°C est un copolymère issu de monomères qui sont tel(s) que l'homopolymère correspondant a une Tg supérieure ou égale à 40°C et de monomères qui sont tel(s) que l'homopolymère correspondant a une Tg inférieure ou égale à 20°C.

. Avantageusement, la deuxième séquence ayant une Tg inférieure ou égale à 20°C ou ayant une Tg supérieure ou égale à 40°C est un homopolymère.

Selon une première variante, le polymère séquencé comprend :

- une première séquence de Tg comprise entre 20 et 40°C, par exemple ayant une Tg de 21 à 39°C, qui est un copolymère comprenant acrylate d'isobornyle/méthacrylate d'isobutyle/acrylate d'éthyl-2 hexyle,
- une deuxième séquence de Tg inférieure ou égale à 20°C, par exemple allant de -65 à -35°C, qui est un homopolymère de méthacrylate de méthyle et
- une séquence intermédiaire qui est un copolymère statistique acrylate d'isobornyle /méthacrylate d'isobutyle/ acrylate d'éthyl-2 hexyle.

Selon une deuxième variante, le polymère séquencé peut comprendre :

- une première séquence de Tg supérieure ou égale à 40°C, par exemple allant de 85 à 115°C, qui est un copolymère méthacrylate d'isobornyle/méthacrylate d'isobutyle,
- une deuxième séquence de Tg inférieure ou égale à 20°C, par exemple allant de -35 à -5°C, qui est un homopolymère d'acrylate d'isobutyle et
- une séquence intermédiaire qui est un copolymère statistique méthacrylate d'isobornyle/méthacrylate d'isobutyle/ acrylate d'isobutyle.

Selon une troisième variante, le polymère séquencé peut comprendre :

- une première séquence de Tg supérieure ou égale à 40°C, par exemple allant de 60 à 90°C, qui est un copolymère acrylate d'isobornyle/méthacrylate d'isobutyle,
- une deuxième séquence de Tg inférieure ou égale à 20°C, par exemple allant de -35 à -5°C, qui est un homopolymère d'acrylate d'isobutyle et
- une séquence intermédiaire qui est un copolymère statistique acrylate d'isobornyle/méthacrylate d'isobutyle/ acrylate d'isobutyle.

Dans un mode de réalisation, l'agent filmogène est un polymère organique filmogène soluble dans la phase grasse liquide.
Lorsque la phase grasse liquide de la composition comprend au moins une huile, l'agent filmogène peut être un polymère soluble dans ladite huile. Dans ce cas, on parle de polymère liposoluble. Le polymère liposoluble peut être d'un type chimique quelconque et peut être notamment choisi parmi:

a) les homopolymères et les copolymères liposolubles et amorphes des oléfines, des cyclooléfines, du butadiène, de l'isoprène, du styrène, des éthers, des esters ou amides vinyliques, des esters ou amides de l'acide (méth) acrylique contenant un groupement alkyle en $C_{4-50}$ linéaire, ramifié ou cyclique, et préférablement amorphes. Les homopolymères et les copolymères liposolubles préférés sont obtenus à partir de monomères choisis parmi le groupe constitué par le (méth)acrylate d'isooctyle, le (méth)acrylate d'isononyle, le (méth)acrylate de 2-éthylhexyle, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth)acrylate de tridécyle, le (méth) acrylate de stéaryle, ou des mélanges de ceux-ci. On citera, par exemple, le copolymère d'acrylate d'alkyle/acrylate de cycloalkyle commercialisé par PHOENIX CHEM. sous la dénomination GIOVAREZ AC-5099 ML, et les copolymères de vinylpyrrolidone, tels que les copolymères d'un alkène en $C_2$ à $C_{30}$, tel qu'en $C_3$ à $C_{22}$, et des associations de ceux-ci, peuvent être utilisés. Comme exemples de copolymères de VP pouvant être utilisés dans l'invention, on peut citer le copolymère de VP/laurate de vinyle, de VP/stéarate de vinyle, la polyvinylpyrrolidone (PVP) butylée, de VP/hexadécène, de VP/triacontène ou de VP/acide acrylique/méthacrylate de lauryle.

Comme copolymères liposolubles particuliers, on peut citer :

i) les polymères de silicone-acrylique greffés ayant un squelette siliconé, des greffons acryliques ou ayant un squelette acrylique, les greffons de silicone tels que le produit commercialisé sous la dénomination SA 7 0.5 par 3M et décrit dans les brevets US 5 725 882, US 5 209 924, US 4 972 037, US 4 981 903, US 4 981 902, US 5 468 477, et dans les brevets US 5 219 560 et EP 0 388 582.

ii) les polymères liposolubles portant des groupements fluorés appartenant à l'une des classes décrites dans le texte ci-dessus, en particulier ***Fomblin*** ceux décrits dans le brevet US 5 948 393, les copolymères de (méth) acrylate d'alkyle/(méth)acrylate de perfluoroalkyle décrits dans les brevets EP 0 815 836 et US 5 849 318.

iii) les polymères ou copolymères résultant de la polymérisation ou la copolymérisation d'un monomère éthylénique, comprenant une ou plusieurs liaisons éthyléniques, de préférence conjuguées (ou diènes). Comme polymères ou copolymères résultant de la polymérisation ou la copolymérisation d'un monomère éthylénique, on peut utiliser des copolymères vinyliques, acryliques ou méthacryliques.

Dans un mode de réalisation, l'agent filmogène est un copolymère bloc comprenant au moins un bloc constitué de motifs styrène ou dérivés du styrène (par exemple le méthylstyrène, le chlorostyrène ou le chlorométhylstyrène). Le copolymère comprenant au moins un bloc styrène peut être un copolymère dibloc ou tribloc, voire un copolymère multibloc, en étoile ou radial. Le copolymère comprenant au moins un bloc styrène peut comprendre en outre, par exemple, un bloc alkylstyrène (AS), un bloc éthylène/butylène (EB) un bloc éthylène/ propylène (EP), un bloc butadiène (B), un bloc isoprène (I), un bloc acrylate (A), un bloc méthacrylate (MA) ou une association de ces blocs. Le copolymère comprenant au moins un bloc constitué de motifs styrène ou dérivés du styrène peut être un copolymère dibloc ou tribloc, et en particulier du type polystyrène/polyisoprène ou polystyrène/polybutadiène, tels que ceux commercialisés ou fabriqués sous la dénomination « Luvitol HSB » par BASF et ceux du type polystyrène/copoly(éthylène-propylène) ou de manière alternative du type polystyrène/copoly(éthylène/butylène), tels que ceux commercialisés ou fabriqués sous la marque de fabrique « Kraton » par Shell Chemical Co. ou Gelled Permethyl 99A par Penreco, peuvent être utilisés.

On peut citer par exemple le le Kraton G1650 (SEBS), le Kraton G1651 (SEBS), le Kraton G1652 (SEBS), le Kraton G1657X (SEBS), le Kraton G1701X (SEP), le Kraton G1702X (SEP), le Kraton G1726X (SEB), le Kraton D-1101 (SBS), le K raton D-1102 (SBS), le Kraton D-1107 (SIS), le Gelled Permethyl 99A-750, le Gelled Permethyl 99A-753-58 (mélange de polymère bloc en étoile et de polymère tribloc), le Gelled Permethyl 99A-753-59 (mélange de polymère bloc en étoile et de polymère tribloc), le Versagel 5970 et le Versagel 5960 de chez Penreco (mélange de polymère en étoile et de polymère tribloc dans l'isododécane).

Des copolymères de styrène-méthacrylate peuvent également être utilisés tels que les polymère commercialisés sous les référenceq OS 129880, OS 129881 et OS 84383 de chez Lubrizol (copolymère de styrène-méthacrylate).

Dans un mode de réalisation, l'agent filmogène est choisi parmi les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester) et d'au moins un autre monomère qui peut être un ester vinylique (différent de l'ester vinylique déjà présent), une $\alpha$-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être partiellement réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les copolymères liposolubles, et en particulier ceux résultant de copolymérisation d'esters vinyliques ayant de 9 à 22 atomes de carbone ou d'acrylates ou de méthacrylates d'alkyle, les radicaux alkyles ayant de 10 à 20 atomes de carbone.

De tels copolymères liposolubles peuvent être choisis parmi les copolymères de polystéarate de vinyle, de polystéarate de vinyle réticulé à l'aide de divinylbenzène, de diallyléther ou de phtalate de diallyle, les copolymères de poly(méth)acrylate de stéaryle, de polylaurate de vinyle, de poly(méth)acrylate de lauryle, ces poly(méth)acrylates pouvant être réticulés à l'aide de diméthacrylate de l'éthylène glycol ou de tétraéthylène glycol.

Les copolymères liposolubles définis précédemment sont connus et notamment décrits dans la demande FR-A-2232303 ; ils peuvent avoir un poids moléculaire moyen en poids allant de 2.000 à 500.000 et de préférence de 4.000 à 200.000.

Comme exemples de polymères liposolubles pouvant être utilisés dans l'invention, on peut citer les polyalkylènes, les copolymères d'alcènes en $C_2$-$C_{20}$, en particulier le polybutène.

b) les polycondensats amorphes et liposolubles, de préférence ne comprenant pas de groupements donneurs d'interactions hydrogène, en particulier les polyesters aliphatiques ayant des chaînes latérales alkyle en $C_{4-50}$ ou bien les polyesters résultant de la condensation de dimères d'acides gras, voire les polyesters comprenant un segment siliconé sous la forme d'une séquence, greffon ou groupement terminal, tel que défini dans la demande

de brevet FR 0 113 920.

c) les polysaccharides amorphes et liposolubles comprenant des chaînes latérales alkyl (éther ou ester), en particulier les alkylcelluloses ayant un radical alkyle en $C_1$ à $C_8$ saturé ou insaturé, linéaire ou ramifié, tel que l'éthylcellulose et la propylcellulose.

Le polymère filmogène peut être choisi en particulier parmi les polymères cellulosiques tels que la nitrocellulose, l'acétate de cellulose, l'acétobutyrate de cellulose, l'acétopropionate de cellulose, l'éthyl cellulose, ou bien encore les polyuréthanes, les polymères acryliques, les polymères vinyliques, les polyvinylbutyrals, les résines alkydes, les résines issues des produits de condensation d'aldéhyde tels que les résines arylsulfonamide formaldéhyde comme la résine toluène sulfonamide formaldéhyde, les résines aryl-sulfonamide époxy.

Comme polymère filmogène, on peut notamment utiliser la nitrocellulose RS 1/8 sec ; RS ¼ sec. ; ½ sec. ; RS 5 sec. ; RS 15 sec. ; RS 35 sec. ; RS 75 sec.; RS 150 sec ; AS ¼ sec. ; AS ½ sec. ; SS ¼ sec. ; SS ½ sec. ; SS 5 sec., notamment commercialisée par la société HERCULES ; les résine toluène sulfonamide formaldéhyde "Ketjentflex MS80" de la société AKZO ou "Santolite MHP", " Santolite MS 80" de la société FACONNIER ou "RESIMPOL 80" de la société PAN AMERICANA, la résine alkyde "BECKOSOLODE 230-70-E" de la société DAINIPPON, la résine acrylique "ACRYLOID B66" de la société ROHM & HAAS, la résine polyuréthane "TRIXENE PR 4127" de la société BAXENDEN.

d) les résines de silicone, généralement solubles ou gonflables dans les huiles de silicone. Ces résines sont des polymères de polyorganosiloxanes réticulés.

La nomenclature des résines de silicone est connue sous le nom de "MDTQ", la résine étant décrite en fonction des différentes unités monomèriques siloxane qu'elle comprend, chacune des lettres "MDTQ" caractérisant un type d'unité.

La lettre M représente l'unité monofonctionelle de formule $(CH_3)_3SiO_{1/2}$, l'atome de silicium étant relié à un seul atome d'oxygène dans le polymère comprenant cette unité.

La lettre D signifie une unité difonctionnelle $(CH_3)_2SiO_{2/2}$ dans laquelle l'atome de silicium est relié à deux atomes d'oxygène

La lettre T représente une unité trifonctionnelle de formule $(CH_3)SiO_{3/2}$.

Dans les motifs M, D, T définis précédemment, au moins un des groupes méthyles peut être substitués par un groupe R différent du groupe méthyle tel qu'un radical hydrocarboné (notamment alkyle) ayant de 2 à 10 atomes de carbone ou un groupe phényl ou bien encore un groupe hydroxyle.

Enfin, la lettre Q signifie une unité tetrafonctionnelle $SiO_{4/2}$ dans laquelle l'atome de silicium est lié à quatre atomes d'hydrogène eux mêmes liés au reste du polymère.

Divers résines de propriétés différentes peuvent être obtenues à partir de ces différentes unités, les propriétés des ces polymères variant en fonction du type de monomères (ou unités), du type et du nombre de radicaux subsitués, de la longueur de la chaîne polymérique, du degré de ramification et de la taille des chaines pendantes.

A titre d'exemple de ces résines silicones, on peut citer :

- les siloxysilicates qui peuvent être des triméthylsiloxysilicate de formule $[(CH_3)_3XSiXO]_xX(SiO_{4/2})_y$ (unités MQ) dans laquelle x et y sont des entiers allant de 50 à 80,
- les polysilesquioxanes de formule $(CH_3SiO_{3/2})_x$ (unités T) dans laquelle x est supérieur à 100 et dont au moins un des radicaux méthyle peut être substitué par un groupement R tel que défini plus haut,
- les polymethylsilsesquioxanes qui sont des polysilsesquioxanes dans lesquels aucun des radicaux méthyle n'est substitué par un autre groupement. De tels polymethylsilsesquioxanes sont décrits dans le document US 5,246,694 dont le contenu est incorporé par référence.

A titre d'exemples de résines polymethylsilsesquioxanes commercialement disponibles, on peut citer celles qui sont commercialisés :

- par la société Wacker sous la référence Resin MK tels que la Belsil PMS MK : polymère comprenant des unités répétitives $CH_3SiO_{3/2}$ (unités T), pouvant aussi comprendre jusqu'à 1% en poids d'unités $(CH_3)_2SiO_{2/2}$ (unités D) et présentant un poids moléculaire moyen d'environ 10000,
- par la société SHIN-ETSU sous les références KR-220L qui sont composé d'unités T de formule $CH_3SiO_{3/2}$ et ont des groupes terminaux Si-OH (silanol), sous la référence KR-242A qui comprennent 98% d'unités T et 2% d'unités diméthyle D et ont des groupes terminaux Si-Ohou encore sous la référence KR-251 comprenant 88% d'unités T et 12% d'unités dimethyl D et ont des groupes terminaux Si-OH.

Comme résines siloxysilicates, on peut citer les résines trimethylsiloxysilicate (TMS) éventuellement sous forme de poudres. De telles résines sont commercialisées sous la référence SR1000 par la société General Electric ou sous la référence TMS 803 par la société Wacker. On peut encore citer les résines timéthylsiloxysilicate commercialisées dans un solvant tel que la cyclomethicone, vendues sous la dénomination "KF-7312J" par la société Shin-Etsu, "DC 749", "DC 593" par la société Dow Corning.

e) Les polyamides siliconés du type polyorganosiloxane tels que ceux décrits dans les documents US-A-5 874 069, US-A-5,919,441, US-A-6,051,216 et US-A-5,981,680.

Selon l'invention, ces polymères siliconés peuvent appartenir aux deux familles suivantes :

1) des polyorganosiloxanes comportant au moins deux groupes capables d'établir des interactions hydrogène, ces deux groupes étant situés dans la chaîne du polymère, et/ou
2) des polyorganosiloxanes comportant au moins deux groupes capables d'établir des interactions hydrogène, ces deux groupes étant situés sur des greffons ou ramifications.

Les polymères comportant deux groupes capables d'établir des interactions hydrogène dans la chaîne du polymère peuvent être des polymères comprenant au moins un motif répondant à la formule :

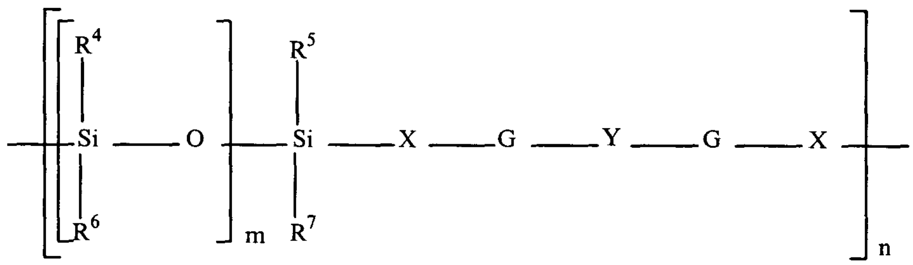

(II)

dans laquelle :

1) $R^4$, $R^5$, $R^6$ et $R^7$, identiques ou différents, représentent un groupe choisi parmi :

- les groupes hydrocarbonés, linéaires, ramifiés ou cycliques, en $C_1$ à $C_{40}$, saturés

ou insaturés, pouvant contenir dans leur chaîne un ou plusieurs atomes d'oxygène, de soufre et/ou d'azote, et pouvant être substitués en partie ou totalement par des atomes de fluor,

- les groupes aryles en $C_6$ à $C_{10}$, éventuellement substitués par un ou plusieurs groupes alkyle en $C_1$ à $C_4$,
- les chaînes polyorganosiloxanes contenant ou non un ou plusieurs atomes d'oxygène, de soufre et/ou d'azote,

2) les X, identiques ou différents, représentent un groupe alkylène di-yle, linéaire ou ramifié en $C_1$ à $C_{30}$, pouvant contenir dans sa chaîne un ou plusieurs atomes d'oxygène et/ou d'azote,
3) Y est un groupe divalent alkylène linéaire ou ramifié, arylène, cycloalkylène, alkylarylène ou arylalkylène, saturé ou insaturé, en $C_1$ à $C_{50}$, pouvant comporter un ou plusieurs atomes d'oxygène, de soufre et/ou d'azote, et/ou porter comme substituant l'un des atomes ou groupes d'atomes suivants : fluor, hydroxy, cycloalkyle en $C_3$ à $C_8$, alkyle en $C_1$ à $C_{40}$, aryle en $C_5$ à $C_{10}$, phényle éventuellement substitué par 1 à 3 groupes alkyle en $C_1$ à $C_3$, hydroxyalkyle en $C_1$ à $C_3$ et amino alkyle en $C_1$ à $C_6$, ou
4) Y représente un groupe répondant à la formule :

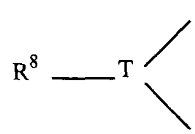

dans laquelle

- T représente un groupe hydrocarboné trivalent ou tétravalent, linéaire ou ramifié, saturé ou insaturé, en $C_3$ à $C_{24}$ éventuellement substitué par une chaîne polyorganosiloxane, et pouvant contenir un ou plusieurs atomes choisis parmi O, N et S,

ou T représente un atome trivalent choisi parmi N, P et Al, et

- $R^8$ représente un groupe alkyle en $C_1$ à $C_{50}$, linéaire ou ramifié, ou une chaîne polyorganosiloxane, pouvant comporter un ou plusieurs groupes ester, amide, uréthane, thiocarbamate, urée, thiourée et/ou sulfonamide qui peut être lié ou non à une autre chaîne du polymère,

5) les G, identiques ou différents, représentent les groupes divalents choisis parmi :

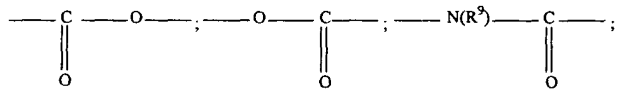

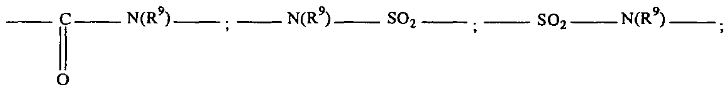

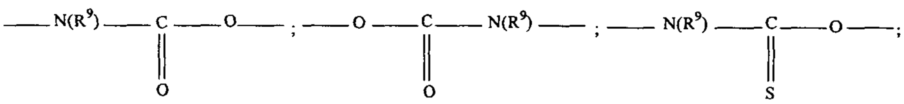

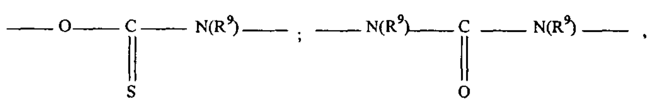

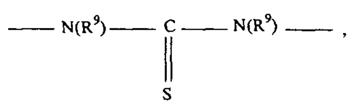

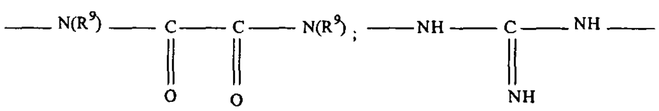

; et

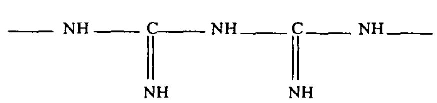

où $R^9$ représente un atome d'hydrogène ou un groupe alkyle, linéaire ou ramifié, en $C_1$ à $C_{20}$, à condition

qu'au moins 50% des $R^9$ du polymère représente un atome d'hydrogène et qu'au moins deux des groupes G du polymère soient un autre groupe que :

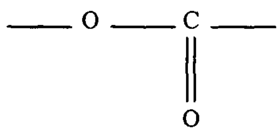 et 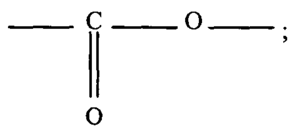

6) n est un nombre entier allant de 2 à 500, de préférence de 2 à 200, et m est un nombre entier allant de 1 à 1000, de préférence de 1 à 700 et mieux encore de 6 à 200.

Selon l'invention, 80% des $R^4$, $R^5$, $R^6$ et $R^7$, du polymère sont choisis de préférence parmi les groupes méthyle, éthyle, phényle et 3,3,3-trifluoropropyle.

Selon l'invention, Y peut représenter divers groupes divalents, comportant éventuellement de plus une ou deux valences libres pour établir des liaisons avec d'autres motifs du polymère ou copolymère. De préférence, Y représente un groupe choisi parmi :

a) les groupes alkylène linéaires en $C_1$ à $C_{20}$, de préférence en $C_1$ à $C_{10}$,
b) les groupes alkylène ramifiés pouvant comporter des cycles et des insaturations non conjuguées, en $C_{30}$ à $C_{56}$,
c) les groupes cycloalkylène en $C_5$-$C_6$,
d) les groupes phénylène éventuellement substitués par un ou plusieurs groupes alkyle en $C_1$ à $C_{40}$,
e) les groupes alkylène en $C_1$ à $C_{20}$, comportant de 1 à 5 groupes amides,
f) les groupes alkylène en $C_1$ à $C_{20}$, comportant un ou plusieurs substituants, choisis parmi les groupes hydroxyle, cycloalcane en $C_3$ à $C_8$, hydroxyalkyle en $C_1$ à $C_3$ et alkylamines en $C_1$ à $C_6$,
g) les chaînes polyorganosiloxane de formule :

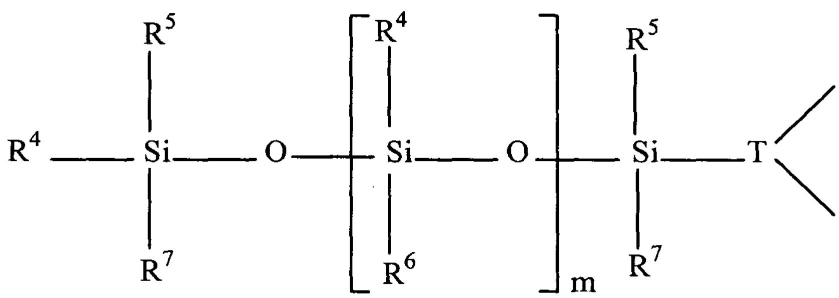

dans laquelle $R^4$, $R^5$, $R^6$, $R^7$, T et m sont tels que définis ci-dessus, et
h) les chaînes polyorganosiloxanes de formule :

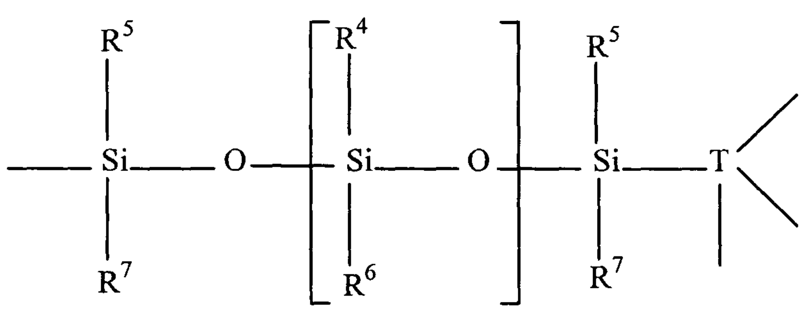

Les polyorganosiloxanes de la seconde famille peuvent être des polymères comprenant au moins un motif répondant à la formule (III) :

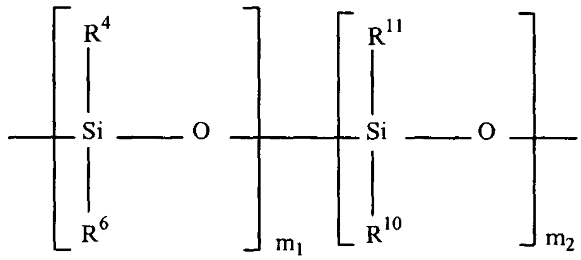 (III)

dans laquelle

- $R^4$ et $R^6$, identiques ou différents, sont tels que définis ci-dessus pour la formule (II),
- $R^{10}$ représente un groupe tel que défini ci-dessus pour $R^4$ et $R^6$, ou représente le groupe de formule -X-G-$R^{12}$ dans laquelle X et G sont tels que définis ci-dessus pour la formule (II) et $R^{12}$ représente un atome d'hydrogène ou un groupe hydrocarboné, linéaire, ramifié ou cyclique, saturé ou insaturé, en $C_1$ à $C_{50}$ comportant éventuellement dans sa chaîne un ou plusieurs atomes choisis parmi O, S et N, éventuellement substitué par un ou plusieurs atomes de fluor et/ou un ou plusieurs groupes hydroxyle, ou un groupe phényle éventuellement substitué par un ou plusieurs groupes alkyle en $C_1$ à $C_4$,
- $R^{11}$ représente le groupe de formule -X-G-$R^9$ dans laquelle X, G et $R^{12}$ sont tels que définis ci-dessus,
- $m_1$ est un nombre entier allant de 1 à 998, et
- $m_2$ est un nombre entier allant de 2 à 500.

Selon l'invention, le polymère utilisé, peut être un homopolymère, c'est-à-dire un polymère comportant plusieurs motifs identiques, en particulier des motifs de formule (II) ou de formule (III).

Selon l'invention, on peut aussi utiliser un polymère constitué par un copolymère comportant plusieurs motifs de formule (II) différents, c'est-à-dire un polymère dans lequel l'un au moins des $R^4$, $R^5$, $R^6$, $R^7$, X, G, Y, m et n est différent dans l'un des motifs. Le copolymère peut être aussi formé de plusieurs motifs de formule (III), dans lequel l'un au moins des $R^4$, $R^6$, $R^{10}$, $R^{11}$, $m_1$ et $m_2$ est différent dans l'un au moins des motifs.

On peut encore utiliser un copolymère comportant au moins un motif de formule (II) et au moins un motif de formule (III), les motifs de formule (II) et les motifs de formule (III) pouvant être identiques ou différents les uns des autres.

Selon une variante, on peut encore utiliser un copolymère comprenant de plus au moins un motif hydrocarboné comportant deux groupes capables d'établir des interactions hydrogènes choisis parmi les groupes ester, amide, sulfonamide, carbamate, thiocarbamate, urée, uréthane, thiourée, oxamido, guanidino, biguanidino et leurs combinaisons.

Ces copolymères peuvent être des copolymères blocs, des copolymères séquencés ou des copolymères greffés.

Selon l'invention le polymère filmogène peut être un solide insoluble dans la phase grasse de la composition à une température ambiante, par exemple, d'environ 25°C. Le polymère est également insoluble dans la phase grasse à sa température de ramollissement, à l'inverse d'une cire même d'origine polymérique qui est elle soluble dans la phase organique liquide (ou phase grasse) à sa température de fusion. En ce sens, le polymère n'est pas une cire.

La composition selon l'invention comprend avantageusement au moins une dispersion stable de particules de polymère essentiellement sphériques d'un ou plusieurs polymères, dans une phase grasse physiologiquement acceptable.

Ces dispersions peuvent notamment se présenter sous forme de nanoparticules de polymères en dispersion stable dans ladite phase organique liquide. Les nanoparticules sont de préférence d'une taille moyenne comprise entre 5 et 800 nm, et mieux entre 50 et 500 nm. Il est toutefois possible d'obtenir des tailles de particules de polymère allant jusqu'à 1μm.

De préférence, les particules de polymères en dispersion sont insolubles dans les alcools hydrosolubles tels que, par exemple, l'éthanol.

Les polymères en dispersion utilisables dans la composition de l'invention ont de préférence un poids moléculaire de l'ordre de 2000 à 10 000 000 g/mol, et une Tg de -100°C à 300°C et mieux de -50° à 100°C, de préférence de -10°C à 50°C.

Il est possible d'utiliser des polymères filmifiables, de préférence ayant une Tg basse, inférieure ou égale à la température de la peau et notamment inférieure ou égale à 40°C.

Parmi les polymères filmogènes, on peut citer des homopolymères ou des copolymères radicalaires, acryliques ou vinyliques, de préférence ayant une Tg inférieure ou égale à 40°C et notamment allant de -10° à 30°C, utilisés

seul ou en mélange.

Par polymère radicalaire, on entend un polymère obtenu par polymérisation de monomères à insaturation notamment éthylénique, chaque monomère étant susceptible de s'homopolymériser (à l'inverse des polycondensats). Les polymères radicalaires peuvent être notamment des polymères, ou des copolymères, vinyliques, notamment des polymères acryliques.

Les polymères acryliques peuvent résulter de la polymérisation de monomères à insaturation éthylénique ayant au moins un groupement acide et/ou des esters de ces monomères acides et/ou des amides de ces acides.

Comme monomère porteur de groupement acide, on peut utiliser des acides carboxyliques insaturés $\alpha,\beta$-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), comme les (méth)acrylates d'alkyle, en particulier d'alkyle en $C_1$-$C_{20}$, de préférence en $C_1$-$C_8$, les (méth)acrylates d'aryle, en particulier d'aryle en $C_6$-$C_{10}$, les (méth)acrylates d'hydroxyalkyle, en particulier d'hydroxyalkyle en $C_2$-$C_6$. Comme (méth)acrylates d'alkyle, on peut citer le (méth)acrylate de méthyle, d'éthyle, de butyle, d'isobutyle, d'éthyl-2 hexyle et de lauryle. Comme (méth)acrylates d'hydroxyalkyle, on peut citer le (méth)acrylate d'hydroxyéthyle, le (méth)acrylate de 2-hydroxypropyle. Comme (méth)acrylates d'aryle, on peut citer l'acrylate de benzyle ou de phényle.

Les esters de l'acide (méth)acrylique particulièrement préférés sont les (méth)acrylates d'alkyle.

Comme polymère radicalaire, on utilise de préférence les copolymères d'acide (méth)acrylique et de (méth) acrylate d'alkyle, notamment d'alkyle en $C_1$-$C_4$. Plus préférentiellement, on peut utiliser les acrylates de méthyle éventuellement copolymérisés avec l'acide acrylique.

Comme amides des monomères acides, on peut citer les (méth)acrylamides, et notamment les N-alkyl (méth) acrylamides, en particulier d'alkyle en $C_2$-$C_{12}$ tels que le N-éthyl acrylamide, le N-t-butyl acrylamide, le N-octyl acrylamide ; les N- dialkyl ($C_1$-$C_4$) (méth)acrylamides.

Les polymères acryliques peuvent également résulter de la polymérisation de monomères à insaturation éthylénique ayant au moins un groupe amine, sous forme libre ou bien partiellement ou totalement neutralisée, ou bien encore partiellement ou totalement quaternisée. De tels monomères peuvent être par exemple le (méth)acrylate de diméthylaminoéthyle, le méthacrylamide de diméthylaminoéthyle, la vinylamine, la vinylpyridine, le chlorure de diallyldiméthylammonium.

Les polymères vinyliques peuvent également résulter de l'homopolymérisation ou de la copolymérisation d'au moins un monomère choisi parmi les esters vinyliques et les monomères styrèniques. En particulier, ces monomères peuvent être polymérisés avec des monomères acides et/ou leurs esters et/ou leurs amides, tels que ceux mentionnés précédemment. Comme exemple d'esters vinyliques, on peut citer l'acétate de vinyle, le propionate de vinyle, le néodécanoate de vinyle, le pivalate de vinyle, le benzoate de vinyle et le t-butyl benzoate de vinyle. Comme monomères styréniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme autres monomères vinyliques utilisables, on peut encore citer :

- la N-vinylpyrrolidone, la vinylcaprolactame, les vinyl N-alkyl($C_1$-$C_6$) pyrroles, les vinyl-oxazoles, les vinyl-thiazoles, les vinylpyrimidines, les vinylimidazoles,
- les oléfines tels que l'éthylène, le propylène, le butylène, l'isoprène, le butadiène.

Le polymère vinylique peut être réticulé à l'aide d'un ou plusieurs monomères difonctionnels, notamment comprenant au moins deux insaturations éthyléniques, tel que le diméthacrylate d'éthylène glycol ou le phtalate de diallyle.

De façon non limitative, les polymères en dispersion de l'invention peuvent être choisis parmi, les polymères ou copolymères suivants : polyuréthanes, polyuréthanes-acryliques, polyurées, polyurée-polyuréthanes, polyester-polyuréthanes, polyéther-polyuréthanes, polyesters, polyesters amides, alkydes ; polymères ou copolymères acryliques et/ou vinyliques ; copolymères acryliques-silicone ; polyacrylamides ; polymères siliconés comme les polyuréthanes ou acryliques siliconés, polymères fluorés et leurs mélanges.

Le ou les polymères en dispersion dans la phase grasse peuvent représenter en matière sèche de 5 à 40% du poids de la composition, de préférence de 5 à 35 % et mieux de 8 à 30%.

Selon un mode de mise en oeuvre, les particules de polymère en dispersion sont stabilisées en surface par un stabilisant solide à température ambiante. Dans ce cas, la quantité en matière sèche de la dispersion représente la quantité totale de polymère + stabilisant, sachant que la quantité de polymère ne peut être inférieure à 5%.

Les particules de polymère sont de préférence stabilisées en surface grâce à un stabilisant, qui peut être un

polymère séquencé, un polymère greffé, et/ou un polymère statistique, seul ou en mélange. La stabilisation peut être effectuée par tout moyen connu, et en particulier par ajout direct du polymère stabilisant lors de la polymérisation.

Le stabilisant peut être également présent dans le mélange avant polymérisation du polymère. Toutefois, il est également possible de l'ajouter en continu, notamment lorsqu'on ajoute également les monomères en continu.

On peut utiliser 2-30% en poids de stabilisant par rapport au mélange initial de monomères, et de préférence 5-20% en poids.

Lorsqu'on utilise un polymère greffé et/ou séquencé en tant que stabilisant, on choisit le solvant de synthèse de telle manière qu'au moins une partie des greffons ou séquences dudit polymère-stabilisant soit soluble dans ledit solvant, l'autre partie des greffons ou séquences n'y étant pas soluble. Le polymère-stabilisant utilisé lors de la polymérisation doit être soluble, ou dispersible, dans le solvant de synthèse. De plus, on choisit de préférence un stabilisant dont les séquences ou greffons insolubles présentent une certaine affinité pour le polymère formé lors de la polymérisation.

Parmi les polymères greffés, on peut citer les polymères siliconés greffés avec une chaîne hydrocarbonée ; les polymères hydrocarbonés greffés avec une chaîne siliconée.

Ainsi on peut utiliser des copolymères blocs greffés ou séquencés comprenant au moins un bloc de type polyorganosiloxane et au moins un bloc d'un polymère radicalaire, comme les copolymères greffés de type acrylique/silicone qui peuvent être employés notamment lorsque le milieu non aqueux est siliconé.

On peut aussi utiliser des copolymères blocs greffés ou séquencés comprenant au moins un bloc de type polyorganosiloxane et au moins d'un polyéther. Le bloc polyorganopolysiloxane peut être notamment un polydiméthylsiloxane ou bien encore un poly alkyl($C_2$-$C_{18}$) méthyl siloxane ; le bloc polyéther peut être un poly alkylène en $C_2$-$C_{18}$, en particulier polyoxyéthylène et/ou polyoxypropylène. En particulier, on peut utiliser les diméthicones copolyol ou des alkyl ($C_2$-$C_{18}$) diméthicones copolyol tels que ceux vendu sous la dénomination "Dow Corning 3225C" par la société Dow Corning, les lauryl méthicones tels que ceux vendu sous la dénomination "Dow Corning Q2-5200 par la société "Dow Corning".

Comme copolymères blocs greffés ou séquencés, on peut citer aussi ceux comprenant au moins un bloc résultant de la polymérisation d'au moins un monomère éthylénique, à une ou plusieurs liaisons éthyléniques éventuellement conjuguées, comme l'éthylène ou les diènes tels que le butadiène et l'isoprène, et d'au moins un bloc d'un polymère vinylique et mieux styrénique. Lorsque le monomère éthylénique comporte plusieurs liaisons éthyléniques éventuellement conjuguées, les insaturations éthyléniques résiduelles après la polymérisation sont généralement hydrogénées. Ainsi, de façon connue, la polymérisation de l'isoprène conduit, après hydrogénation, à la formation de bloc éthylène-propylène, et la polymérisation de butadiène conduit, après hydrogénation, à la formation de bloc éthylène-butylène. Parmi ces polymères, on peut citer les copolymères séquencés, notamment de type "dibloc" ou "tribloc" du type polystyrène/polyisoprène (SI), polystyrène/polybutadiène (SB) tels que ceux vendus sous le nom de 'LUVITOL HSB' par BASF, du type polystyrène/copoly(éthylène-propylène) (SEP) tels que ceux vendus sous le nom de 'Kraton' par Shell Chemical Co ou encore du type polystyrène/copoly(éthylène-butylène) (SEB). En particulier, on peut utiliser le Kraton G1650 (SEBS), le Kraton G1651 (SEBS), le Kraton G1652 (SEBS), le Kraton G 1657X (SEBS), le Kraton G1701X (SEP), le Kraton G1702X (SEP), le Kraton G1726X (SEB), le Kraton D-1101 (SBS), le Kraton D-1102 (SBS), le Kraton D-1107 (SIS). Les polymères sont généralement appelés des copolymères de diènes hydrogénés ou non.

On peut aussi utiliser les Gelled Permethyl 99A-750, 99A-753-59 et 99A-753-58 (mélange de tribloc et de polymère en étoile), Versagel 5960 de chez Penreco (tribloc + polymère en étoile) ; OS129880, OS129881 et OS84383 de chez Lubrizol (copolymère styrène/méthacrylate).

Comme copolymères blocs greffés ou séquencés comprenant au moins un bloc résultant de la polymérisation d'au moins un monomère éthylénique à une ou plusieurs liaisons éthyléniques et d'au moins un bloc d'un polymère acrylique, on peut citer les copolymères bi- ou triséquencés poly(méthylacrylate de méthyle)/polyisobutylène ou les copolymères greffés à squelette poly(méthylacrylate de méthyle) et à greffons polyisobutylène.

Comme copolymères blocs greffés ou séquencés comprenant au moins un bloc résultant de la polymérisation d'au moins un monomère éthylénique à une ou plusieurs liaisons éthyléniques et d'au moins un bloc d'un polyéther tel qu'un polylkylène en $C_2$-$C_{18}$ (polyéthyléné et/ou polyoxypropyléné notamment), on peut citer les copolymères bi- ou triséquencés polyoxyéthylène/polybutadiène ou polyoxyéthylène/polyisobutylène.

Lorsqu'on utilise un polymère statistique en tant que stabilisant, on le choisit de manière à ce qu'il possède une quantité suffisante de groupements le rendant soluble dans le solvant de synthèse envisagé.

On peut ainsi employer des copolymères à base d'acrylates ou de méthacrylates d'alkyle issus d'alcools en $C_1$-$C_4$, et d'acrylates ou de méthacrylates d'alkyle issus d'alcools en $C_8$-$C_{30}$. On peut en particulier citer le copolymère méthacrylate de stéaryle/méthacrylate de méthyle.

Lorsque le solvant de synthèse du polymère est apolaire, il est préférable de choisir en tant que stabilisant, un polymère apportant une couverture des particules la plus complète possible, plusieurs chaînes de polymères-stabilisants venant alors s'adsorber sur une particule de polymère obtenu par polymérisation.

Dans ce cas, on préfère alors utiliser comme stabilisant, soit un polymère greffé, soit un polymère séquencé,

de manière à avoir une meilleure activité interfaciale. En effet, les séquences ou greffons insolubles dans le solvant de synthèse apportent une couverture plus volumineuse à la surface des particules.

Lorsque le solvant de synthèse comprend au moins une huile de silicone, l'agent stabilisant est de préférence choisi dans le groupe constitué par les copolymères blocs greffés ou séquencés comprenant au moins un bloc de type polyorganosiloxane et au moins un bloc d'un polymère radicalaire ou d'un polyéther ou d'un polyester comme les blocs polyoxypropyléné et/ou oxyéthyléné.

Lorsque le solvant de synthèse ne comprend pas d'huile de silicone, l'agent stabilisant est de préférence choisi dans le groupe constitué par :

- (a) les copolymères blocs greffés ou séquencés comprenant au moins un bloc de type polyorganosiloxane et au moins un bloc d'un polymère radicalaire ou d'un polyéther ou d'un polyester,
- (b) les copolymères d'acrylates ou de méthacrylates d'alkyle issus d'alcools en $C_1$-$C_4$, et d'acrylates ou de méthacrylates d'alkyle issus d'alcools en $C_8$-$C_{30}$,
- (c) les copolymères blocs greffés ou séquencés comprenant au moins un bloc résultant de la polymérisation d'au moins un monomère éthylénique, à liaisons éthyléniques conjuguées, et au moins un bloc d'un polymère vinylique ou acrylique ou d'un polyéther ou d'un polyester, ou leurs mélanges.

De préférence, on utilise des polymères dibloc comme agent stabilisant.

Le polymère filmogène liposoluble ou en dispersion dans une phase grasse peut également être utilisé en une quantité allant de 0,01% à 20% (en matière active) par rapport au poids total de la composition, tel que par exemple de 1% à 10%, le cas échéant.

Selon un autre mode de réalisation, le polymère filmogène peut être choisi parmi les dispersions aqueuses de particules polymères, dans le cas où la composition selon l'invention comprend une phase aqueuse.

La dispersion aqueuse comprenant un ou plusieurs polymères filmogènes peut être préparée par l'homme de l'art sur la base de ses connaissances générales, en particulier par une polymérisation en émulsion ou par une mise en dispersion du polymère précédemment formé.

Parmi les polymères filmogènes pouvant être utilisés dans la composition selon la présente invention, on peut citer les polymères synthétiques du type polycondensat ou du type radical, les polymères d'origine naturelle, et des mélanges de ceux-ci.

Parmi les polycondensats, on peut également citer les polyuréthanes anioniques, cationiques, non ioniques ou amphotères, les polyuréthane-acryliques, les polyuréthanes-polyvinylpyrrolidones, les polyester-polyuréthanes, les polyéther-polyuréthanes, les polyurées, les polyurée/polyuréthanes, et des mélanges de ceux-ci.

Les polyuréthanes peuvent être par exemple un copolymère de polyuréthane aliphatique, cycloaliphatique ou aromatique, de polyurée/polyuréthane ou de polyurée comprenant, seul ou en tant que mélange :

- au moins une séquence d'origine polyester linéaire ou ramifiée, aliphatique et/ou cycloaliphatique et/ou aromatique, et/ou
- au moins une séquence d'origine polyéther aliphatique et/ou cycloaliphatique et/ou aromatique, et/ou
- au moins une séquence siliconée, substituée ou non substituée, ramifiée ou non ramifiée, par exemple de polydiméthylsiloxane ou de polyméthylphénylsiloxane, et/ou
- au moins une séquence comprenant des groupements fluorés.

Les polyuréthanes tels que définis dans l'invention peuvent également être obtenus à partir de polyesters ramifiés ou non ramifiés ou à partir d'alkydes comprenant des hydrogènes mobiles qui sont modifiés au moyen d'une polyaddition avec un diisocyanate et un composé co-réactif bifonctionnel organique (par exemple dihydro, diamino ou hydroxy-amino), comprenant en outre soit un groupement carboxylate ou acide carboxylique, soit un groupement sulfonate ou acide sulfonique, voire un groupement amine tertiaire neutralisable ou un groupement ammonium quaternaire.

On peut également citer les polyesters, les polyesteramides, les polyesters à chaîne grasse, les polyamides et les résines d'époxyester.

Les polyesters peuvent être obtenus de manière connue au moyen de la polycondensation de diacides aliphatiques ou aromatiques avec des diols aliphatiques ou aromatiques ou avec des polyols. L'acide succinique, l'acide glutarique, l'acide adipique, l'acide pimélique, l'acide subérique ou l'acide sébacique peuvent être utilisés comme diacides aliphatiques. L'acide téréphtalique ou l'acide isophtalique, voire un dérivé tel que l'anhydride phtalique, peuvent être utilisés comme diacides aromatiques. L'éthylène glycol, le propylène glycol, le diéthylène glycol, le néopentyl glycol, le cyclohexanediméthanol et le 4,4-N-(1-méthylpropylidène)bisphénol, peuvent être utilisés comme diols aliphatiques. Le glycérol, le pentaérythritol, le sorbitol et le triméthylolpropane peuvent être utilisés comme polyols.

Les polyesteramides peuvent être obtenus de manière analogue aux polyesters, au moyen de la polycondensation de diacides avec des diamines ou des aminoalcools. L'éthylènediamine, l'hexaméthylènediamine, et la méta- ou para-phénylènediamine peuvent être utilisées comme diamine. La monoéthanolamine peut être utilisée comme aminoalcool.

Comme monomère portant un groupement anionique pouvant être utilisé pendant la polycondensation, on peut citer par exemple, l'acide diméthylolpropionique, l'acide trimellitique ou un dérivé tel que l'anhydride trimellitique, le sel de sodium de l'acide 3-sulfopentanediol et le sel de sodium de l'acide 5-sulfo-1,3-benzènedicarboxylique. Les polyesters ayant une chaîne grasse peuvent être obtenus par l'intermédiaire de l'utilisation de diols ayant une chaîne grasse lors de la polycondensation. Les résines d'époxyester peuvent être obtenues par la polycondensation d'acides gras avec un condensat au niveau des extrémités $\alpha,\omega$-diépoxy.

Les polymères radicalaires peuvent être en particulier les polymères ou les copolymères acryliques et/ou vinyliques. Les polymères à radical anionique sont préférés. Comme monomère portant un groupement anionique pouvant être utilisé lors de la polymérisation radicalaire, on peut citer l'acide acrylique, l'acide méthacrylique, l'acide crotonique, l'anhydride maléique et l'acide 2-acrylamido-2-méthylpropanesulfonique.

Les polymères acryliques peuvent résulter de la copolymérisation de monomères choisis parmi les esters et/ou les amides de l'acide acrylique ou de l'acide méthacrylique. Comme exemples de monomères du type ester, on peut citer le méthacrylate de méthyle, le méthacrylate d'éthyle, le méthacrylate de butyle, le méthacrylate d'isobutyle, le méthacrylate de 2-éthylhexyle et le méthacrylate de lauryle. Comme exemples de monomères du type amide, on peut citer le N-t-butylacrylamide et le N-t-octylacrylamide.

On utilise de préférence les polymères acryliques obtenus par la copolymérisation de monomères à insaturation éthylénique contenant des groupements hydrophiles, préférablement de nature non ionique, tels que l'acrylate d'hydroxyéthyle, l'acrylate de 2-hydroxypropyle, le méthacrylate d'hydroxyéthyle et le méthacrylate de 2-hydroxypropyle.

Les polymères vinyliques peuvent résulter de l'homopolymérisation ou de la copolymérisation de monomères choisis parmi les esters vinyliques, le styrène ou le butadiène. Comme exemples d'esters vinyliques, on peut citer l'acétate de vinyle, le néodécanoate de vinyle, le pivalate de vinyle, le benzoate de vinyle et le t-butylbenzoate de vinyle.

On peut également utiliser des copolymères d'acrylique/silicone, voire des copolymères de nitrocellulose/acrylique.

On peut également citer les polymères résultant de la polymérisation radicalaire d'un ou plusieurs monomères radicalaires, à l'intérieur et/ou partiellement à la surface de particules préexistantes d'au moins un polymère choisi parmi le groupe constitué par les polyuréthanes, les polyurées, les polyesters, les polyesteramides et/ou les alkydes. Ces polymères sont généralement appelés « polymères hybrides ».

Lorsqu'une dispersion aqueuse de particules polymères est utilisée, la teneur en matière sèche de ladite dispersion aqueuse peut être de l'ordre de 3 à 60% en poids, et préférablement de 10 à 50%.

La taille des particules polymères en dispersion aqueuse peut être comprise entre 10 et 500 nm, et elle est préférablement comprise entre 20 et 150 nm, permettant l'obtention d'un film ayant un brillant notable. Cependant, on peut utiliser des tailles de particules allant jusqu'à un micron.

Comme dispersion aqueuse de polymère filmogène, on peut utiliser les dispersions acryliques vendues sous les dénominations « Neocryl XK-90® », « Neocryl A-1070® », « Neocryl A-1090® », « Neocryl BT-62® », « Neocryl A-1079® » et « Neocryl A-523® » par la société AVECIA-NEORESINS, « Dow Latex 432® » par la société DOW CHEMICAL, « Daitosol 5000 AD® » ou « Daitosol 5000 SJ » par la société DAITO KASEY KOGYO; « Syntran 5760 » par la société Interpolymer ou encore les dispersions aqueuses de polyuréthane vendues sous les dénominations « Neorez R-981® » et « Neorez R-974® » par la société AVECIA-NEORESINS, les « Avalure UR-405® », « Avalure UR-410® », « Avalure UR-425® », « Avalure UR-450® », « Sancure 875® », « Sancure 861® », « Sancure 878® » et « Sancure 2060® » par la société GOODRICH, « Impranil 85® » par la société BAYER, «Aquamere H-1511® » par la société HYDROMER; les sulfopolyesters vendus sous le nom de marque « Eastman AQ® » par la société EASTMAN CHEMICAL PRODUCTS, les dispersions vinyliques comme le « Mexomère PAM », les dispersions aqueuses de polyvinyl acétate comme le « Vinybran® » de la société Nisshin Chemical ou celles commercialisées par la société UNION CARBIDE, les dispersions aqueuses de terpolymère vinyl pyrrolidone, diméthylaminopropyl méthacrylamide et chlorure de lauryldiméthylpropylmethacrylamidoammonium telles que le Styleze W-d'ISP, les dispersion aqueuse de polymère hybrides polyuréthane/polyacryliques telles que celles commercialisées sous les références « Hybridur® » par la société AIR PRODUCTS ou « Duromer ® » de NATIONAL STARCH, les dispersions type core/shell : par exemple celles commercialisées par la société ATOFINA sous la référence Kynar ( core : fluoré -shell : acrylique) ou encore ceux décrits dans le document US 5 188 899 (core : silice - shell : silicone) et leurs mélanges.

Dans le cas où la composition comprend une phase aqueuse, le polymère filmogène peut être un polymère hydrosoluble. Le polymère hydrosoluble est donc solubilisé dans la phase aqueuse de la composition.

Parmi les polymères filmogènes hydrosolubles, on peut citer les polymères cationiques suivants :

(1) les polymères ou copolymères acryliques, tels que les polyacrylates

ou les polyméthacrylates ; les copolymères de la famille (1) peuvent contenir en outre un ou plusieurs motifs dérivant de comonomères pouvant être choisis parmi la famille des acrylamides, des méthacrylamides, des diacétoneacrylamides, des acrylamides et méthacrylamides substitués sur l'azote par des alkyles inférieurs, des acides acrylique ou méthacrylique ou des esters de ceux-ci, des vinyllactames tels que la vinylpyrrolidone ou le vinylcaprolactame, des esters vinyliques.

Ainsi, parmi ces copolymères de la famille (1), on peut citer :

- les copolymères d'acrylamide et de méthacrylate de diméthylaminoéthyle quaternisés par le sulfate de diméthyle, ou par un halogénure de diméthyle tel que celui commercialisé sous la dénomination HERCOFLOC par la société HERCULES,
- le copolymère d'acrylamide et de chlorure de méthacryloyloxyéthyltriméthylammonium décrit, par exemple, dans la demande de brevet EP-A-0 809 76 et commercialisé sous la dénomination BINA QUAT P 100 par la société CIBA GEIGY,
- le copolymère d'acrylamide et de méthosulfate de méthacryloyloxyéthyltriméthylammonium commercialisé sous la dénomination RETEN par la société HERCULES,
- les copolymères de vinylpyrrolidone/acrylate ou méthacrylate de dialkylaminoalkyle, quaternisés ou non quaternisés, tels que les produits commercialisés sous la dénomination « GAFQUAT » par la société ISP, tels que par exemple « GAFQUAT 734 » ou « GAFQUAT 755 », ou bien les produits désignés par « COPOLYMER 845, 958 et 937 ». Ces polymères sont décrits en détail dans les brevets français 2 077 143 et 2 393 573,
- les terpolymères de méthacrylate de diméthylaminoéthyle/vinylcaprolactame/vinylpyrrolidone tels que le produit commercialisé sous la dénomination GAFFIX VC 713 par la société ISP, et
- le copolymère de vinylpyrrolidone/diméthylaminopropylméthacrylamide quaternisé tel que le produit commercialisé sous la dénomination « GAFQUAT HS 100 » par la société ISP.

(2) les polysaccharides quaternisés décrits plus particulièrement dans les brevets US 3 589 578 et US 4 031 307, tels que les gommes de guar contenant des groupements trialkylammonium cationiques. De tels produits sont commercialisés en particulier sous les dénominations commerciales JAGUAR C13 S, JAGUAR C 15 et JAGUAR C 17 par la société MEYHALL.

(3) les copolymères de vinylpyrrolidone et de vinylimidazole quaternaires ;

(4) les chitosanes ou les sels de ceux-ci ;

(5) les dérivés de cellulose cationiques, tels que les copolymères de cellulose ou de dérivés de cellulose greffés par un monomère hydrosoluble comprenant un ammonium quaternaire et décrits en particulier dans le brevet US 4 131 576, tels que les hydroalkyl celluloses, comme les hydroxyméthyl, hydroxyéthyl ou hydroxypropyl celluloses greffées en particulier par un sel de méthacryloyloxyéthyltriméthylammonium, de méthacrylamidopropyltriméthylammonium ou de diméthyldiallylammonium. Les produits commercialisés correspondant à cette définition sont plus particulièrement les produits commercialisés sous la dénomination « CELQUAT L 200 » et « CELQUAT H 100 » par la National Starch Company.

Parmi les polymères hydrosolubles filmogènes, on peut citer les polymères amphotères suivants :

(1) les polymères résultant de la copolymérisation d'un monomère dérivé d'un composé vinylique portant un groupement carboxylique tel que plus particulièrement l'acide acrylique, l'acide méthacrylique, l'acide maléique, l'acide alphachloroacrylique, et d'un monomère basique dérivé d'un composé vinylique substitué contenant au moins un atome de base tel que plus particulièrement un méthacrylate et acrylate de dialkylaminoalkyle, et un dialkylaminoalkylméthacrylamide et acrylamide. De tels composés sont décrits dans le brevet américain n° 3 836 537.

(2) les polymères comprenant des motifs dérivant :

a) d'au moins un monomère choisi parmi les acrylamides ou les méthacrylamides substitués sur l'azote par un radical alkyle,
b) d'au moins un comonomère acide contenant un ou plusieurs groupements carboxyliques réactifs, et
c) d'au moins un comonomère basique tel que les esters, ayant des substituants amine primaire, secondaire, tertiaire et quaternaire, d'acides acrylique et méthacrylique, et le produit de la quaternisation du méthacrylate de diméthylaminoéthyle par du sulfate de diméthyle ou de diéthyle.

(3)les alkoylpolyaminoamides réticulés dérivés totalement ou en partie de polyaminoamides.

(4)les polymères comprenant des motifs zwitterioniques.

(5)le polymère dérivé du chitosane.

(6)les polymères dérivés de la N-carboxyalkylation du chitosane, tels que le N-carboxyméthyl chitosane ou le N-carboxybutyl chitosane commercialisé sous la dénomination « EVALSAN » par la société JAN DEKKER.
(7)les copolymères du ($C_1$-$C_5$)alkylvinyléther/anhydride maléique partiellement modifié par une semi-amidification par une N,N-dialkylaminoalkylamine, telle que la N,N-diméthylaminopropylamine ou par une semi-estérification par une N,N-dialcanolamine. Ces copolymères peuvent également comporter d'autres comonomères vinyliques tels que le vinylcaprolactame.

Les polymères filmogènes hydrosolubles sont préférablement choisis parmi le groupe constitué par :

- les protéines telles que les protéines d'origine végétale, telles que les protéines de blé ou de soja ; les protéines d'origine animale, telles que la kératine, par exemple les hydrolysats de kératine et les kératines sulfoniques ;
- les polymères anioniques, cationiques, amphotères ou non ioniques de la chitine ou du chitosane ;
- les polymères cellulosiques, tels que l'hydroxyéthyl cellulose, l'hydroxypropyl cellulose, la méthylcellulose, l'éthylhydroxyéthyl cellulose, la carboxyméthyl cellulose, et les dérivés quaternisés de la cellulose ;
- les polymères ou copolymères acryliques tels que les polyacrylates ou les polyméthacrylates ;
- les polymères vinyliques, tels que les polyvinylpyrrolidones, les copolymères du méthylvinyléther et de l'anhydride maléique, le copolymère de l'acétate de vinyle et de l'acide crotonique, les copolymères de la vinylpyrrolidone et de l'acétate de vinyle ;
- les copolymères de la vinylpyrrolidone et du caprolactame ; les alcools polyvinyliques ;
- les polymères éventuellement modifiés d'origine naturelle, tels que :

  . la gomme arabique, la gomme de guar, les dérivés du xanthane, la gomme de karaya ;
  . les alginates et les carraghénanes ;
  . les glycoaminoglycanes, l'acide hyaluronique et ses dérivés ;
  . la gomme laque, la gomme sandaraque, les dammars, l'élémis, les copals ;
  . l'acide désoxyribonucléique;
  . les mucopolysaccharides, tels que l'acide hyaluronique, le sulfate de chondroïtine, et des mélanges de ceux-ci.

Ces polymères seront utilisés en particulier si l'on désire une élimination plus ou moins appréciable du film par de l'eau.
Afin d'améliorer la nature filmogène d'un polymère huileux ou aqueux, il est possible d'ajouter au système polymère un agent de coalescence qui sera choisi parmi les agents de coalescence connus.
Selon un mode de réalisation de l'invention, le polymère filmogène peu être choisi parmi les polymères ayant un squelette organique non siliconé greffé par des monomères contenant un polysiloxane. Ces polymères peuvent être liposolubles, lipodispersibles, hydrosolubles ou dispersibles en milieu aqueux, le cas échéant.
Les polymères ayant un squelette organique non siliconé greffé par des monomères contenant un polysiloxane sont constitués d'une chaîne organique principale formée de monomères organiques ne comprenant pas la silicone, sur laquelle on greffe, au sein de ladite chaîne ainsi qu'éventuellement sur au moins l'une des extrémités de celle-ci, au moins un macromère de polysiloxane.
Dans ce qui suit, on doit comprendre que l'expression « macromère de polysiloxane » désigne, ainsi qu'il est généralement accepté, tout monomère contenant une chaîne polymère du type polysiloxane dans sa structure.
Les monomères organiques non siliconés constituant la chaîne principale du polymère siliconé greffé peuvent être choisis parmi les monomères à insaturation éthylénique pouvant être polymérisés par la méthode radicalaire, les monomères polymérisables par polycondensation tels que ceux formant les polyamides, les polyesters, les polyuréthanes, les monomères à cycle ouvrant tels que ceux du type oxazoline ou caprolactone.
Les polymères ayant un squelette organique non siliconé greffé par des monomères contenant un polysiloxane selon la présente invention peuvent être obtenus conformément à toute méthode connue de l'homme de l'art, en particulier par la réaction entre (i) un macromère de polysiloxane de départ correctement fonctionnalisé sur la chaîne de polysiloxane et (ii) un ou plusieurs composés organiques non s iliconés, eux même correctement fonctionnalisés par une fonction qui est capable de réagir avec le groupement ou les groupements fonctionnel(s) porté(s) par ladite silicone en formant une liaison covalente ; un exemple classique d'une telle réaction est la réaction radicalaire entre un groupement vinyle porté à l'une des extrémités de la silicone avec une double liaison d'un monomère à insaturation éthylénique de la chaîne principale.
Les polymères ayant un squelette organique non siliconé greffé par des monomères contenant un polysiloxane selon l'invention sont choisis de préférence parmi ceux décrits dans les brevets US 4 693 935, US 4 728 571 et US 4 972 037 et les demandes de brevet EP-A-0 412 704, EP-A-0 412 707, EP-A-0 640 105 et WO 95/00578. Il concerne des copolymères obtenus par la polymérisation radicalaire à partir de monomères à insaturation éthylénique et de monomères ayant un groupement terminal vinyle, ou bien des copolymères obtenus par la réaction d'une polyoléfine contenant des groupements fonctionnalisés et un m acromère de polysiloxane ayant une fonction terminale réactive

avec lesdits groupements fonctionnalisés.

Une famille particulière de polymères siliconés greffés convenables pour la mise en oeuvre de la présente invention est constituée par les polymères siliconés greffés contenant :

a) de 0 à 98% en poids d'au moins un monomère lipophile (A) de faible polarité lipophile à insaturation éthylénique, polymérisable par la méthode radicalaire ;
b) de 0 à 98% en poids d'au moins un monomère polaire hydrophile (B) à insaturation éthylénique, copolymérisable avec le monomère ou les monomères du type (A) ;
c) de 0,01 à 50% en poids d'au moins un macromère de polysiloxane (C) de formule générale :

$$X(Y)_nSi(R)_{3-m}Z_m \qquad (I)$$

dans laquelle :

X désigne un groupement vinyle copolymérisable avec les monomères (A) et (B) ;
Y désigne un groupement ayant une liaison divalente ;
R désigne hydrogène, alkyle ou alkoxy en $C_1$-$C_6$, aryle en $C_6$-$C_{12}$;
Z désigne un motif polysiloxane monovalent ayant un poids moléculaire moyen en nombre d'au moins 500 ;
n vaut 0 ou 1 et m est un nombre entier allant de 1 à 3 ; les pourcentages étant calculés par rapport au poids total des monomères (A), (B) et (C).

Ces polymères ont un poids moléculaire moyen en nombre allant de 10 000 à 2 000 000, et préférablement une température de transition vitreuse Tg ou une température de fusion cristalline Tm d'au moins -20°C.

Comme exemples de monomères lipophiles (A), on peut citer les esters d'alcool en $C_1$-$C_{18}$ et de l'acide acrylique ou méthacrylique ; les esters d'alcool en $C_{12}$-$C_{30}$ et de l'acide méthacrylique , le styrène ; les macromères de polystyrène ; l'acétate de vinyle ; le propionate de vinyle ; l'alpha-méthylstyrène ; le tertio-butylstyrène ; le butadiène ; le cyclohexadiène ; le cyclohexadiène [sic] ; l'éthylène ; le propylène ; le vinyltoluène, les esters de l'acide acrylique ou méthacrylique et de 1,1-dihydroperfluoroalcanols ou d'homologues de ceux-ci ; les esters de l'acide acrylique ou méthacrylique et d'omega-hydrofluoroalcanols ; les esters de l'acide acrylique ou méthacrylique et de fluoroalkylsulfonamidoalcools ; les esters de l'acide acrylique ou méthacrylique et de fluoroalkylalcools ; les esters de l'acide acrylique ou méthacrylique et d'alcool-fluoroéthers ; ou des mélanges de ceux-ci. Les monomères (A) préférés sont choisis au sein du groupe constitué par le méthacrylate de n-butyle, le méthacrylate d'isobutyle, l'acrylate de tertio-butyle, le méthacrylate de tertio-butyle, le méthacrylate de 2-éthylhexyle, le méthacrylate de méthyle, l'acrylate de 2-(N-méthylperfluorooctanesulfonamido)éthyle, l'acrylate de 2-(N-butylperfluorooctanesulfona-mido)éthyle, ou des mélanges de ceux-ci.

Comme exemples de monomères (B) polaires, on peut citer l'acide acrylique, l'acide méthacrylique, le N,N-diméthylacrylamide, le méthacrylate de diméthylaminoéthyle, le méthacrylate de diméthylaminoéthyle quaternisé, le (méth)acrylamide, le N-t-butylacrylamide, l'acide maléique, l'anhydride maléique et les hemi-esters de ceux-ci, les (méth)acrylates d'hydroxyalkyle, le chlorure de diallyldiméthylammonium, la vinylpyrrolidone, les éthers vinyliques, les maléimides, la vinylpyridine, le vinylimidazole, les composés polaires vinyliques et hétérocycliques, le sulfonate de styrène, l'alcool allylique, l'alcool vinylique, le vinylcaprolactame ou des mélanges de ceux-ci. Les monomères (B) sont choisis de préférence au sein du groupe constitué par l'acide acrylique, le N,N-diméthylacrylamide, le méthacrylate de diméthylaminoéthyle, le méthacrylate de diméthylaminoéthyle quaternisé, la vinylpyrrolidone, et des mélanges de ceux-ci.

On cite notamment le produit KP 561 ou le KP 562 commercialisé par Shin Etsu tel que le monomère (A) et choisi parmi les esters d'alcool en $C_{18}$-$C_{22}$ et de l'acide méthacrylique.

Les macromères de polysiloxane (C) de formule (I) sont choisis de préférence parmi ceux correspondant à la formule générale (II) suivante :

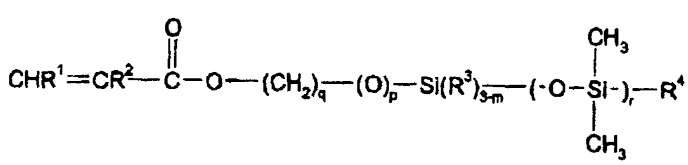 (II)

dans laquelle :

$R^1$ est hydrogène ou -COOH (préférablement hydrogène) ;
$R^2$ est hydrogène, méthyle ou $-CH_2COOH$ (préférablement méthyle) ;
$R^3$ est alkyle, alkoxy ou alkylamino en $C_1$-$C_6$, aryle en $C_6$-$C_{12}$ ou hydroxyle (préférablement méthyle) ;
$R^4$ est alkyle, alkoxy ou alkylamino en $C_1$-$C_6$, aryle en $C_6$-$C_{12}$ ou hydroxyle (préférablement méthyle) ;
q est un nombre entier allant de 2 à 6 (préférablement 3) ;
p vaut 0 ou 1 ;
r est un nombre entier allant de 5 à 700 ;
m est un nombre entier allant de 1 à 3 (préférablement 1).

On utilise de préférence les macromères de polysiloxane de formule :

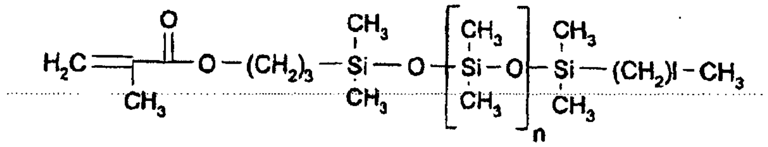

n étant un nombre allant de 5 à 700 et l étant un nombre entier compris entre 0 et 3.

Un mode de réalisation de l'invention consiste en l'utilisation d'un copolymère capable d'être obtenu par une polymérisation radicalaire à partir du mélange de monomères constitué par :

a) 60% en poids d'acrylate de tertio-butyle ;
b) 20% en poids d'acide acrylique ;
c) 20% en poids de macromère siliconé de formule :

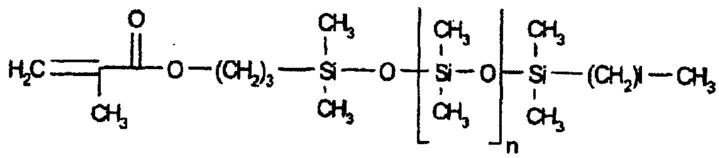

n étant un nombre allant de 5 à 700 et l étant un nombre entier compris entre 0 et 3, les pourcentages en poids étant calculés par rapport au poids total des monomères.

Un autre mode de réalisation particulier de l'invention consiste en l'utilisation d'un copolymère capable d'être obtenu par u ne polymérisation radicalaire à partir du mélange de monomères constitué par :

a) 80% en poids d'acrylate de tertio-butyle ;
b) 20% en poids de macromère siliconé de formule :

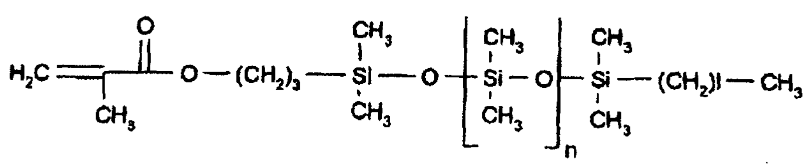

n étant un nombre allant de 5 à 700 et l étant un nombre entier compris entre 0 et 3, les pourcentages en poids étant calculés par rapport au poids total des monomères.

Une autre famille particulière de polymères siliconés greffés ayant un squelette organique non siliconé convenable pour une mise en oeuvre de la présente invention est constituée par les copolymères siliconés greffés capables d'être obtenus par l'extrusion réactive d'un macromère de polysiloxane à fonction terminale réactive sur un polymère du type polyoléfine comprenant des groupements réactifs capables de réagir avec la fonction terminale du macromère de polysiloxane pour former une liaison covalente permettant le greffage de la silicone sur la chaîne principale de la

polyoléfine. Ces polymères, ainsi que leur procédé de préparation, sont décrits dans la demande de brevet WO 95/00578.

Les polyoléfines réactives sont choisies de préférence parmi les polyéthylènes ou les polymères de monomères dérivés de l'éthylène, tels que le propylène, le styrène, l'alkylstyrène, le butylène, le butadiène, les (méth)acrylates, les esters vinyliques ou équivalents, comprenant des fonctions réactives capables de réagir avec la fonction terminale du macromère de polysiloxane. Ils sont choisis plus particulièrement parmi les copolymères d'éthylène ou de dérivés d'éthylène et les monomères choisis parmi ceux comprenant une fonction carboxylique tels que l'acide (méth) acrylique ; ceux comprenant une fonction anhydride d'acide tels que l'anhydride de l'acide maléique ; ceux comprenant une fonction chlorure d'acide tels que le chlorure de l'acide (méth)acrylique ; ceux comprenant une fonction ester tels que les esters de l'acide (méth)acrylique ; et ceux comportant une fonction isocyanate.

Les macromères siliconés sont choisis de préférence parmi les polysiloxanes comprenant un groupement fonctionnalisé, à l'extrémité de la chaîne de polysiloxane ou à proximité de l'extrémité de ladite chaîne, choisis au sein du groupe constitué par les alcools, les thiols, les époxy, les amines primaires et secondaires, et tout particulièrement parmi ceux correspondant à la formule générale :

$$T\text{-}(CH_2)_6\text{-}Si\text{-}[\text{-}(OSiR^5R^6)_t\text{-}R^7]_y \qquad \text{(III)}$$

dans laquelle T est choisi parmi le groupe constitué par $NH_2$, NHRN, une fonction époxy, OH, SH ; $R^5$, $R^6$, $R^7$ et RN, indépendamment, désignent alkyle en $C_1$-$C_6$, phényle, benzyle ou alkylphényle en $C_6$-$C_{12}$, hydrogène ; s est un nombre allant de 2 à 00 [sic], t est un nombre allant de 0 à 1000 et y est un nombre allant de 1 à 3. Ils ont un poids moléculaire moyen en nombre allant préférablement de 5000 à 300 000, plus préférablement de 8000 à 200 000, et plus particulièrement de 9000 à 40 000.

Selon un mode de réalisation préféré, le polymère filmogène peut être acheté auprès de la Minnesota Mining and Manufacturing Company sous les dénominations commerciales de polymères « Silicone Plus ». Par exemple, le poly(méthacrylate d'isobutyle-co-FOSEA de méthyle)-g-poly(diméthylsiloxane) est commercialisé sous la dénomination commerciale SA 70-5 IBMMF.

Selon une autre forme préférée de l'invention, le polymère filmogène est choisi parmi les polymères siliconés greffés par des monomères organiques non siliconés. Ces polymères peuvent être liposolubles, lipodispersables, hydrosolubles ou dispersables en milieu aqueux, le cas échéant.

Ledit polymère ou lesdits polymères siliconé(s) greffé(s) ayant un squelette de polysiloxane greffé par des monomères organiques non siliconés contenant une chaîne principale de silicone (ou de polysiloxane $(/SiO\text{-})_n$) sur laquelle se trouve greffé, à l'intérieur de ladite chaîne ainsi qu'éventuellement à l'une au moins de ses extrémités, au moins un groupement organique ne comprenant pas de silicone.

Les polymères ayant un squelette de polysiloxane greffé par des monomères organiques non siliconés selon l'invention peuvent être des produits commerciaux existants ou bien ils peuvent être obtenus par tout moyen connu de l'homme de l'art, en particulier par une réaction entre (i) une silicone de départ correctement fonctionnalisée sur un ou plusieurs de ces atomes de silicium et (ii) un composé organique non siliconé lui-même correctement fonctionnalisé par une fonction qui est capable de réagir avec le groupement ou les groupements fonctionnel(s) porté(s) par ladite silicone en formant une liaison covalente ; un exemple classique d'une telle réaction est la réaction d'hydrosylilation entre des groupements /Si-H et des groupements vinyliques $CH_2$=CH-, voire la réaction entre des groupements thio-fonctionnels -SH avec ces mêmes groupements vinyle.

Des exemples de polymères ayant un squelette de polysiloxane greffé par des monomères organiques non siliconés convenables pour une mise en oeuvre de la présente invention, ainsi que leur méthode spécifique de préparation, sont décrits en particulier dans les demandes de brevet EP-A-0 582 152, WO 93/23009 et WO 95/03776, dont les enseignements sont totalement inclus dans la présente description à titre de références non limitatives.

Selon un mode de réalisation particulièrement préféré de la présente invention, le polymère siliconé, ayant un squelette de polysiloxane greffé par des monomères organiques non siliconés, mis en oeuvre, est constitué du résultat d'une copolymérisation radicalaire entre, d'une part, au moins un monomère organique anionique non siliconé à insaturation éthylénique et/ou un monomère organique hydrophobe non siliconé à insaturation éthylénique et, d'autre part, une silicone présentant dans sa chaîne au moins un groupement fonctionnel, et préférablement plusieurs, capable de réagir avec lesdites insaturations éthyléniques desdits monomères non siliconés en formant une liaison covalente, en particulier des groupements thio-fonctionnels.

Selon la présente invention, lesdits monomères anioniques à insaturation éthylénique sont de préférence choisis, seuls ou comme mélanges, parmi les acides carboxyliques insaturés, linéaires ou ramifiés, éventuellement neutralisés partiellement ou totalement sous forme d'un sel, ce ou ces acide(s) carboxylique(s) insaturé(s) p ouvant être plus particulièrement l'acide acrylique, l'acide méthacrylique, l'acide maléique, l'acide itaconique, l'acide fumarique

et l'acide crotonique. Les sels convenables sont en particulier les sels alcalins, alcalino-terreux et d'ammonium. On notera que, de même, dans le polymère siliconé greffé final, le groupement organique de nature anionique qui est constitué du résultat de l'(homo)polymérisation radicalaire d'au moins un monomère anionique du type acide carboxylique insaturé peut être, après réaction, post-neutralisé par une base (soude, ammoniaque, etc.) pour l'amener sous la forme d'un sel.

Selon la présente invention, les monomères hydrophobes à insaturation éthylénique sont de préférence choisis, seuls ou comme mélange, parmi les esters de l'acide acrylique d'alcanols et/ou les esters de l'acide méthacrylique d'alcanols. Les alcanols sont de préférence en $C_1$-$C_{30}$ et plus particulièrement en $C_1$-$C_{22}$. Les monomères préférés sont choisis parmi le groupe constitué par le (méth)acrylate d'isooctyle, le (méth)acrylate d'isononyle, le (méth)acrylate de 2-éthylhexyle, le (méth)acrylate de lauryle, le (méth)-acrylate d'isopentyle, le (méth)acrylate de n-butyle, le (méth) acrylate d'isobutyle, le (méth)acrylate de méthyle, le (méth)acrylate de tertio-butyle, le (méth)acrylate de tridécyle et le (méth)acrylate de stéaryle, ou des mélanges de ceux-ci.

Une famille de polymères siliconés ayant un squelette de polysiloxane greffé par des monomères organiques non siliconés, convenant particulièrement bien à la mise en oeuvre de la présente invention, est constituée par les polymères siliconés comprenant dans leur structure le motif de formule IV ci-dessous :

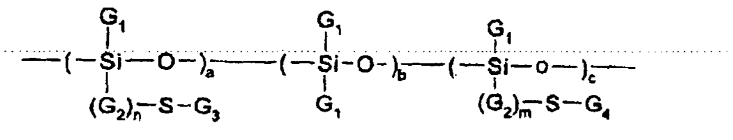

(IV)

dans laquelle les radicaux $G_1$, identiques ou différents, représentent hydrogène ou un radical alkyle en $C_1$-$C_{10}$, voire un radical phényle ; les radicaux $G_2$, identiques ou différents, représentent représente [sic] un groupement alkylène en $C_1$-$C_{10}$; $G_3$ représente un reste polymère résultant de l'(homo)polymérisation d'au moins un monomère anionique à insaturation éthylénique ; $G_4$ représente un reste polymère résultant de l'(homo)polymérisation d'au moins un monomère d'au moins un monomère hydrophobe [sic] à insaturation éthylénique ; m et n sont égaux à 0 ou 1 ; a est un nombre entier allant de 0 à 50 ; b est un nombre entier pouvant être compris entre 10 et 350, c est un nombre entier allant de 0 à 50 ; à condition que l'un des paramètres a et c soit différent de 0.

Le motif de formule (IV) du texte ci-dessus possède de préférence au moins une, et encore plus préférablement l'ensemble, des caractéristiques suivantes :

- les radicaux $G_1$ désignent un radical alkyle, préférablement un radical méthyle ;
- n ne vaut pas zéro, et les radicaux $G_2$ représentent un radical divalent en $C_1$-$C_3$, préférablement un radical propylène ;
- $G_3$ représente un radical polymère résultant de l'(homo)polymérisation d'au moins un monomère du type acide carboxylique à insaturation éthylénique, préférablement l'acide acrylique et/ou l'acide méthacrylique ;
- $G_4$ représente un radical polymère résultant de l'(homo)polymérisation d'au moins un monomère du type (méth) acrylate d'alkyle en $C_1$-$C_{10}$, préférablement le (méth)acrylate d'isobutyle ou de méthyle.

Des exemples de polymères siliconés correspondant à la formule (IV) sont en particulier des polydiméthylsiloxanes (PDMS) sur lesquels sont greffés, par l'intermédiaire d'une liaison secondaire du type thiopropylène, des motifs polymères mixtes du type acide poly(méth)acrylique et du type poly(méth)acrylate d'alkyle.

D'autres exemples de polymères siliconés correspondant à la formule (IV) sont en particulier des polydiméthylsiloxanes (PDMS) sur lesquels sont greffés, par l'intermédiaire d'une liaison secondaire du type thiopropylène, des motifs polymères du type poly(méth)acrylate d'isobutyle.

De tels polymères comportent les polymères comprenant au moins un groupement de formule :

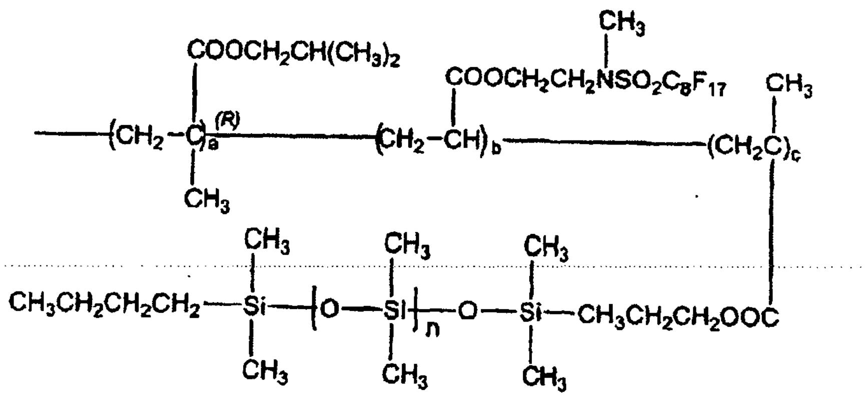

dans laquelle

a, b et c, pouvant être identiques ou différents, sont chacun un nombre allant de 1 à 100 000 ; et les groupements terminaux, pouvant être identiques ou différents, sont choisis chacun parmi les groupements alkyle linéaires en $C_1$-$C_{20}$, les groupements alkyle à chaîne ramifiée en $C_3$-$C_{20}$, les groupements aryle en $C_3$-$C_{20}$, les groupements alkoxy linéaires en $C_1$-$C_{20}$ et les groupements alkoxy ramifiés en $C_3$-$C_{20}$.

De tels polymères sont divulgués dans les brevets US n° 4 972 037, 5 061 481, 5 209 924, 5 849 275 et 6 033 650, et WO 93/23446 et WO 95/06078.

Une autre famille de polymères siliconés ayant un squelette de polysiloxane greffé par des monomères organiques non siliconés, convenant particulièrement bien à la mise en oeuvre de la présente invention, est constituée par les polymères siliconés comprenant dans leur structure le motif de formule (V) ci-dessous :

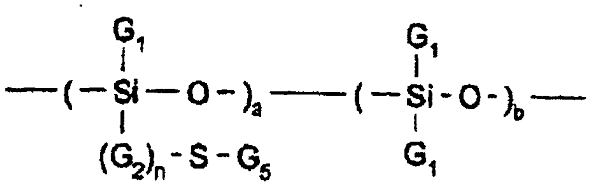

(V)

dans laquelle les radicaux $G_1$ et $G_2$ ont la même signification que ci-dessus; $G_5$ représente un reste polymère résultant de l'(homo)polymérisation d'au moins un monomère d'au moins un monomère hydrophobe [sic] à insaturation éthylénique ou de la copolymérisation d'au moins un monomère anionique à insaturation éthylénique et d'au moins un monomère hydrophobe à insaturation éthylénique ; n est égal à 0 ou 1 ; a est un nombre entier allant de 0 à 50 ; b est un nombre entier pouvant être compris entre 10 et 350 ; à condition que a soit différent de 0.

Le motif de formule (V) du texte ci-dessus possède de préférence au moins une, et encore plus préférablement l'ensemble, des caractéristiques suivantes :

- les radicaux $G_1$ désignent un radical alkyle, préférablement un radical méthyle ;
- n ne vaut pas zéro, et les radicaux $G_2$ représentent un radical divalent en $C_1$-$C_3$, préférablement un radical propylène.

La masse moléculaire en nombre des polymères siliconés ayant un squelette de polysiloxane greffé par des monomères organiques non siliconés de l'invention varie préférablement d'environ 10 000 à 1 000 000, et encore plus préférablement d'environ 10 000 à 100 000.

La composition peut contenir de 0,5 à 60% en poids, mieux de 1 à 40%, préférablement de 2 à 30% en poids de matière sèche d'agent filmogène par rapport au poids total de la composition.

Plus généralement, la quantité totale de polymère doit être en quantité suffisante pour former sur la peau et/ou les lèvres un film cohésif capable de suivre les mouvements de la peau et/ou des lèvres sans se décoller ou craquer.

Lorsque le polymère a une température de transition vitreuse trop élevée pour l'utilisation désirée, on peut y associer un plastifiant de façon à abaisser cette température du mélange utilisé. Le plastifiant peut être choisi parmi les plastifiants utilisés habituellement dans le domaine d'application, et notamment parmi les composés pouvant être des solvants pour le polymère.

**Milieu physiologiquement acceptable**

Par « milieu physiologiquement acceptable », on désigne un milieu non toxique et susceptible d'être appliqué sur la peau ou les lèvres d'êtres humains. Le milieu physiologiquement acceptable est généralement adapté à la nature du support sur lequel doit être appliquée la composition ainsi qu'à l'aspect sous lequel la composition est destinée à être conditionnée.

Phase aqueuse

La composition selon l'invention peut comprendre au moins un milieu aqueux, constituant une phase aqueuse, qui peut former la phase continue de la composition.

La phase aqueuse peut être constituée essentiellement d'eau.

Elle peut également comprendre un mélange d'eau et de solvant organique miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en $C_3$-$C_4$, et les aldéhydes en $C_2$-$C_4$.

La phase aqueuse (eau et éventuellement le solvant organique miscible à l'eau) peut être présente, à une teneur allant de 1 % à 95 % en poids, notamment allant de 3 % à 80 % en poids, et en particulier allant de 5 % à 60 % en poids par rapport au poids total de la composition.

Cette phase aqueuse peut, le cas échéant, être épaissie, gélifiée ou structurée en y incorporant en outre un gélifiant aqueux traditionnel notamment d'origine minérale comme l'argile par exemple et/ou organique comme un polymère gélifiant aqueux.

Un tel milieu peut également comprendre au moins une huile volatile telle que définie ci-après.

Phase grasse

La composition, notamment lorsqu'elle est destinée à être appliquée sur les lèvres, peut comporter au moins une phase grasse et notamment au moins un corps gras liquide à température ambiante (25°C) et à pression atmosphérique et/ou un corps gras solide à température ambiante et à pression atmosphérique tel que les cires, les gommes et leurs mélanges. La phase grasse peut en outre contenir des agents gélifiants et structurants d'huiles de nature organique et/ou des solvants organiques lipophiles.

Selon une variante particulière de l'invention, la composition cosmétique est exempte de paraffine, de vaseline et de lanoline. En effet, les lanolines présentent l'inconvénient d'être sensibles à la chaleur et aux ultraviolets, et ont tendance à s'oxyder dans le temps avec un dégagement d'odeur désagréable, ce qui limite leur utilisation dans les compositions cosmétiques. De plus, lorsque les lanolines sont associées avec des huiles couramment utilisées dans le domaine cosmétique, les compositions obtenues présentent des problèmes de collant, qui sont d'autant plus prononcés que l'huile utilisée possède une viscosité élevée.

La phase grasse de la composition selon l'invention peut notamment comprendre, à titre de corps gras liquide, au moins une huile volatile ou non volatile ou un de leurs mélanges.

Par « huile volatile », on entend au sens de l'invention toute huile susceptible de s'évaporer au contact de la peau en moins d'une heure, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,01 à 300 mm de Hg (1,33 Pa à 40.000 Pa) et de préférence supérieure à 0,3 mm de Hg (30 Pa).

Par "huile non volatile", on entend une huile restant sur la peau à température ambiante et pression atmosphérique au moins plusieurs heures et ayant notamment une pression de vapeur inférieure à 0,01 mm de Hg (1,33 Pa).

Ces huiles volatiles ou non volatiles peuvent être des huiles hydrocarbonées notamment d'origine animales ou végétales, des huiles siliconées, ou leurs mélanges. On entend par "huile hydrocarbonée", une huile contenant principalement des atomes d'hydrogène et de carbone et éventuellement des atomes d'oxygène, d'azote, de soufre et/ou de phosphore.

Les huiles hydrocarbonées volatiles peuvent être choisies parmi les huiles hydrocarbonées ayant de 8 à 16 atomes de carbone, et notamment les alcanes ramifiés en $C_8$-$C_{16}$ comme les isoalcanes en $C_8$-$C_{16}$ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars® ou de Permetyls®, les esters ramifiés en $C_8$-$C_{16}$ tels que le néopentanoate d'iso-hexyle, et leurs mélanges. D'autres huiles hydrocarbonées volatiles comme les distillats de pétrole, notamment ceux vendus sous la dénomination Shell Solt® par la société SHELL, peuvent aussi être utilisées.

Comme huiles volatiles, on peut aussi utiliser les silicones volatiles, comme par exemple les huiles de silicones

linéaires ou cycliques volatiles, notamment celles ayant une viscosité ≤ 8 centistokes ($8 \times 10^{-6}$ $m^2/s$), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile volatile peut être présente dans la composition selon l'invention à une teneur allant de 0,1 % à 98 % en poids, notamment de 1% à 65 % en poids, et en particulier de 2 % à 50 % en poids, par rapport au poids total de la composition.

Les huiles non volatiles peuvent notamment être choisies parmi les huiles hydrocarbonées le cas échéant fluorées et/ou les huiles siliconées non volatiles.

Comme huile hydrocarbonée non volatile, on peut notamment citer :

- les huiles hydrocarbonées d'origine animale,
- les huiles hydrocarbonées d'origine végétale telles que les triglycérides constitués d'esters d'acides gras et de glycérol dont les acides gras peuvent avoir des longueurs de chaînes variées de $C_4$ à $C_{24}$, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société Stéarineries Dubois ou ceux vendus sous les dénominations Miglyol 810®, 812® et 818® par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane et leurs mélanges,
- les esters de synthèse comme les huiles de formule $R_1COOR_2$ dans laquelle $R_1$ représente le reste d'un acide gras linéaire ou ramifié comportant de 1 à 40 atomes de carbone et $R_2$ représente une chaîne hydrocarbonée notamment ramifiée contenant de 1 à 40 atomes de carbone à condition que $R_1 + R_2$ soit ≥ 10, comme par exemple l'huile de Purcellin (octanoate de cétostéaryle), le myristate d'isopropyle, le palmitate d'isopropyle, les benzoates d'alcools en $C_{12}$ à $C_{15}$, le laurate d'hexyle, l'adipate de düsopropyle, l'isononanoate d'isononyle, le palmitate de 2-éthyl-hexyle, l'isostéarate d'isostéaryle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle ; les esters de polyols et les esters du pentaérythritol,
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique et leurs mélanges.

  Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et les 2-phényléthyl triméthylsiloxysilicates et leurs mélanges.

  Les huiles non volatiles peuvent être présentes dans la composition selon l'invention en une teneur allant de 0,01 à 90 % en poids, notamment de 0,1 % à 85% en poids, et en particulier de 1 % à 70 % en poids, par rapport au poids total de la composition.

  Les huiles peuvent représenter de 0,01 à 99 % du poids total de la composition, de préférence de 0,05 à 60 % et mieux de 1 à 35 %.

On peut inclure dans le cadre de la présente invention des huiles, différentes de l'ester de dimère diol et d'acide décrit précédemment dont le poids moléculaire est compris entre 650 et 10.000 g/mol, de préférence entre 750 et 7.500 g/mol.

Selon un mode de mise en oeuvre, la composition de l'invention comprend une phase huileuse comprenant au moins 5 % en poids d'une huile de masse molaire comprise entre 650 et 10.000 g/mol, de préférence entre 750 et 7.500 g/mol.

L'huile de masse molaire élevée est de préférence choisie parmi les polymères lipophiles :

- les esters d'acides gras linéaires ayant un nombre total de carbone allant de 35 à 70,
- les esters hydroxylés,
- les esters aromatiques,
- les esters d'alcool gras ou d'acides gras ramifiés en $C_{24}$-$C_{28}$,
- les huiles siliconées,
- les huiles d'origine végétale, et leurs mélanges.

L'huile de masse molaire élevée est de préférence choisie parmi les polybutylènes, les polyisobutylènes hydrogénés, les polydécènes, les polydécènes hydrogénés, les copolymères de la vinylpyrrolidone tel que le PVP / héxadécène copolymère, le tétrapélargonate de pentaérythrityle, le triisostéarate de polyglycérol-2, le tridécyl trimellitate, le citrate de triisoarachidyle, le tétraisononanoate de pentaérythrityle, le triisostéarate de glycéryle, le tétraisostéarate de pentaérythrityle, le tri décyl-2 tétradécanoate de glycéryle, les silicones phénylées, l'huile de sésame, et leurs mélanges.

Plus généralement, le corps gras liquide à température ambiante et à pression atmosphérique peut être présent à raison de 0,01 à 90 % en poids et notamment de 0,1 à 85 % en poids, par rapport au poids de la phase grasse.

La composition cosmétique peut comporter, notamment dans le cas où elle est destinée à être appliquée sur les lèvres, une phase huileuse ayant un indice de réfraction compris entre 1,47 et 1,51, ce qui peut permettre d'obtenir une brillance relativement élevée.

En ce qui concerne le corps gras solide à température ambiante et à pression atmosphérique, il peut être choisi parmi les cires, les gommes et leurs mélanges. Ce corps gras solide peut être présent à raison de 0,01 à 50 %, notamment de 0,1 à 40 % et en particulier de 0,2 à 30 % en poids, par rapport au poids total de la phase grasse.

En particulier, la composition peut contenir au moins une cire.

Par "cire" au sens de la présente invention, on entend un composé gras lipophile, solide à température ambiante (25°C), à changement d'état solide/liquide réversible, ayant une température de fusion supérieure à 30°C pouvant aller jusqu'à 200° C, une dureté supérieure à 0,5 MPa, et présentant à l'état solide une organisation cristalline anisotrope. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Les cires utilisables dans l'invention sont des composés solides à température ambiante, destinés à structurer la composition en particulier sous forme de stick ; elles peuvent être hydrocarbonées, fluorées et/ou siliconées et être d'origine végétale, minérale, animale et/ou synthétique. En particulier, elles présentent une température de fusion supérieure à 40°C et mieux supérieure à 45°C.

Comme cire utilisable dans l'invention, on peut citer celles généralement utilisées dans le domaine cosmétique : elles sont notamment d'origine naturelle comme la cire d'abeilles, la cire de Carnauba, de Candelilla, d'Ouricoury, du Japon, de fibres de liège ou de canne à sucre, de riz, de Montan, la paraffine, les cires de lignite ou microcristalline, la cérésine ou l'ozokérite, les huiles hydrogénées comme l'huile de jojoba ; les cires synthétiques comme les cires de polyéthylène issues de la polymérisation ou copolymérisation de l'éthylène et les cires de Fischer-Tropsch ou encore des esters d'acides gras comme l'octacosanyl stéarate, les glycérides concrets à 40°C et mieux à 45°C, les cires des ilicones comme les alkyl- ou alkoxydiméthicones ayant une chaîne alkyle ou alcoxy de 10 à 45 atomes de carbone, les esters de poly(di)méthylsiloxane solide à 40°C dont la chaîne ester comporte au moins 10 atomes de carbone ; et leurs mélanges.

La composition peut comprendre des gommes. Les gommes utilisables dans l'invention se présentent généralement sous forme solubilisée dans une huile, les polymères sont solides à température ambiante et les résines peuvent être liquides ou solides à température ambiante.

Par gomme on entend un corps gras qui se présente sous forme de polymère solide à température ambiante ayant un poids moléculaire moyen en poids de 50 000 à 1 000 000. La gomme est souvent vendue en dispersion dans un solvant organique, du type huile de silicone.

La nature et la quantité des gommes ou cires sont fonction des propriétés mécaniques et des textures recherchées. A titre indicatif, la cire peut représenter de 0,01 à 50 %, de préférence de 2 à 40 %, et mieux de 5 à 30 % du poids total de la composition.

Au sens de l'invention, on entend désigner sous ce terme tout composé, organique et/ou inorganique introduit dans la composition cosmétique afin d'ajuster ses propriétés en terme de texture ou en d'autres termes pour contrôler ses propriétés rhéologiques. Les pigments et nacres sont notamment exclus de cette définition.

Selon une variante particulière de l'invention, les compositions cosmétiques comprennent moins de 15% en poids, en particulier moins de 10% en poids, notamment moins de 7% en poids de charge par rapport au poids total de la composition.

Il peut notamment s'agir de charges sphériques comme par exemple le talc, le stéarate de zinc, le mica, le kaolin, les poudres de polyamide (Nylon®) (Orgasol® de chez Atochem), les poudres de polyéthylène, les poudres de polymères de tétrafluoroéthylène (Téflon®), l'amidon, le nitrure de bore, des microsphères polymériques telles que celles de chlorure de polyvinylidène/acrylonitrile comme l'Expancel® (Nobel Industrie), de copolymères d'acide acrylique (Polytrap® de la société Dow Corning), les microbilles de résine de silicone (Tospearls® de Toshiba, par exemple), et les organopolysiloxanes élastomères.

La composition selon l'invention peut en outre contenir des agents tensioactifs émulsionnants présents notamment en une proportion allant de 0,1 à 30 % en poids, et mieux de 5 % à 15 % en poids, par rapport au poids total de la composition.

Ces agents tensioactifs peuvent être choisis parmi des agents tensioactifs anioniques ou non ioniques. On peut se reporter au document « Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs utilisés préférentiellement dans la composition selon l'invention sont choisis :

- parmi les tensioactifs non-ioniques : les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de $C_1$-$C_6$ alkyl glucose polyoxyéthylénés, et leurs mélanges,
- parmi les tensioactifs anioniques : les acides gras en $C_{16}$-$C_{30}$ neutralisés par les amines, l'ammoniaque ou les sels alcalins, et leurs mélanges.

On utilise de préférence des tensioactifs permettant l'obtention d'émulsion huile-dans-eau ou cire-dans-eau.

La composition de l'invention, comprend généralement au moins un agent de coloration pouvant notamment être présent à raison de 0,01 % à 40 % en poids, notamment de 0,01 % à 30 % en poids et en particulier de 0,05 % à 25 % en poids, par rapport au poids total de la composition.

Ces ou cet agent de coloration peuvent être choisis parmi les pigments, les colorants hydrosolubles ou liposolubles, les nacres et leurs mélanges.

Par pigments, il faut comprendre des particules blanches ou colorées, minérales ou organiques, insolubles dans la phase hydrophile liquide, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées.

Les pigments peuvent être présents dans la composition à raison de 0,01 % à 25 % en poids, en particulier de 0,01 % à 15 % en poids, et notamment de 0,02 % à 5 % en poids par rapport au poids de la composition.

Comme pigments minéraux utilisables dans l'invention, on peut citer les oxydes de titane, de zirconium ou de cérium ainsi que les oxydes de zinc, de fer ou de chrome, le bleu ferrique, le violet de manganèse, le bleu outremer et l'hydrate de chrome. Parmi les pigments organiques utilisables dans l'invention, on peut citer le noir de carbone, les pigments de type D & C, et les laques à base de carmin de cochenille, de baryum, strontium, calcium, aluminium ou encore les dicéto pyrrolopyrrole (DPP) décrits dans les documents EP-A-542669, EP-A-787730, EP-A-787731 et WO-A- 96/08537. La quantité et/ou le choix de ces pigments sont généralement ajustés en prenant en compte la quantité en nanotubes présente dans la composition cosmétique considérée.

Les nacres peuvent être présentes dans la composition à raison de 0,01 % à 25 % en poids, notamment de 0,01 % à 15 % en poids, et en particulier de 0,02 % à 5 % en poids, par rapport au poids total de la composition.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique

ou de l'oxyde de chrome, le mica titane avec un pigment organique du type précité ainsi que les pigments nacrés à base d'oxychlorure de bismuth.

La composition peut comprendre également des colorants hydrosolubles ou liposolubles en une teneur allant de 0,01 % à 6 % en poids, par rapport au poids total de la composition, notamment allant de 0,01 % à 3 % en poids. Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, et le jaune quinoléine. Les colorants hydrosolubles sont par exemple le jus de betterave et le bleu de méthylène.

La composition selon l'invention peut, de plus, comprendre tous les ingrédients classiquement utilisés dans les domaines concernés et plus spécialement dans le domaine cosmétique et dermatologique. Ces ingrédients sont en particulier choisis parmi les vitamines, les antioxydants, les épaississants, les oligo-éléments, les adoucissants, les séquestrants, les parfums, les agents alcalinisants ou acidifiants, les conservateurs, les filtres UV, les actifs hydrophiles ou lipophiles et leurs mélanges. Les quantités de ces différents ingrédients sont celles classiquement utilisées dans les domaines concernés et par exemple de 0,01 % à 20 % du poids total de la composition.

Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction considérée.

La composition de l'invention peut être obtenue selon les procédés de préparation classiquement utilisés en cosmétique ou en dermatologie.

La composition de l'invention peut se présenter sous la forme de composition solide, compactée ou coulée en stick ou en coupelle, pâteuse ou liquide. Avantageusement, elle se présente sous forme solide, à savoir sous forme dure (ne s'écoulant pas sous son propre poids) notamment coulée ou compactée, par exemple en stick ou en coupelle.

En l'occurrence, elle peut se présenter sous la forme de rouges à lèvres, baumes à lèvres, fonds de teint coulés, produits anti-cernes, produits « correcteurs » ou « embellisseurs » de teint, et/ou fards à paupières ou à joues.

Elle peut toutefois se présenter sous forme de pâte, de solide ou de crème. Elle peut être une émulsion huile-dans-eau ou eau-dans-huile, un gel anhydre, solide ou souple ou encore sous forme de poudre libre ou compactée et même sous forme biphasique. Selon une variante particulière, elle se présente sous la forme d'une émulsion.

La composition selon l'invention peut se présenter sous la forme d'une composition, colorée ou non, sous forme d'une composition de protection solaire ou de démaquillage

ou encore sous forme d'une composition hygiénique. Elle contient notamment des actifs cosmétiques. Elle peut alors être utilisée comme base de soin ou de traitement pour la peau comme les mains ou le visage ou pour les lèvres (baumes à lèvres, protégeant les lèvres du froid et/ou du soleil et/ou du vent) ou encore comme déodorant. Comme actif cosmétique utilisable dans l'invention, on peut citer les vitamines A, E, C, B3, les provitamines comme le D-panthénol, les actifs apaisants comme l'$\alpha$-bisabolol, l'aloe vera, l'allantoïne, les extraits de plantes ou les huiles essentielles, les agents protecteurs ou restructurants comme les céramides, les actifs fraîcheur comme le menthol et ses dérivés, les émollients (beurre de cacao, diméthicone), les hydratants (arginine PCA), les actifs antirides, les acides gras essentiels, et leurs mélanges.

La composition de l'invention peut également se présenter sous la forme d'un produit de maquillage de la peau, en particulier du visage comme un fond de teint, un blush, un fard comme un produit de tatouage semi-permanent ou de maquillage des lèvres comme un rouge ou un brillant à lèvres, présentant éventuellement des propriétés de soin ou de traitement, un produit de maquillage des phanères comme par exemple un vernis à ongles, un mascara, un eyeliner, un produit de coloration ou de soin des cheveux.

Bien entendu la composition de l'invention doit être cosmétiquement acceptable, à savoir non toxique et susceptible d'être appliquée sur la peau, les phanères ou les lèvres d'êtres humains.

Les exemples de compositions ci-après sont donnés à titre illustratif et sans caractère limitatif.

**Exemple 1 : synthèse d'une dispersion de particules de polymère :**

On prépare une dispersion de copolymère non réticulé d'acrylate de méthyle et d'acide acrylique dans un rapport 85/15, dans l'heptane, selon la méthode de l'exemple 1 du document EP-A-749 746. Lorsque la polymérisation est terminée, on ajoute du polyisobutène hydrogéné et on distille l'heptane sous vide.

On obtient ainsi une dispersion de particules de poly(acrylate de méthyle/acide acrylique) stabilisées en surface dans le polyisobutène hydrogéné par un copolymère dibloc séquencé p olystyrène/copoly(éhylène-propylène) vendu sous le nom de Kraton G1701, ayant un taux de matière sèche de 21% en poids, et une taille moyenne de particule égale à 150 nm.

**Exemple 2: Rouge à lèvres**

| **Nom chimique** | **Exemple 2** |
|---|---|
| Dispersion de polymère de l'exemple 1 | 30 |
| Triglycéride d'acide 2-décyl tétradécanoïque | 2.02 |
| Copolymères dimères dilinoleyl diols / dimères dilinoléiques | 10 |
| Octyldodécanol | 9 |
| BHT | 0.07 |
| Mélange de parabens | 0.4 |
| Polycaprolactone de PM 1250 g/mol (CAPA 1215 de SOLVAY) | 9 |
| Polyacrylate de stéryle (Interlimer IPA 13-1 de | - |

(suite)

| Nom chimique | Exemple 2 |
|---|---|
| LANDEC) | |
| Copolymère vinyl pyrrolidone / eicosène | 6 |
| Cire microcristalline | 10 |
| Cire de polyéthylène | 2 |
| Cire de polyméthylène de PF 40°C | 10 |
| Pigments | 6.03 |
| Silice enrobée dimethicone | 5 |
| Parfum | 0.48 |
| TOTAL | 100 |

Mode opératoire :

Toutes les matières premières sont pesées dans un poêlon à double paroi à circulation d'huile, puis mises à chauffer sous agitation (turbine).
Après fonte totale des matières et homogénéisation du mélange, celui-ci est broyé 5 fois de suite à la broyeuse tricylindre. La pâte obtenue est mise à stabiliser pendant 24 heures à 20°C puis conditionnée dans des bouillottes.

Evaluation cosmétique (in vivo) :

La formule est testée en demi-lèvres sur 7 femmes. Les testrices notent sur une échelle de 1 à 10 le niveau de migration de la formule au bout d'une heure (1 = peu, 10 = beaucoup). La migration de l'exemple 2 est égale à 1,14.

Evaluation (in vitro) :

La formule est testée in vitro selon le t est de « Push & Pull », consistant à évaluer la résistance de la formule à l'eau et à l'huile. Les résultats sont les suivants :

| | Exemple 2 |
|---|---|
| Tenue à la pression | 102.25 |
| Tenue à la pression + essuyage | 60.43 |

Exemple 4 : rouge à lèvres

| | |
|---|---|
| Isoparaffine hydrogénée (Parleam de NOF) | 5,7 |
| Copolymère dimère diol dilinoléique/dimère Dilinoléique (LUSPLAN DD-DA 5 de NFC) | 5,7 |
| Mélange d'huiles végétales hydrogénées (soja/coprah/palme/colza) | |
| LIPEX 451 de KARLSHAMNS | 20 |
| Cire de polyéthylène (PM 500) | 10,5 |
| Cire ozokérite (OZOKERITE WAX SP 1020 P de STRAHL et PITCH) | 2,8 |
| Pigments | 10,2 |
| Polybutène (Indopol H1500) | 5,0 |
| Dispersion de polymère dans l'isohexadécane | 40 |

Préparation de la dispersion de polymère :

On prépare une dispersion de copolymère non réticulé d'acrylate de méthyle et d'acide acrylique dans un rapport 95/5, dans l'isododécane, selon la méthode de l'exemple 1 du document EP-A-749 746, en remplaçant l'heptane par l'isododécane. On obtient ainsi une dispersion de particules de poly(acrylate de méthyle/acide acrylique) stabilisées en surface dans l'isododécane par un copolymère dibloc séquencé polystyrène/copoly(éhylène-propylène) vendu sous le nom de Kraton G1701, ayant un taux de matière sèche de 25% en poids.

Le rouge à lèvres présente ne bonne tenue, un bon niveau de brillance et ne transfert pas.

## Revendications

1. Composition cosmétique comprenant dans un milieu physiologiquement acceptable au moins un ester de dimère diol et d'au moins un acide mono-carboxylique en $C_4$ à $C_{34}$ ou di-carboxylique, et au moins un agent filmogène.

2. Composition selon la revendication 1, **caractérisée en ce que** l'acide mono-carboxylique est choisi parmi

   - les acides linéaires saturés tels que l'acide butanoïque, l'acide pentanoïque, l'acide hexanoïque, l'acide heptanoïque, l'acide octanoïque, l'acide nonanoïque, l'acide décanoïque, l'acide undécanoïque, l'acide dodécanoïque, l'acide tridécanoïque, l'acide tétradécanoïque, l'acide heptadécanoïque, l'acide hexadécanoïque, l'acide pentadécanoïque, l'acide octadécanoïque, l'acide nonadécanoïque, l'acide eicosanoïque, l'acide docosanoïque, l'acide tétracosanoïque,
   - les acides gras,
   - des hydroxyacides tels que l'acide 2-hydroxybutanoïque, l'acide 2-hydropentanoïque, l'acide 2-hydroxyhexanoïque, l'acide 2-hydroxyheptanoïque, l'acide 2-hydroxyoctanoïque, l'acide 2-hydroxynonanoïque, l'acide 2-hydroxydécanoïque, l'acide 2-hydroxyundécanoïque, l'acide 2-hydroxydodécanoïque, l'acide 2-hydroxytridécanoïque, l'acide 2-hydroxytétradécanoïque, l'acide 2-hydroxyhexadécanoïque, l'acide 2-hydroxyheptadécanoïque, l'acide 2-hydroxyoctadécanoïque, l'acide 12-hydroxyoctadécanoïque, l'acide 2-hydroxynonadécanoïque, l'acide 2-hydroxyeicosanoïque, l'acide 2-hydroxydocosanoïque, l'acide 2-hydroxytétracosanoïque et
   - des acides cycliques tels que l'acide cyclohexanoïque, la rosine hydrogénée, la rosine, l'acide abiétique, l'acide abiétique hydrogéné, l'acide benzoïque, l'acide p-oxybenzoïque, l'acide p-aminobenzoïque, l'acide cinnamique, l'acide p-méthoxycinnamique, l'acide salicylique, l'acide gallique, l'acide pyrrolidonecarboxylique, l'acide nicotinique
   - et leurs mélanges.

3. Composition selon la revendication 2, **caractérisée en ce que** l'acide gras est choisi parmi :

   - des acides gras ramifiés tels que l'acide isobutanoïque, l'acide isopentanoïque, l'acide pivalique, l'acide isohexanoïque, l'acide isoheptanoïque, l'acide isooctanoïque, l'acide diméthyloctanoïque, l'acide isononanoïque, l'acide isodécanoïque, l'acide isoundécanoïque, l'acide isododécanoïque, l'acide isotridécanoïque, l'acide isotétradécanoïque, l'acide isopentadécanoïque, l'acide isohexadécanoïque, l'acide isoheptadécanoïque, l'acide isooctadécanoïque, l'acide isononadécanoïque, l'acide isoeicosanoïque, l'acide 2-éthylhexanoïque, l'acide 2-butyloctanoïque, l'acide 2-hexyldécanoïque, l'acide 2-octyldodécanoïque, l'acide 2-décyltétradécanoïque, l'acide 2-dodécylhexadécanoïque, l'acide 2-tétradécyloctadécanoïque, l'acide 2-hexadécyloctadécanoïque,
   - d es acides gras linéaires insaturés en $C_8$ à $C_{34}$, tels que l'acide undécénoïque, l'acide lindérique, l'acide myristoléique, l'acide palmitoléique, l'acide oléique, l'acide linoléique, l'acide élaïdinique, l'acide gadolénoïque, l'acide eicosapentaénoïque, l'acide docosahexaénoïque, l'acide érucique, l'acide brassidique, l'acide arachidonique et
   - des acides gras d'origine naturelle, tels que les acides gras d'huile d'orange, d'huile d'avocat, d'huile de macadamia, d'huile d'olive, d'huile de soja hydrogénée, d'huile de jojoba, d'huile de palme, d'huile de ricin, d'huile de germe de blé, d'huile de safran, d'huile de grains de coton, d'huile de vison
   - et leurs mélanges.

4. Composition selon la revendication 1, **caractérisée en ce que** l'acide dicarboxylique est choisi parmi

   - les composés de formule (I) $HOOC-(CH_2)_n-COOH$ dans laquelle n est un nombre entier de 1 à 16, de préférence de 3 à 16, et

- les dimères diacides obtenus par dimérisation d'au moins un acide mono-carboxylique insaturé.

**5.** Composition selon la revendication 4, **caractérisée en ce que** l'acide mono-carboxylique insaturé est un acide gras insaturé en $C_8$ à $C_{34}$, notamment en $C_{12}$ à $C_{22}$, en particulier en $C_{16}$ à $C_{20}$ et plus particulièrement en $C_{18}$.

**6.** Composition selon la revendication 5, **caractérisée en ce que** l'acide gras insaturé est choisi parmi l'acide undécénoïque, l'acide lindérique, l'acide myristoléique, l'acide palmitoléique, l'acide oléique, l'acide linoléique, l'acide élaïdinique, l'acide gadolénoïque, l'acide eicosapentaénoïque, l'acide docosahexaénoïque, l'acide érucique, l'acide brassidique, l'acide arachidonique et leurs mélanges.

**7.** Composition selon la revendication 4, **caractérisée en ce que** l'acide dicarboxylique est un dimère diacide.

**8.** Composition selon l'une quelconque des revendications 4 à 7, **caractérisée en ce que** le dimère diacide est le diacide dilinoléique.

**9.** Composition selon la revendication 4, **caractérisée en ce que** le dimère diacide est saturé.

**10.** Composition selon l'une quelconque des revendications précédentes, **caractérisée en ce que** le dimère diol dérive de l'hydrogénation d'un dimère diacide.

**11.** Composition selon la revendication 10, **caractérisée en ce que** le dimère diacide dérive de la dimérisation d'un acide gras insaturé, notamment en $C_8$ à $C_{34}$ et en particulier en $C_{12}$ à $C_{22}$, en particulier en $C_{16}$ à $C_{20}$, et plus particulièrement en $C_{18}$.

**12.** Composition selon la revendication précédente, **caractérisée en ce que** l'acide gras insaturé est tel que défini en revendication 5 ou 6.

**13.** Composition selon l'une quelconque des revendications 10 à 12, **caractérisée en ce que** le dimère diol dérive de l'hydrogénation du diacide dilinoléique.

**14.** Composition selon l'une quelconque des revendications 10 à 13, **caractérisée en ce que** le dimère diol est saturé.

**15.** Composition selon l'une quelconque des revendications 12 à 14, **caractérisée en ce que** le dimère diacide est identique au dimère diacide dont dérive le dimère diol.

**16.** Composition selon les revendications 8 et 13, **caractérisée en ce que** l'ester est un composé de formule générale (II) :

$$HO\text{-}R^1\text{-}(\text{-}OCO\text{-}R^2\text{-}COO\text{-}R^1\text{-})_h\text{-}OH \qquad (I)$$

dans laquelle :

$R^1$ représente un reste de dimère diol obtenu par hydrogénation du diacide dilinoléique,
$R^2$ représente un reste de diacide dilinoléique hydrogéné, et
h représente un entier variant de 1 à 9.

**17.** Composition selon l'une quelconque des revendications précédentes, **caractérisée en ce que** l'ester est présent à raison de 1 à 99 % en poids, notamment de 2 à 60 % en poids, en particulier de 5 à 40 % en poids et plus particulièrement de 10 à 35 % en poids par rapport au poids total de la composition.

**18.** Composition selon l'une quelconque des revendications précédentes, **caractérisée en ce que** le polymère filmogène est choisi parmi les homopolymères et les copolymères liposolubles et amorphes des oléfines, des cyclooléfines, du butadiène, de l'isoprène, du styrène, des éthers, des esters ou amides vinyliques, des esters ou amides de l'acide (méth)acrylique contenant un groupement alkyle e n $C_{4\text{-}50}$ linéaire, ramifié ou cyclique, et préférablement amorphes.

**19.** Composition selon l'une quelconque d es revendications 1 à 17, **caractérisée en ce que** le polymère filmogène est choisi parmi les homopolymères et les copolymères obtenus à partir de monomères choisis parmi le groupe constitué par le (méth)acrylate d'isooctyle, le (méth)acrylate d'isononyle, le (méth)acrylate de 2-éthylhexyle, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth)acrylate de tridécyle, le (méth)acrylate de stéaryle, ou des mélanges de ceux-ci, en particulier un copolymère d'acrylate d'alkylelacrylate de cycloalkyle et les copolymères de vinylpyrrolidone/décadécène.

**20.** Composition l'une quelconque des revendications 1 à 17, **caractérisée en ce que** le polymère filmogène est choisi parmi les polycondensats amorphes et liposolubles, ne comprenant préférablement pas de groupements donneurs d'interactions hydrogène, en particulier les polyesters ayant des chaînes latérales alkyle en $C_{4-50}$ ou bien les polyesters résultant de la condensation de dimères d'acides gras, voire les polyesters comprenant un segment siliconé sous la forme d'une séquence, greffon ou groupement terminal, solide à température ambiante.

**21.** Composition l'une quelconque des revendications 1 à 17, **caractérisée en ce que** le polymère filmogène est choisi parmi les polysaccharides amorphes et liposolubles comprenant des chaînes latérales alkyl(éther ou ester), en particulier l'éthylcellulose, les polymères de silicone-acrylique greffés ayant un squelette siliconé, des greffons acryliques ou ayant un squelette acrylique, les greffons de silicone.

**22.** Composition selon l'une quelconque des revendications 1 à 1 7, **caractérisée en ce que** le polymère filmogène porte des groupements fluorés.

**23.** Composition selon la revendication 22, **caractérisée en ce que** le polymère filmogène est choisi parmi les copolymères de (méth)acrylate d'alkyle/(méth)acrylate de perfluoroalkyle.

**24.** Composition selon l'une quelconque des revendications 1 à 17, **caractérisée en ce que** le polymère filmogène est choisi parmi les polymères ou copolymères résultant de la polymérisation ou la copolymérisation d'un monomère éthylénique, comprenant une ou plusieurs liaisons éthyléniques, de préférence conjuguées.

**25.** Composition selon la revendication 24, **caractérisée en ce que** le polymère ou copolymère résultant de la polymérisation ou la copolymérisation d'un monomère éthylénique est choisi parmi les polystyrène/copoly(éthylène/butylène).

**26.** Composition selon l'une quelconque des revendications 1 à 1 7, **caractérisée en ce que** le polymère filmogène est choisi parmi les polymères ayant un squelette organique non siliconé greffé par des monomères contenant un polysiloxane.

**27.** Composition selon l'une quelconque des revendications 1 à 17, dans laquelle ledit au moins un polymère filmogène est choisi parmi les polymères siliconés greffés par des monomères organiques non siliconés.

**28.** Composition selon l'une quelconque des revendications 1 à 17, dans laquelle ledit polymère filmogène est choisi parmi les polyamides siliconés.

**29.** Composition selon l'une quelconque des revendications 1 à 17, dans laquelle ledit polymère filmogène est choisi parmi les résines de silicone telles que les siloxysilicates, les polysilsesquioxanes et leur mélange.

**30.** Composition selon l'une quelconque des revendications 1 à 1 7, **caractérisée en ce que** le polymère filmogène est un polymère séquencé comprenant au moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.

**31.** Composition selon la revendication 30, **caractérisée en ce** le polymère séquencé comprend au moins une première séquence ayant une température de transition vitreuse (Tg) supérieure ou égale à 40°C et et au moins une deuxième séquence ayant une température de transition vitreuse inférieure ou égale à 20°C, lesdites première et deuxième séquences étant reliées entre elles par une séquence intermédiaire comprenant au moins un monomère constitutif de la première séquence et au moins un monomère constitutif de la deuxième séquence.

**32.** Composition selon la revendication 31, **caractérisée en ce que** la première séquence est issue d'au moins un momomère choisi parmi le méthacrylate de méthyle, le méthacrylate d'isobutyle, le (méth)acrylate d'isobornyle.

**33.** Composition selon l'une des revendications 31 ou 32, **caractérisée en ce que** la deuxième séquence est issue d'au moins un momomère choisi parmi les acrylates d'alkyle dont la chaîne alkyle comprend de 1 à 10 atomes de carbone, à l'exception du groupe butyle.

**34.** Composition selon l'une des revendications 31 à 33, **caractérisée en ce que** la première ou la deuxième séquence ou les deux comprennent au moins un motif issu d'un monomère additionnel choisi parmi les monomères hydrophiles, les monomères à insaturation éthylénique comprenant un ou plusieurs atomes de silicium, et leurs mélanges.

**35.** Composition selon la revendication précédente, **caractérisée en ce que** chacune des première et deuxième séquence du polymère séquencé comprend au moins un monomère choisi parmi les esters d'acide (meth)acrylique, et éventuellement au moins un monomère choisi parmi l'acide (meth)acrylique et leurs mélanges.

**36.** Composition selon l'une des revendications 30 à 35, **caractérisée en ce que** chacune des première et deuxième séquence du polymère séquencé est issue en totalité d'au moins un monomère choisi parmi l'acide acrylique, les esters d'acide (méth)acrylique, et éventuellement d'au moins un monomère choisi parmi l'acide (méth)acrylique, et leurs mélanges.

**37.** Composition selon l'une des revendications 1 à 17, **caractérisée en ce qu'**elle comprend une phase grasse et que les particules de polymère sont solides et insolubles dans ladite phase grasse à une température de 25°C.

**38.** Composition selon la revendication précédente, **caractérisée en ce que** le polymère n'est pas une cire.

**39.** Composition selon l'une des revendications 37 ou 38, **caractérisée en ce que** les particules de polymère ont une taille moyenne comprise entre 5 et 800 nm.

**40.** Composition selon l'une des revendications 37 à 39, **caractérisée en ce que** le polymère est un polymère hydrocarboné.

**41.** Composition selon l'une des revendications 37 à 40, **caractérisée en ce que** les particules de polymère sont insolubles dans les alcools hydrosolubles.

**42.** Composition selon l'une des revendications 37 à 41, **caractérisée en ce que** les particules de polymère sont choisies parmi les polyuréthanes, polyuréthanes-acryliques, polyurées, polyurée/polyuréthanes, polyester-polyuréthanes, polyéther-polyuréthanes, polyesters, polyesters amides, polyesters à chaîne grasse, alkydes ; polymères ou copolymères acryliques et/ou vinyliques ; copolymères acryliques-silicone ; polyacrylamides ; polymères siliconés, polymères fluorés et leurs mélanges.

**43.** Composition selon l'une des revendications 37 à 42, **caractérisée en ce que** le polymère représente, en matière sèche, de 5 à 40% du poids total de la composition, de préférence de 5 à 35% et mieux encore de 8 à 30%.

**44.** Composition selon l'une des revendications 37 à 43, **caractérisée en ce qu'**elle comprend un agent stabilisant choisi parmi les polymères séquencés, les polymères greffés, les polymères statistiques, et leurs mélanges.

**45.** Composition selon la revendication précédente, **caractérisée en ce que** le stabilisant est un polymère dibloc.

**46.** Composition selon l'une des revendications 37 à 45, **caractérisée en ce que** la phase grasse contient moins de 40%, de préférence moins de 30%, de préférence encore au moins de 20%, de préférence encore moins de 10% en poids d'au moins une huile volatile.

**47.** Composition selon l'une des revendications 37 à 46, **caractérisée en ce que** la phase grasse est exempte d'huile volatile.

**48.** Composition selon l'une des revendications 37 à 47, **caractérisée en ce que** la phase grasse contient une huile non volatile, de préférence hydrocarbonée.

**49.** Composition selon la revendication précédente, **caractérisée en ce que** l'huile non volatile représente de 5% à 80% en poids du poids total de la composition.

**50.** Composition selon la revendication 49, **caractérisée en ce que** l'huile non volatile représente de 10% à 60% en poids, de préférence de 10% à 40% en poids, de préférence encore de 15 à 30% en poids du poids total de la composition.

**51.** Composition selon l'une des revendications 48 à 50, **caractérisée en ce que** l'huile non volatile est apolaire.

**52.** Composition selon la revendication précédente **caractérisée en ce que** l'huile hydrocarbonée non volatile est choisie parmi les hydrocarbures linéaires ou ramifiés tels que les huiles de paraffine, de vaseline et de naphtalène, le polyisobutène hydrogéné, et leurs mélanges.

**53.** Composition selon l'une des revendications 1 à 17, **caractérisée en ce qu'**elle comprend une phase grasse liquide et que le polymère filmogène est un polymère éthylénique greffé comprenant un squelette éthylénique insoluble dans ladite phase grasse liquide et des chaînes latérales liées de manière covalente audit squelette et solubles dans ladite phase grasse liquide.

**54.** Composition selon la revendication 53, **caractérisée en ce que** le polymère éthylénique greffé est un polymère acrylique greffé.

**55.** Composition selon l'une des revendications 53 ou 54, **caractérisée en ce que** le polymère éthylénique greffé est un polymère acrylique susceptible d'être obtenu par polymérisation radicalaire dans un milieu organique de polymérisation :

- d'au moins un monomère acrylique, et éventuellement d'au moins un monomère additionnel vinylique non acrylique, pour former ledit squelette insoluble ; et
- d'au moins un macromonomère comportant un groupe terminal polymérisable pour former les chaînes latérales, ledit macromonomère ayant une masse moléculaire moyenne en poids supérieure ou égale à 200 et la teneur en macromonomère polymérisé représentant de 0,05 à 20% en poids du polymère.

**56.** Composition selon la revendication précédente, **caractérisée en ce que** le monomère acrylique est choisi parmi, seul ou en mélange, les monomères suivants, ainsi que leurs sels :

- (i) les (méth)acrylates de formule :

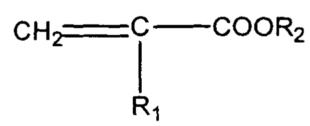

dans laquelle :

$R_1$ désigne un atome d'hydrogène ou un groupe méthyle ;
$R_2$ représente un groupe choisi parmi :

un groupe alkyle linéaire ou ramifié, comprenant de 1 à 6 atomes de carbone, ledit groupe pouvant comporter dans sa chaîne un ou plusieurs hétéroatomes choisis parmi O, N et S; et/ou pouvant comporter un ou plusieurs substituants choisis parmi - OH, les atomes d'halogène (F, Cl, Br, I) et -NR'R" avec R' et R" identiques ou différents choisis parmi les alkyles linéaires ou ramifiés en $C_1$-$C_4$; et/ou pouvant être
substitué par au moins un groupe polyoxyalkylène, notamment polyoxyéthylène et/ou polyoxypropylène, ledit groupe polyoxyalkylène étant constitué par la répétition de 5 à 30 motifs oxyalkylène ;
un groupe alkyle cyclique comprenant de 3 à 6 atomes de carbone, ledit groupe pouvant comporter dans sa chaîne un ou plusieurs hétéroatomes choisis parmi O, N et S, et/ou pouvant comporter un ou plusieurs substituants choisis parmi OH et les atomes d'halogène (F, Cl, Br, I) ;

- (ii) les (méth)acrylamides de formule :

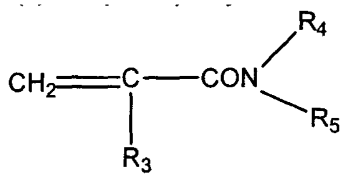

dans laquelle :

$R_3$ désigne un atome d'hydrogène ou un groupe méthyle ;
$R_4$ et $R_5$, identiques ou différents, représentent un atome d'hydrogène ou un groupe alkyle, linéaire ou ramifié, comportant de 1 à 6 atomes de carbone, pouvant comporter un ou plusieurs substituants choisis parmi -OH, les atomes d'halogène (F, Cl, Br, I) et -NR'R" avec R' et R " identiques ou différents choisis parmi les alkyles linéaires ou ramifiés en $C_1$-$C_4$;ou
$R_4$ représente un atome d'hydrogène et $R_5$ représente un groupe 1,1-diméthyl-3-oxobutyle ;

- (iii) les monomères (méth)acryliques comprenant au moins une fonction acide carboxylique, phosphorique ou sulfonique, tels que l'acide acrylique, l'acide méthacrylique, l'acide acrylamidopropanesulfonique.

**57.** Composition selon la revendication 56, **caractérisée en ce que** le monomère acrylique est choisi parmi les (méth) acrylates de méthyle, d'éthyle, de propyle, de butyle, d'isobutyle; les (méth)acrylates de méthoxyéthyle ou d'éthoxyéthyle ; le méthacrylate de trifluoroéthyle; le méthacrylate de diméthylaminoéthyle, le méthacrylate de diéthylaminoéthyle, le (méth)acrylate de 2-hydroxypropyle, le (méth)acrylate de 2-hydroxyéthyle ; le diméthylaminopropylméthacrylamide; et leurs sels.

**58.** Composition selon l'une des revendications 56 ou 57, **caractérisée en ce que** le monomère acrylique est choisi parmi l'acrylate de méthyle, l'acrylate de méthoxyéthyle, le méthacrylate de méthyle, le méthacrylate de 2-hydroxyéthyle, l'acide (meth)acrylique, le méthacrylate de diméthylaminoéthyle, et leurs mélanges.

**59.** Composition selon l'une quelconque des revendications 53 à 58, **caractérisée par le fait que** le polymère greffé comprend de l'acide (méth)acrylique.

**60.** Composition selon l'une quelconque des revendications 55 à 59, **caractérisée par le fait que** les monomères acryliques comprennent au moins l'acide (méth)acrylique et au moins un monomère choisi parmi les (méth)acrylates et les (méth)acrylamides décrits aux points (i) et (ii) dans la revendication 56.

**61.** Composition selon l'une quelconque des revendications 55 à 60, **caractérisée par le fait que** les monomères acryliques comprennent au moins l'acide (méth)acrylique et au moins un monomère choisi parmi les (méth)acrylates d'alkyle en $C_1$-$C_3$.

**62.** Composition selon l'une des revendications 56 à 64, **caractérisée en ce que** le polymère acrylique greffé est susceptible d'être obtenu par polymérisation radicalaire d'un ou plusieurs monomère(s) acrylique(s) et d'un ou plusieurs monomère(s) additionnel(s) vinylique(s) non acrylique(s), et dudit macromonomère.

**63.** Composition selon la revendication précédente, **caractérisée en ce que** les monomères additionnels vinyliques non acryliques sont choisis parmi :

- les esters vinylique de formule : $R_6$-COO-CH=$CH_2$

dans laquelle $R_6$ représente un groupe alkyle linéaire ou ramifié, comprenant de 1 à 6 atomes, ou un groupe alkyle cyclique comportant de 3 à 6 atomes de carbone et/ou un groupe aromatique, par exemple de type benzénique, anthracénique, et naphtalénique ;

- les monomères vinyliques non acryliques comprenant au moins une fonction acide carboxylique, phosphorique ou sulfonique, tels que l'acide crotonique, l'anhydride maléique, l'acide itaconique, l'acide fumarique, l'acide maléique, l'acide styrènesulfonique, l'acide vinylbenzoïque, l'acide vinylphosphorique et les sels de ceux-ci ;

- les monomères vinyliques non acryliques comprenant au moins une fonction amine tertiaire, tels que la 2-vinylpyridine, la 4-vinylpyridine ; et leurs mélanges.

**64.** Composition selon l'une des revendications 56 à 67, **caractérisée en ce que** le macromonomère comporte à une des extrémités de la chaîne un groupe terminal polymérisable choisi parmi un groupe vinyle ou un groupe (méth) acrylate, et de préférence un groupe (méth)acrylate.

**65.** Composition selon l'une des revendications 53 à 64, **caractérisée en ce que** la phase grasse liquide comprend un composé organique liquide choisi parmi :

- les composés organiques liquides ayant un paramètre de solubilité global selon l'espace de solubilité de Hansen inférieur ou égal à 18 $(MPa)^{1/2}$,
- les monoalcools ayant un paramètre de solubilité global selon l'espace de solubilité de Hansen inférieur ou égal à 20 $(MPa)^{1/2}$ ; et
- leurs mélanges.

**66.** Composition selon l'une des revendications 1 à 17, **caractérisée en ce que** le polymère filmogène est choisi parmi les polyuréthanes, les polyuréthane-acryliques, les polyuréthanes-polyvinylpyrrolidones, les polyester-polyuréthanes, les polyéther-polyuréthanes, les polyurées, les polyurée/polyuréthanes, et leurs mélanges.

**67.** Composition selon la revendication 66, **caractérisée en ce que** le polymère filmogène est un copolymère de polyuréthane aliphatique, cycloaliphatique ou aromatique, un copolymère de polyurée/polyuréthane ou de polyurée comprenant, seul ou en tant que mélange :

- au moins une séquence d'origine polyester linéaire ou ramifiée, aliphatique et/ou cycloaliphatique et/ou aromatique, et/ou
- au moins une séquence d'origine polyéther aliphatique et/ou cycloaliphatique et/ou aromatique, et/ou
- au moins une séquence siliconée, substituée ou non substituée, ramifiée ou non ramifiée, par exemple de polydiméthylsiloxane ou de polyméthylphénylsiloxane, et/ou
- au moins une séquence comprenant des groupements fluorés.

**68.** Composition selon la revendication 66, **caractérisée en ce que** le polymère filmogène est choisi parmi les polyesters, les polyesteramides, les polyesters à chaîne grasse, les polyamides et les résines d'époxyester.

**69.** Composition selon la revendication 66, **caractérisée en ce que** le polymère filmogène est choisi parmi les polymères acryliques, les copolymères acryliques et les polymères vinyliques.

**70.** Composition selon l'une des revendications 1 à 17, **caractérisée en ce que** le polymère filmogène est choisi parmi :

- les protéines telles que les protéines d'origine végétale, telles que les protéines de blé ou de soja ; les protéines d'origine animale, telles que la kératine, par exemple les hydrolysats de kératine et les kératines sulfoniques ;
- les polymères anioniques, cationiques, amphotères ou non ioniques de la chitine ou du chitosane ;
- les polymères cellulosiques, tels que l'hydroxyéthyl cellulose, l'hydroxypropyl cellulose, la méthylcellulose, l'éthylhydroxyéthyl cellulose, la carboxyméthyl cellulose, et les dérivés quaternisés de la cellulose ;
- les polymères ou copolymères acryliques tels que les polyacrylates ou les polyméthacrylates ;
- les polymères vinyliques, tels que les polyvinylpyrrolidones, les copolymères du méthylvinyléther et de l'anhydride maléique, le copolymère de l'acétate de vinyle et de l'acide crotonique, les copolymères de la vinylpyrrolidone et de l'acétate de vinyle ;
- les copolymères de la vinylpyrrolidone et du caprolactame ; les alcools polyvinyliques ;
- les polymères éventuellement modifiés d'origine naturelle, tels que :

  . la gomme arabique, la gomme de guar, les dérivés du xanthane, la gomme de karaya ;
  . les alginates et les carraghénanes ;
  . les glycoaminoglycanes, l'acide hyaluronique et ses dérivés ;
  . la gomme laque, la gomme sandaraque, les dammars, l'élémis, les copals ;
  . l'acide désoxyribonucléique ;
  . les mucopolysaccharides, tels que l'acide hyaluronique, le sulfate de chondroïtine, et des mélanges de ceux-ci.

**71.** Composition selon l'une quelconque des revendications précédentes, **caractérisée par le fait qu'**elle comprend une huile volatile.

**72.** Composition selon la revendication précédente, **caractérisée par le fait qu'**elle comprend une huile volatile choisie parmi l'octaméthylcyclotétrasiloxane, le décaméthylcyclopentasiloxane, le dodécaméthylcyclohexasiloxane, l'heptaméthylhexyltrisiloxane, l'heptaméthyloctyltrisiloxane, l'octaméthyltrisiloxane, le décaméthyltétrasiloxane, l'isododécane, l'isodécane, l'isohexadécane.

**73.** Composition selon la revendication 71 ou 72, **caractérisée par le fait que** l'huile volatile est présente en une teneur allant de 1 % à 70 % en poids, par rapport au poids total de la composition, de préférence allant de 5 % à 50 % en poids, et préférentiellement allant de 10 % à 35 % en poids.

**74.** Composition selon l'une quelconque des revendications précédentes, **caractérisée par le fait qu'**elle comprend une huile non volatile.

**75.** Composition selon la revendication précédente, **caractérisée par le fait que** l'huile non volatile est présente en une teneur allant de 1 % à 80 % en poids, par rapport au poids total de la composition, de préférence allant de 5 % à 60 % en poids, et préférentiellement allant de 10 % à 50 % en poids.

**76.** Composition selon l'une des revendications précédentes , **caractérisée en ce que** la proportion en agent filmogène va de 0,5 à 60%, préférablement de 1 à 40%, plus préférablement de 2 à 30% en poids de matière sèche par rapport au poids total de la composition.

**77.** Composition cosmétique selon l'une quelconque des revendications précédentes, **caractérisée en ce qu'**elle comprend, en outre, une ou des matières colorantes choisies parmi les colorants hydrosolubles et les matières colorantes pulvérulentes, tels que les pigments, les nacres et les paillettes.

**78.** Composition selon la revendication précédente, **caractérisée en ce que** la matière colorante est présente en une teneur allant de 0,01 % à 50 % en poids, par rapport au poids de la composition, de préférence de 0,01 % à 30 % en poids.

**79.** Composition selon l'une quelconque des revendications précédentes, **caractérisée par le fait qu'**elle comprend au moins un corps gras choisi parmi les cires, les corps gras pâteux, les gommes et leurs mélanges.

**80.** Composition selon l'une quelconque des revendications précédentes, **caractérisée par le fait qu'**elle comprend un ingrédient cosmétique choisi parmi les vitamines, les épaississants, les gélifiants, les oligo-éléments, les adoucissants, les séquestrants, les parfums, les agents alcalinisants ou acidifiants, les conservateurs, les filtres solaires, les tensioactifs, les anti-oxydants, les fibres, les agents anti-chutes des cheveux, les agents de soin du cil, les agents anti-pelliculaires, les agents propulseurs, ou leurs mélanges.

**81.** Composition cosmétique selon l'une quelconque des revendications précédentes, **caractérisée en ce qu'**elle se présente sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de poudre, de pâte, notamment de pâte souple ou de pâte anhydre, de stick ou de solide coulé.

**82.** Composition cosmétique selon l'une quelconque des revendications 1 à 65, **caractérisée en ce qu'**elle se présente sous forme anhydre.

**83.** Composition cosmétique selon l'une quelconque des revendications précédentes, **caractérisée en ce qu'**il s'agit d'une composition de maquillage ou de soin des matières kératiniques.

**84.** Composition selon l'une des revendications 1 à 83, **caractérisée en ce qu'**il s'agit d'un produit de maquillage des yeux.

**85.** Composition selon l'une des revendications 1 à 83, **caractérisée en ce qu'**elle est un mascara.

**86.** Composition selon l'une des revendications 1 à 83, **caractérisée en ce qu'**il s'agit d'un produit de maquillage des

lèvres.

**87.** Composition selon l'une des revendications 1 à 83, **caractérisée en ce qu'**il s'agit d'un produit de maquillage du teint.

**88.** Composition selon l'une des revendications 1 à 83, **caractérisée en ce qu'**il s'agit d'un produit de maquillage des ongles.

**89.** Procédé de maquillage et/ou de soin de la peau, des lèvres comprenant l'application sur la peau, les lèvres et/ou les phanères d'au moins une composition selon l'une quelconque des revendications 1 à 88.

Office européen des brevets

# RAPPORT DE RECHERCHE EUROPEENNE

Numéro de la demande
EP 05 29 1036

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X,D | FR 2 795 309 A (NIPPON FINE CHEMICAL COMPANY LIMITED) 29 décembre 2000 (2000-12-29) * revendications 1-18 * * page 1, ligne 15 - ligne 21 * * page 2, ligne 16 - ligne 30 * * page 4, ligne 5 - page 6, ligne 9 * * page 9, ligne 7 - ligne 22 * * page 16, ligne 33 - page 17, ligne 2 * * page 17, ligne 33 - page 18, ligne 1 * * exemples 20-24,27,52,56,58,60,62,64 * | 1-6,17, 37,38, 40,77-89 | A61K7/025 A61K7/032 A61K7/043 |
| Y | | 7-16, 18-36, 39,41-76 | |
| Y,D | DATABASE WPI Week 200251 Derwent Publications Ltd., London, GB; AN 2002-475555 XP002315084 & JP 2002 128623 A (KOSE KK) 9 mai 2002 (2002-05-09) * abrégé * | 7-16 | |
| Y | US 2003/235553 A1 (SHAOXIANG LU ET AL.) 25 décembre 2003 (2003-12-25) * revendications 1,28 * * alinéas [0002], [0009], [0013], [0014], [0018], [0023] - [0027], [0284], [0290], [0291], [0293], [0294], [0300], [0302] - [0358] * * alinéas [0370], [0384] - [0402] * | 18-28, 30-36, 39, 41-64, 66-76 | DOMAINES TECHNIQUES RECHERCHES (Int.Cl.7) A61K |
| Y | US 2004/042980 A1 (MOHAMED KANJI ET AL.) 4 mars 2004 (2004-03-04) * alinéas [0055], [0056] * | 29 | |

-/--

1

Le présent rapport a été établi pour toutes les revendications

| Lieu de la recherche | Date d'achèvement de la recherche | Examinateur |
|---|---|---|
| Berlin | 5 septembre 2005 | Alvarez Alvarez, C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Office européen des brevets

# RAPPORT DE RECHERCHE EUROPEENNE

Numéro de la demande
EP 05 29 1036

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| Y | EP 0 923 928 A (L'OREAL) 23 juin 1999 (1999-06-23) * alinéa [0048]; revendications 1,11 * ----- | 65 | |
| A | EP 1 066 826 A (KAO CORPORATION) 10 janvier 2001 (2001-01-10) * alinéas [0014], [0015], [0047] * ----- | 1 | |
| A | EP 1 066 815 A (DAIKIN INDUSTRIES LTD.) 10 janvier 2001 (2001-01-10) * alinéa [0066] * ----- | 1 | |
| A | US 6 342 556 B1 (BATDORF ET AL.) 29 janvier 2002 (2002-01-29) * colonne 3, ligne 24 - ligne 38 * ----- | 1 | |

DOMAINES TECHNIQUES RECHERCHES (Int.Cl.7)

Le présent rapport a été établi pour toutes les revendications

1

| Lieu de la recherche | Date d'achèvement de la recherche | Examinateur |
|---|---|---|
| Berlin | 5 septembre 2005 | Alvarez Alvarez, C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................................
& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 05 29 1036

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

05-09-2005

| Document brevet cité au rapport de recherche | Date de publication | Membre(s) de la famille de brevet(s) | Date de publication |
|---|---|---|---|
| FR 2795309 A | 29-12-2000 | FR 2795309 A1 | 29-12-2000 |
| | | JP 2001072530 A | 21-03-2001 |
| JP 2002128623 A | 09-05-2002 | AUCUN | |
| US 2003235553 A1 | 25-12-2003 | AU 2003260300 A1 | 06-01-2004 |
| | | WO 2004000247 A1 | 31-12-2003 |
| | | EP 1515685 A1 | 23-03-2005 |
| | | US 2004170586 A1 | 02-09-2004 |
| US 2004042980 A1 | 04-03-2004 | US 2004247549 A1 | 09-12-2004 |
| EP 0923928 A | 23-06-1999 | FR 2772602 A1 | 25-06-1999 |
| | | BR 9805775 A | 11-04-2000 |
| | | CA 2255150 A1 | 22-06-1999 |
| | | CN 1225260 A | 11-08-1999 |
| | | EP 0923928 A1 | 23-06-1999 |
| | | JP 3256507 B2 | 12-02-2002 |
| | | JP 11236314 A | 31-08-1999 |
| | | PL 330455 A1 | 05-07-1999 |
| | | US 6682748 B1 | 27-01-2004 |
| EP 1066826 A | 10-01-2001 | JP 2001019633 A | 23-01-2001 |
| | | JP 2001226225 A | 21-08-2001 |
| | | JP 2001286411 A | 16-10-2001 |
| | | CN 1283448 A ,C | 14-02-2001 |
| | | DE 60016672 D1 | 20-01-2005 |
| | | EP 1066826 A2 | 10-01-2001 |
| | | ID 26471 A | 11-01-2001 |
| | | US 6492307 B1 | 10-12-2002 |
| EP 1066815 A | 10-01-2001 | JP 11269231 A | 05-10-1999 |
| | | EP 1066815 A1 | 10-01-2001 |
| | | US 6641805 B1 | 04-11-2003 |
| | | WO 9948464 A1 | 30-09-1999 |
| US 6342556 B1 | 29-01-2002 | CA 2202587 A1 | 16-10-1997 |
| | | JP 10036716 A | 10-02-1998 |

EPO FORM P0460

Pour tout renseignement concernant cette annexe : voir Journal Officiel de l'Office européen des brevets, No.12/82